# EUROPEAN PATENT SPECIFICATION

(11) **EP 2 702 070 B1**
(45) Date of publication and mention of the grant of the patent: **30.09.2015**
(21) Application number: 12717317.7
(22) Date of filing: 27.04.2012
(51) Int. Cl.: C07K 14/005

(54) **NEW ANTIMICROBIAL AGENTS**
NEUE ANTIMIKROBIELLE MITTEL
NOUVEAUX AGENTS ANTIMICROBIENS

(30) Priority: 27.04.2011 EP 11163897
(43) Date of publication of application: 05.03.2014
(73) Proprietor: Lysando AG, 9497 Triesenberg (LI)
(72) Inventor: MILLER, Stefan, 93055 Regensburg (DE)
(74) Representative: Rückerl, Florian
(86) International application number: PCT/EP2012/057808
(87) International publication number: WO 2012/146738

(56) References cited:
- MANOHARADAS S ET AL: "Antimicrobial activity of a chimeric enzybiotic towards Staphylococcus aureus", JOURNAL OF BIOTECHNOLOGY, ELSEVIER SCIENCE PUBLISHERS, AMSTERDAM, NL, vol. 139, no. 1, 1 January 2009 (2009-01-01), pages 118-123, XP025796283, ISSN: 0168-1656, DOI: 10.1016/J.JBIOTEC.2008.09.003 [retrieved on 2008-09-26]
- BORYSOWSKI J ET AL: "Bacteriophage endolysins as a novel class of antibacterial agents", EXPERIMENTAL BIOLOGY AND MEDICINE, SOCIETY FOR EXPERIMENTAL BIOLOGY AND MEDICINE, US, vol. 231, no. 4, 1 April 2006 (2006-04-01) , pages 366-377, XP002449938, ISSN: 1535-3702
- HERMOSO ET AL: "Taking aim on bacterial pathogens: from phage therapy to enzybiotics", CURRENT OPINION IN MICROBIOLOGY, CURRENT BIOLOGY LTD, GB, vol. 10, no. 5, 1 October 2007 (2007-10-01), pages 461-472, XP022338241, ISSN: 1369-5274, DOI: 10.1016/J.MIB.2007.08.002
- DATABASE UniProt [Online] 7 June 2005 (2005-06-07), "SubName: Full=ORF007;", XP002678019, retrieved from EBI accession no. UNIPROT:Q4ZD58 Database accession no. Q4ZD58
- FERNANDES SOFIA ET AL: "Novel Chimerical Endolysins with Broad Antimicrobial Activity Against Methicillin-Resistant Staphylococcus aureus.", MICROBIAL DRUG RESISTANCE (LARCHMONT, N.Y.) JUN 2012 LNKD- PUBMED:22432707, vol. 18, no. 3, 1 June 2012 (2012-06-01), pages 333-343, XP002678020, ISSN: 1931-8448

## Description

The present invention relates to a fusion protein comprising an endolysin with an amino acid sequence according to SEQ ID NO: 1 and fragments and/or derivatives thereof and an additional cationic or polycationic peptide, an amphipatic peptide, a sushi peptide, a defensin, a hydrophobic peptide or an antimicrobial peptide fused to said endolysin, fragment and/or derivative at the N- and/or C-terminus. Moreover, the present invention relates to nucleic acid molecules encoding said fusion protein, vectors comprising said nucleic acid molecules and host cells comprising either said nucleic acid molecules or said vectors: In addition, the present invention relates to said fusion protein for use as a medicament, in particular for the treatment or prevention of staphylococcal infections, as diagnostic means, as cosmetic substance or as sanitizing agent. The present invention also relates to the use of said fusion protein for the treatment or prevention of staphylococcal contamination of foodstuff, of food processing equipment, of food processing plants, of feed for livestock animals, of surfaces coming into contact with foodstuff, of medical devices, of surfaces in hospitals and surgeries. Furthermore, the present invention relates to a pharmaceutical composition comprising said fusion protein.

The bacterium *Staphylococcus aureus* is a facultative anaerobic, Gram-positive coccus. It occurs almost everywhere in the nature, as well as on the skin and the upper respiratory tract of about 25 to 30 % humans. Usually *Staphylococcus aureus* does not cause any symptoms. Due to more favourable conditions or a weak immune system, *Staphylococcus aureus* may spread and thus, causes in humans infections of the skin, like pyoderma, particularly folliculitis, furuncle, carbuncle, abscesses of the sweat glands and pemphigus, and like scaled skin syndrome, endocarditis, toxic shock syndrome and sepsis. In cases such bacteria are resistant against several important antibiotics (multi-resistant), it is hard to eliminate these bacteria and they become very dangerous after infection of third parties.

Multi-resistant *Staphylococcus aureus* are *Staphylococcus aureus* strains which are resistant against all commercially available β-lactam antibiotics, e.g. penicillin. But they are often resistant against more than one class of antibiotics, such as chinolone, tetracyclins, aminoglycosides, erythromycin and sulfonamides. The increasing rate of multi-resistant *S. aureus* strains represents a serious problem in the therapy of infections due to *S. aureus.* Thus, there is a need for antimicrobial agents against *Staphylococcus aureus.*

Endolysins are peptidoglycan hydrolases encoded by bacteriophages (or bacterial viruses). They are synthesized during late gene expression in the lytic cycle of phage multiplication and mediate the release of progeny virions from infected cells through degradation of the bacterial peptidoglycan. They are either ß-(1,4)-glycosylases (lysozymes), transglycosylases, amidases or endopeptidases. Antimicrobial application of endolysins was already suggested in 1991 by Gasson (GB2243611). Although the killing capacity of endolysins has been known for a long time, the use of these enzymes as antibacterials was ignored due to the success and dominance of antibiotics. Only after the appearance of multiple antibiotic resistant bacteria this simple concept of combating human pathogens with endolysins received interest. A compelling need to develop totally new classes of antibacterial agents emerged and endolysins used as 'enzybiotics' - a hybrid term of 'enzymes' and 'antibiotics' - perfectly met this need. In 2001, Fischetti and coworkers demonstrated for the first time the therapeutic potential of bacteriophage Cl endolysin towards group A streptococci (Nelson et al., 2001). Since then many publications have established endolysins as an attractive and complementary alternative to control bacterial infections, particularly by Grain-positive bacteria. Subsequently different endolysins against other Gram-positive pathogens such as *Streptococcus pneumoniae* (Loeffler et al., 2001), *Bacillus anthracis* (Schuch et al., 2002), *S. agalactiae* (Cheng et al., 2005) and *Staphylococcus aureus* (Rashel et al, 2007) have proven their efficacy as enzybiotics. However, it is also known that endolysins can, under some conditions (e.g. high ionic strength), create stable protoplast, where the internal bacterial cell pressure is not sufficient to lead to a cell burst. Under these conditions the bacterial cell wall can regenerate and the bacteria will survive.

Thus, there is a need for new antimicrobial agents against Staphylococcal bacteria.

This object is solved by the subject matter defined in the claims.

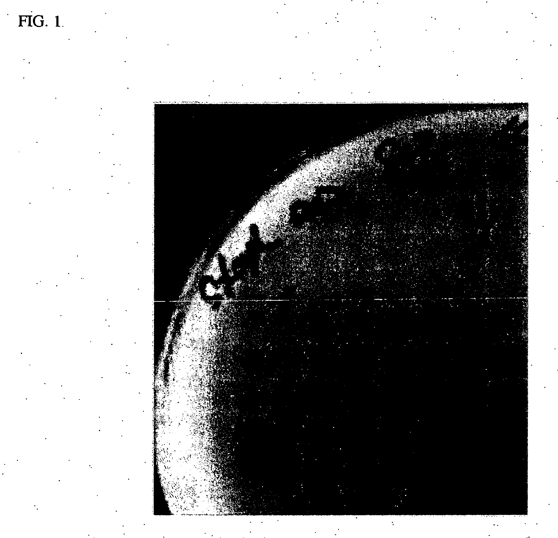

Figure 1 shows a photograph of a Staph. aureus culture grown on an agar plate with a well-defined clear cut spot (black arrow; #2) that appeared within 16h of incubation with Ply2638A-PK.

The term "protein" as used herein refers synonymously to the term "polypeptide". The term "protein" as used herein refers to a linear polymer of amino acid residues linked by peptide bonds in a specific sequence. The amino-acid residues of a protein may be modified by e.g. covalent attachments of various groups such as carbohydrates and phosphate. Other substances may be more loosely associated with the polypeptide chains, such as heme or lipid, giving rise to the conjugated proteins which are also comprised by the term "protein" as used herein. There are various ways in which the polypeptide chains fold have been elucidated, in particular with regard to the presence of alpha helices and beta-pleated sheets. The term "protein" as used herein refers to all four classes of proteins being all-alpha, all-beta, alpha/beta and alpha plus beta. Moreover, the term "protein" refers to a complex, wherein the complex refers to a homomer.

The term "fusion protein" as used herein refers to an expression product resulting from the fusion of two or more nucleic acid sequences. Such a protein may be produced, e.g., in recombinant DNA expression systems. Moreover, the term "fusion protein" as used herein refers to a fusion of a first amino acid sequence, namely the endolysin Ply2638A and fragments and/or derivatives thereof, with a second or further amino acid sequences. The second or further amino acid sequence is preferably a peptide, in particular a cationic and/or a polycationic peptide, an amphipatic peptide, a sushi peptide, a defensin, a hydrophobic peptide and/or an antimicrobial peptide. Preferably, said second and/or further amino acid sequence is foreign to and not substantially homologous with any domain of the first amino acid sequence.

The term "peptide stretch" as used herein refers to any kind of peptide linked to a protein such as an endolysin. In particular the term "peptide stretch" as used herein refers to a cationic peptide or a polycationic peptide, an amphipatic peptide, a sushi peptide, a defensin, a hydrophobic peptide and/or an antimicrobial peptide. However, a peptide stretch in the meaning of the present invention does not refer to His-tags, preferably His₅-tags, His₆-tags, His₇-tags, His₈-tags, His₉-tags, His₁₀-tags, His₁₁-tags, His₁₂-tags, His₁₆-tags and His₂₀-tags, Strep-tags, Avi-tags, Myc-tags, Gst-tags, JS-tags, cystein-tags, FLAG-tags or other tags known in the art, thioredoxin or maltose binding proteins (MBP). The term "tag" in contrast to the term "peptide stretch" as.used herein refers to a peptide which can be useful to facilitate expression and/or affinity purification of a polypeptide, to immobilize a polypeptide to a surface or to serve as a marker or a label moiety for detection of a polypeptide e.g. by antibody binding in different ELISA assay formats as long as the function making the tag useful for one of the above listed facilitation is not caused by the positively charge of said peptide. However, the His₆-tag may, depending on the respective pH, also be positively charged, but is used as affinity purification tool as it binds to immobilized divalent cations and is not used as a peptide stretch according to the present invention.

The term "peptide" as used herein refers to short polypeptides consisting of from about 2 to about 100 amino acid residues, more preferably from about 4 to about 50 amino acid residues, more preferably to about 5 to 30 amino acid residues, wherein the amino group of one amino acid residue is linked to the carboxyl group of another amino acid residue by a peptide bond. A peptide may have a specific function. A peptide can be a naturally occurring peptide or a synthetically designed and produced peptide. The peptide can be, for example, derived or removed from a native protein by enzymatic or chemical cleavage, or can be prepared fusing conventional peptide synthesis techniques (e.g., solid phase synthesis) or molecular biology techniques (see Sambrook, J. et al., Molecular Cloning: A Laboratory Manual, Cold Spring Harbor Press, Cold Spring Harbor, N.Y. (1989)). Preferred synthetically produced peptides are e.g. cationic, polycationic peptides, amphipatic peptides and hydrophobic peptides. Preferred naturally occuring peptides are e.g. amphipatic peptides, sushi peptides, defensines and antimicrobial peptides. The peptides have no or only low cell wall degrading activity but may have cell wall disprupting activity. A peptide in the meaning of the present invention does not refer to His-tags, Strep-tags, thioredoxin or maltose binding proteins (MBP) or the like, which are used to purify or locate proteins.

As used herein, the term "cationic peptide" refers to a peptide having positively charged amino acid residues. Preferably a cationic peptide has a pKa-value of 9.0 or greater. Typically, at least four of the amino acid residues of the cationic peptide can be positively charged, for example, lysine or arginine. "Positively charged" refers to the side chains of the amino acid residues which have a net positive charge at about physiological conditions. The term "cationic peptide" as used herein refers also to polycationic peptides.

The term "polycationic peptide" as used herein refers to a synthetically designed and produced peptide composed of mostly positively charged amino acid residues, in particular lysine, arginine and/or histidine residues, more preferably lysine and/or arginine residues. A peptide is composed of mostly positively charged amino acid residues if at least about 20, 30, 40, 50, 60, 70, 75, 80, 85, 90, 95 or about 100 % of the amino acid residues are positively charged amino acid residues, in particular lysine and/or arginine residues. The amino acid residues being not positively charged amino acid residues can be neutrally charged amino acid residues and/or negatively charged amino acid residues and/or hydrophobic amino acid residues. Preferably the amino acid residues being not positively charged amino acid residues are neutrally charged amino acid residues, in particular serine and/or glycine.

The term, "antimicrobial peptide" (AMP) as used herein refers to any naturally occurring peptide that has microbicidal and/or microbistatic activity on for example bacteria, viruses, fungi, yeasts, mycoplasma and protozoa. Thus, the term "antimicrobial peptide" as used herein refers in particular to any peptide having anti-bacterial, anti-fungal, anti-mycotic, antiparasitic, anti-protozoal, anti-viral, anti-infectious, anti-infective and/or germicidal, algicidal, amoebicidal, microbicidal, bactericidal, fungicidal, parasiticidal, protozoacidal, protozoicidal properties. The antimicrobial peptide may be a member of the RNAse A super family, a defensin, cathelicidin, granulysin, histatin, psoriasin, dermicidine or hepcidin. The antimicrobial peptide may be naturally occurring in insects, fish, plants, arachnids, vertebrates or mammals. Preferably the antimicrobial peptide may be naturally occurring in insects, fish, plants, arachnids, vertebrates or mammals. Preferably the antimicrobial peptide may be naturally occurring in radish, silk moth, wolf spider, frog, preferably in *Xenopus laevis, Rana* frogs, more preferably in *Rana catesbeiana,* toad, preferably Asian toad *Bufo bufo gargarizans,* fly, preferably in *Drosophila,* more preferably in *Drosophila melanogaster,* in *Aedes aegypti,* in honey bee, bumblebee, preferably in *Bombus pascuorum,* flesh fly, preferably in *Sarcophaga peregrine,* scorpion, horseshoe crab, catfish, preferably in *Parasilurus asotus,* cow, pig, sheep, porcine, bovine, monkey and human.

The term "sushi peptide" as used herein refers to complement control proteins (CCP) having short consensus repeats. The sushi module of sushi peptides functions as a protein-protein interaction domain in many different proteins. Peptides containing a Sushi domain have been shown to have antimicrobial activities. Preferably, sushi peptides are naturally occurring peptides.

The term "amphiphatic peptide" as used herein refers to synthetic peptides having both hydrophilic and hydrophobic functional groups. Preferably, the term "amphiphatic peptide" as used herein refers to a peptide having a defined arrangement of hydrophilic and hydrophobic groups e.g. amphiphatic peptides may be e.g. alpha helical, having predominantly non polar side chains along one side of the helix and polar residues along the remainder of its surface.

The term "hydrophobic group" as used herein refers to chemical groups such as amino acid side chains which are substantially water insoluble, but soluble in an oil phase, with the solubility in the oil phase being higher than that in water or in an aqueous phase. In water, amino acid residues having a hydrophobic side chain interact with one another to generate a nonaqueous environment: Examples of amino acid residues with hydrophobic side chains are valine, isoleucine, leucine, methionine, phenylalanine, tryptophan, cysteine, alanine, tyrosine, histidine, threonine, serine, proline and glycine residues.

The term "endolysin" as used herein refers to an enzyme which is suitable to hydrolyse bacterial cell walls. "Endolysins" comprise at least one "enzymatically active domain" (EAD) having at least one of the following activities: endopeptidase; N-acetyl-muramoyl-L-alanine-amidase (amidase), N-acetyl-muramidase, N-acetyl-glucosaminidase (lysozyme) or transglycosylases. In addition, the endolysins may contain also regions which are enzymatically inactive, and bind to the cell wall of the host bacteria, the so-called CBDs (cell wall binding domains). The endolysin may contain one, two or more CBDs. However, the term "endolysin" as used herein refers also to enzymes having at least one EAD but no CBDs. Generally, the cell wall binding domain is able to bind different components on the surface of bacteria. Preferably, the cell wall binding domain is a peptidoglycan binding domain and binds to the bacteria's peptidoglycan.

The term "cell wall" as used herein refers to all components that form the outer cell enclosure of the Gram-positive bacteria and thus guarantee their integrity. In particular, the term "cell wall" as used herein refers to peptidoglycan, the bacterial cell membrane, but also to additional layers deposited on the peptidoglycan as e.g. capsules, outer protein layers or slimes. The fusion proteins aqccording to the present invention have a cell wall degrading activity, i.e. they cleave the components, e.g.the peptidoglycan enzymatically. In contrast the peptides do have no or only very low enzymatic activity but may have cell wall disrupting, i.e. physically disrupting activity.

The term "EAD" as used herein refers to the enzymatically active domain of an endolysin. The EAD is responsible for hydrolysing bacterial peptidoglycans. It exhibits at least one enzymatic activity of an endolysin. The EAD can also be composed of more than one enzymatically active module. The term "EAD" is used herein synonymously with the term "catalytic domain".

The term "domain linker" as used herein refers to an amino acid sequence functioning to connect single protein domains with one another. As a rule domain linkers form no or only few regular secondary structure like α-helices or ß-sheets and can occupy different conformations with the respective structural context. Methods to detect domain linker and properties of linker sequences are well known in the art as e.g. described in Bae et al., 2005, Bioinformatics, 21, 2264-2270 or George & Heringa, 2003, Protein Engineering, 15, 871-879. The term "deletion" as used herein refers to the removal of 1, 2, 3, 4, 5 or more amino acid residues from the respective starting sequence.

The term "insertion" or "addition" as used herein refers to the insertion or addition of 1, 2, 3, 4, 5 or more amino acid residues to the respective starting sequence.

The term "substitution" as used herein refers to the exchange of an amino acid residue located at a certain position for a different one.

The present invention relates to new antibacterial agents against staphylococcal bacteria. Surprisingly the inventors found that in comparison to the wild type Ply2638A endolysin that has only very low bacterial cell wall degrading, i.e. antibacterial activity on *Staphylococcus aureus* strains the fusion proteins according to the present invention have a very strong antibacterial activity on *Staphylococcus aureus* strains. Thus, one aspect of the present invention relates to a fusion protein comprising the endolysin. Ply2638A comprising an amino acid sequence according to SEQ ID NO: 1, a fragment and/or derivative thereof and an additional cationic,polycationic peptide, an amphipatic peptide, a sushi peptide, a defensin, a hydrophobic peptide and/or an antimicrobial peptide fused to the N- and/or C-terminus of said endolysin, fragment and/or derivative thereof. The endolysin Ply2638 comprising an amino acid sequence according to SEQ ID NO: 1 is preferably encoded by a nucleotide sequence according to SEQ ID NO: 2.

The endolysin Ply2638 (GENBANK Accession number YP_239818) having an amino acid sequence according to SEQ ID NO: 1 has a length of 486 amino acids. It comprises a C-terminal cell wall binding domain (CBD) and two N-terminal enzymatic active domains (EADs). The putative C-terminal CBD is a peptidoglycan binding domain (PGB, aa 401-468) having an amino acid sequence according to SEQ ID NO: 3. The N-terminal EADs consists of a putative M23 peptidase (aa 47-146) according to SEQ ID NO: 4 and a putative amidase domain (aa 199-325) according to SEQ ID NO: 5. The PGB and the catalytic domains of the endolysin Ply2638 are connected by linker sequences.

Preferred fragments of the endolysin Ply2638A according to the present invention are polypeptides comprising an amino acid sequence according to SEQ ID NO: 3, according to SEQ ID NO: 4 and/or according to SEQ ID NO: 5. Another preferred fragment of the endolysin according to the present invention comprises an amino acid sequence according to SEQ ID NO: 6. The fragment having an amino acid sequence according to SEQ ID NO: 6 differs from the endolysin having an amino acid sequence according to SEQ ID NO: 1 in that the starting methionine residue has been deleted.

The derivatives of the endolysin according to the present invention are polypeptides comprising an amino acid sequence according to SEQ ID NO: 1, 3, 4, 5 and/or 6 but having additional modification and/or alterations. Said modifications and/or alterations can be mutations in particular deletions, insertions, additions, substitutions or any combinations thereof and/or chemical changes of the amino acid residues, e.g. biotinylation, acetylation, pegylation, chemical changes of the amino-, SH- or carboxyl- groups. Said derivatives according to the present invention exhibit the lytic activity of the Ply2638 endolysin or the activity of the fragments according to the present invention. Said activity can be about 10, 20, 30, 40, 50, 60, 70, 80, 90, 100, 10, 120, 130, 140, 150, 160, 170, 180, 190 or about 200 % of the activity of the Ply2638A and/or the activity of the fragments according to the present invention. The activity can be measured by assays well known in the art by a person skilled in the art as e.g. the plate lysis assay or the liquid lysis assay which are e.g. described in Briers et al., J. Biochem. Biophys Methods 70: 531-533, (2007*) or similar publications.*

In a preferred embodiment of the present invention the fusion protein comprising the endolysin according to SEQ ID NO: 1, a fragment and/or derivative thereof and an additional cationic or polycationic peptide, an amphipatic peptide, a sushi peptide, a defensin, a hydrophobic peptide and/or an antimicrobial peptide at the N- and/or C-terminus of said endolysin, fragment and/or derivative thereof according to the present invention comprises additionally a tag such as a His₆-tag, Strep-tag, Avi-tag, Myc-tag, Gst-tag, JS-tag, cystein-tag, FLAG-tag or other tags known in the art at the N-terminus and/or at the C-terminus. In a preferred embodiment of the present invention said tag is linked to the fusion protein according to the present invention at the C-terminus. Said tag may be linked to said fusion protein over additional amino acid residues. Said additional amino acid residues may be at least 1, 2, 3, 4, 5, 6, 7, 8, 9 or 10 additional amino acid residues. In a preferred embodiment of the present invention the tag is linked to the fusion protein according to the present invention by the additional amino acid residues Leu-Glu or Lys-Gly.

The peptide according to the present invention is preferably covalently bound to the endolysin, fragment and/or derivative thereof according to the present invention. Preferably, said peptide consists of at least 5, more preferably at least of 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 or at least 100 amino acid residues. Especially preferred is a peptide comprising about 5 to about 100 amino acid residues, about 5 to about 50 or about 5 to about 30 amino acid residues. More preferred is a peptide comprising about 6 to about 42 amino acid residues, about 6 to about 39 amino acid residues, about 6 to about 38 amino acid residues, about 6 to about 31 amino acid residues, about 6 to about 25 amino acid residues, about 6 to about 24 amino acid residues, about 6 to about 22 amino acid residues, about 6 to about 21 amino acid residues, about 6 to about 20 amino acid residues, about 6 to about 19 amino acid residues, about 6 to about 16 amino acid residues, about 6 to about 14 amino acid residues, about 6 to about 12 amino acid residues, about 6 to about 10 aniino acid residues or about 6 to about 9 amino acid residues.

Preferably, the peptide is no tag such as a His₆-tag, Strep-tag, Avi-tag, Myc-tag, Gst-tag, JS-tag, cystein-tag, FLAG-tag or other tags known in the art and no thioredoxin or maltose binding proteins (MBP). However, the peptide may comprise in addition such tag or tags or the like, which are used to purify or locate proteins.

Preferably, the peptide has the function to facilitate lysis of Gram-positive bacteria by the fusion protein according to the present invention but has no or only low cell wall degrading activity when administered without being fused to the endolysin, fragment and/or derivative thereof according to the present invention. Subsequently, the antibacterial activity of the fusion protein consisting of the endolysin, fragment and/or derivative thereof according to the present invention and the peptide to be tested can be compared to the endolysin, fragment and/or derivative thereof according the present invention having no peptide as also described in the Examples of the present invention. Preferably, said tests may be carried out on staphylococcal cells as used in the Examples of the present invention. In case the fusion protein has an increased antibacterial activity in comparison to the endolysin, fragment and/or derivative thereof according to the present invention without said peptide for at least one of the tested staphylococcal bacteria species then said peptide has a cell wall disrupting activity. Preferably, the antibacterial activity (in logarithmic units (=log₁₀N₀/Nᵢ)) of the endolysin, fragment and/or derivative thereof according to the present invention is increased, preferably by at least about 5 %, more preferably by at least about 10%, 20%, 30%, 40% 50% 60%, 70%, 80%, 90% or 100% or by any value in the range of up to 100% or even more by a peptide having cell wall disrupting activity.

In a preferred embodiment the peptide according to the present invention is selected from the group of cationic peptides and polycationic peptides.

In one aspect of the present invention the peptide is an cationic and/or polycationic peptide, which comprises one or more of the positively charged amino acid residues of lysine, arginine and/or histidine, in particular of lysine and/or arginine. Preferably, more than about 20, 30, 40, 50, 60, 70, 75, 80, 85, 90, 95 or 99 % of the amino acid residues in said peptide are positively charged amino acid residues, in particular lysine and/or arginine residues. Especially preferred are peptides consisting of about 100 % positively charged amino acid residues, in particular arginine and/or lysine residues, wherein preferably about 60 % to about 70 % of said positively charged amino acid residues are lysine residues and about 30 % to about 40 % of said positively charged amino acid residues are arginine residues. More preferred is a peptide consisting of about 100 % positively charged amino acid residues, in particular arginine and/or lysine residues, wherein preferably about 64 % to about 68 % of said positively charged amino acid residues are lysine and about 32 % to about 36 % of said positively charged amino acid residues are arginine. Peptides consisting of either only arginine or only lysine are also preferred.

Especially preferred are cationic and/or polycationic peptides comprising at least one motive according to SEQ ID NO: 11 (KRKKRK). In particular cationic peptides comprising at least 2, 3, 4, 5, 6, 7, 8, 9, 10, 11, 12, 13, 14, 15, 16 or 17 motives according to SEQ ID NO: 11 (KRKKRK) are preferred. More preferred are cationic peptides comprising at least one KRK motive (lys-arg-lys), preferable at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32 or 33 KRK motives.

In another preferred embodiment of the present invention the cationic peptide comprises beside the positively charged amino acid residues, in particular lysine and/or arginine residues, neutrally charged amino acid residues, in particular glycine and/or serine residues. Preferred are cationic peptides consisting of about 70 % to about 100 %, or about 80 % to about 95 %, or about 85 % to about 90 % positively charged amino acid residues, in particular lysine and/or arginine residues and of about 0 % to about 30 %, or about 5 % to about 20 %, or about 10 % to about 20 % neutrally charged amino acid residues, in particular glycine and/or serine residues. Preferred are peptides consisting of about 4 % to about 8 % serine residues, of about 33 % to about 36 % arginine residues and of about 56 % to about 63 % lysine residues. Especially preferred are peptides comprising at least one motive according to SEQ ID NO: 32 (KRXKR), wherein X is any other amino acid residue than lysine, arginine and histidine. Especially preferred are peptides comprising at least one motive according to SEQ ID NO: 33 (KRSKR). More preferred are cationic peptides comprising at least about 2, 3, 4, 5, 6, 7, 8, 9, 10, 11, 12, 13, 14, 15, 16, 17, 18, 19 or about 20 motives according to SEQ ID NO: 32 (KRXKR) or SEQ ID NO: 33 (KRSKR).

Also preferred are peptides consisting of about 9 to about 16 % glycine residues, of about 4 to about 11 % serine residues, of about 26 to about 32 % arginine residues and of about 47 to about 55 % lysine residues. Especially preferred are peptides comprising at least one motive according to SEQ ID NO: 34 (KRGSG). More preferred are cationic peptides comprising at least about 2, 3, 4, 5, 6, 7, 8, 9, 10, 11, 12, 13, 14, 15, 16, 17, 18, 19 or about 20 motives according to SEQ ID NO: 34 (KRGSG).

In another preferred embodiment of the present invention the cationic peptide comprises beside the positively charged amino acid residues, in particular lysine and/or arginine residues, hydrophobic amino acid residues, in particular valine, isoleucine, leucine, methionine, phenylalanine, tryptophan, cysteine, alanine, tyrosine, histidine, threonin, serine, proline and/or glycine residues. Preferred are cationic peptides consisting of about 70 % to about 100 %, or about 80 % to about 95 %, or about 85 % to about 90 % positively charged amino acid residues, in particular lysine and/or arginine residues and of about 0 % to about 30 %, or about 5 % to about 20 %, or about 10 % to about 20 % hydrophobic amino acid residues in particular valine, isoleucine, leucine, methionine, phenylalanine, tryptophan, cysteine, alanine, tyrosine, histidine, threonin, serine, proline and/or glycine residues.

Especially preferred peptides are presented in the following table:

**Table 1:**

| **Peptide** | **length** | **SEQ ID NO:** |
|---|---|---|
| KRKKRK | 6 | SEQ ID NO: 11 |
| KRKKRKKRK | 9 | SEQ ID NO: 12 |
| RRRRRRRRR | 9 | SEQ ID NO: 13 |
| KKKKKKKK | 8 | SEQ ID NO: 14 |
| KRKKRKKRKK | 10 | SEQ ID NO: 15 |
| KRKKRKKRKKRK | 12 | SEQ ID NO: 16 |
| KRKKRKKRKKRKKR | 14 | SEQ ID NO: 17 |
| KKKKKKKKKKKKKKKK | 16 | SEQ ID NO: 18 |
| KRKKRKKRKKRKKRKKRKK | 19 | SEQ ID NO: 19 |
| RRRRRRRRRRRRRRRRRRR | 19 | SEQ ID NO: 20 |
| KKKKKKKKKKKKKKKKKKK | 19 | SEQ ID NO: 21 |
| KRKKRKKRKRSKRKKRKKRK | 20 | SEQ ID NO: 22 |
| KRKKRKKRKRSKRKKRKKRKK | 21 | SEQ ID NO: 23 |
| KRKKRKKRKKRKKRKKRKKRK | 21 | SEQ ID NO: 24 |
| KRKKRKKRKRGSGKRKKRKKRK | 22 | SEQ ID NO: 25 |
| KRKKRKKRKRGSGSGKRKKRKKRK | 24 | SEQ ID NO: 26 |
| KRKKRKKRKKRKKRKKRKKRKKRKK | 25 | SEQ ID NO: 27 |
| KRKKRKKRKRSKRKKRKKRKRSKRKKRKKRK | 31 | SEQ ID NO: 28 |
| KRKKRKKRKRGSGSGKRKKRKKRKGSGSGKRKKRKKRK | 38 | SEQ ID NO: 29 |
| KRKKRKKRKKRKKRKKRKKRKKRKKRKKRKKRKKRKKRK | 39 | SEQ ID NO: 30 |
| KRKKRKKRKRSKRKKRKKRKRSKRKKRKKRKRSKRKKRKKRK | 42 | SEQ ID NO: 31 |

In a further preferred embodiment of the present invention the fused peptide stretch is an amphipathic peptide, which comprises one or more of the positively charged amino acid residues of lysine, arginine and/or histidine, combined to one or more of the hydrophobic amino acid residues of valine, isoleucine, leucine, methionine, phenylalanine, tryptophan, cysteine, alanine, tyrosine, histidine, threonin, serine, proline and/or glycine. Side chains of the amino acid residues are preferably oriented in order that cationic and hydrophobic surfaces are clustered at opposite sides of the peptide. Preferably, more than about 30, 40, 50, 60 or 70% of the amino acids in said peptide are positively charged amino acids. Preferably, more than about 30, 40, 50, 60 or 70% of the amino acid residues in said peptide are hydrophobic amino acid residues. Advantageously, the amphipathic peptide is fused at the N-terminal and/or the C-terminal end of the polypeptide; fragment and/or derivative according to the present invention having cell wall degrading activity, thus enhancing the amphipathicity of the latter proteins.

In a preferred embodiment at least about 30, 40, 50, 60 or 70% of the said amino acid residues of the amphipathic peptide are either arginine or lysine residues and/or at least about 30, 40, 50, 60 or 70% of the said amino acid residues of the amphipathic peptide are of the hydrophobic amino acid residues valine, isoleucine, leucine, methionine, phenylalanine, tryptophan, cysteine, alanine, tyrosine, histidine, threonin, serine, proline and/or glycine. Preferred amphipatic peptides are Pleurocidin according to SEQ ID NO:45, Cecropin P1 according to SEQ ID NO:52, Buforin II according to SEQ ID NO:47, Buforin I according to SEQ ID NO:61 and Magainin according to SEQ ID NO:44. Further preferred amphipathic peptides are Cathelidicine e.g. LL-37 according to SEQ ID NO:43.

In a further aspect of the present invention the fused peptide stretch is an antimicrobial peptide, which comprises a positive net charge and around 50% hydrophobic amino acid residues. The antimicrobial peptides are amphipathic, with a length of about 12 to about 50 amino acid residues.

Preferred antimicrobial peptides are listed in the following Table 2.

| **Peptid** | **Sequenz** | |
|---|---|---|
| LL-37 | LLGDFFRKSKEKIGKEFKRIVQRIKDFLRNLVPRTES | SEQ ID NO: 42 |
| SMAP-29 | RGLRRLGRKIAHGVKKYGPTVLRIIRIAG | SEQ ID NO: 49 |
| Indolicidin | ILPWKWPWWPWRR | SEQ ID NO: 43 |
| Protegrin | RGGRLCYCRRRFCVCVGR | SEQ ID NO: 51 |
| Cecropin P1 | SWLSKTAKKLENSAKKRISEGIAIAIQGGPR | SEQ ID NO: 52 |
| Magainin | GIGKFLHSAKKFGKAFVGEIMNS | SEQ ID NO: 44 |
| Pleurocidin | GWGSFFKKAAHVGKHVGKAALTHYL | SEQ ID NO: 45 |
| Cecropin A (A.aegypti) | GGLKKLGKKLEGAGKRVFNAAEKALPVVAGAKALRK | SEQ ID NO: 46 |
| Cecropin A (D. melanogaster) | GWLKKIGKKIERVGQHTRDATIQGLGIPQQAANVAATARG | SEQ ID NO: 53 |
| Buforin II | TRSSRAGLQFPVGRVHRLLRK | SEQ ID NO: 47 |
| Sarcotoxin IA | GWLKKIGKKIERVGQHTRDATIQGLGIAQQAANVAATAR | SEQ ID NO: 48 |
| Ascaphine | GIKDWIKGAAKKLIKTVASHIANQ | SEQ ID NO: 54 |
| Apidaecine | ANRPVYIPPPRPPHPRL | SEQ ID NO: 55 |
| Nigrocine | GLLSKVLGVGKKVLCGVSGLVC | SEQ ID NO: 56 |
| Pseudin 1 | GLNTLKKVFQGLH EAIKLINNHVQ | SEQ ID NO: 41 |
| Parasin 1 | KGRGKQGGKVRAKAKTRSS | SEQ ID NO: 57 |
| Lycotoxin | IWLTALKFLGKHAAKKLAKQQLSKL | SEQ ID NO: 58 |
| Ranalexin | FLGGLIVPAMICAVTKKC | SEQ ID NO: 59 |
| Melittin | GIGAVLKVLTTGLPALISWIKRKRQQ | SEQ ID NO: 60 |
| OR-7 | | SEQ ID NO: 50 |
| Buforin I | AGRGKQGGKVRAKAKTRSSRAGLQFPVGRVHRLLRKGNY | SEQ ID NO: 61 |

In a further aspect of the present invention the fused peptide stretch is a sushi peptide which is described by Ding JL, Li P, Ho B Cell Mol Life Sci. 2008 Apr;65(7-8):1202-19. The Sushi peptides: structural characterization and mode of action against Gram-negative bacteria. Preferred sushi peptides are sushi peptides S1 and S3 and multiples thereof; FASEB J. 2000 Sep;14(12):1801-13.

In a further aspect of the present invention the fused peptide stretch is a defensin, preferably Cathelicidine, Cecropin P1, Cecropin A or Magainin II.

In a further aspect of the present invention the fused peptide stretch is a hydrophobic peptide, preferably having the amino acid sequence Phe-Phe-Val-Ala-Pro

Further preferred peptide stretches are listed in the following Table 3:

| | | |
|---|---|---|
| Alpha 4 | PNRAKRVITTFRT | SEQ ID NO: 62 |
| Artilysin1 | GFFIPAVILPSIAFLIVP | SEQ ID NO: 63 |
| Artilysin2 | GKPGWLIKKALVFKKLIRRPLKRLA | SEQ ID NO: 64 |
| WLBU2 variant | KRWVKRVKRVKRWVKRVVRVVKRWVKR | SEQ ID NO: 65 |

In another preferred embodiment of the present invention the peptide of the fusion protein according to the present invention comprise modifications and/or alterations of the amino acid sequences. Such alterations and/or modifications may comprise mutations such as deletions, insertions and additions, substitutions or combinations thereof and/or chemical changes of the amino acid residues, e.g. biotinylation, acetylation, peglyation, chemical changes of the amino-, SH- or carboxyl-groups.

A fusion protein according to the present invention as already outlined above is composed of
(a) an endolysin, fragment and/or derivative thereof according to the present invention, and
(b) a peptide fused to said endolysin, fragment and/or derivative thereof at the Nor C-Terminus, and optionally
(c) a tag, such as a His-tag, Strep-tag, Avi-tag, Myc-tag, Gst-tag, JS-tag, cystein-tag, FLAG-tag or other tags known in the art at the N- and/or C-Terminus.

The two and three components of the fusion protein, respectively, as outlined above may be linked to each other over additional amino acid residues e.g. due to cloning reasons. Moreover, the peptide may be linked to the N- and/or C-terminus of the endolysin, fragment and/or derivative thereof by additional amino acid residues. Said additional amino acid residues may be at least 1, 2, 3, 4, 5, 6, 7, 8, 9 or 10 additional amino acid residues. In a preferred embodiment of the present invention the peptide is linked to the endolysin, fragment and/or derivative according to the present invention by the additional amino acid residues Gly-Ser, Gly-Gly-Ser or Gly-Ser-Gly. The additional amino acid residues linking the N-and/or C-terminus of the endolysin, fragment and/or derivative thereof and the peptide are preferably Gly-Ser-Gly. In case the fusion protein additionally comprises a tag, the fusion protein according to the present invention is preferably linked to said tag by the additional amino acid residues Leu-Glu or Lys-Gly. Preferably the peptide comprises the amino acid methionine (Met), methionine, glycine and serine (Met-Gly-Ser) or alanine, methionine or glycine (Ala-Met-Gly).

Especially preferred is a fusion protein comprising an endolysin, fragment and/or derivative according to the present invention and a peptide having an amino acid sequence according to SEQ ID NO: 12 at the N- and/or C-terminus of the endolysin, fragment and/or derivative according to the present invention. More preferred are fusion proteins having an amino acid sequence according to SEQ ID NO: 7 and SEQ ID NO: 9. Also preferred are fusion proteins having an amino acid sequence according to SEQ ID NO: 7 and SEQ ID NO: 9 with an additional His₆-tag linked to the C- and/or N-terminus of the fusion protein. The fusion proteins having an amino acid sequence according to SEQ ID NO: 7 and SEQ ID NO: 9, respectively, with an additional C-terminal and/or N-terminal His₆-tag linked to the C- and/or N-terminus of the fusion protein by the additional amino acid residues lysine and glycine (Lys-Gly).

In case the peptide is fused to the endolysin, fragment and/or derivative thereof according to the present invention at the C-Terminus, the fusion protein comprises the additional tag preferably at the N-terminus. In an especially preferred embodiment of the present invention the peptide is fused to the endolysin, fragment and/or derivative thereof according to the present invention at the N-Terminus the fusion protein comprises an additional tag preferably at the C-terminus.

Fusion proteins are constructed by linking at least two nucleic acid sequences using standard cloning techniques as described e.g. by Sambrook et al. 2001, Molecular Cloning: A Laboratory Manual. Such a protein may be produced, e.g., in recombinant DNA expression systems. Such fusion proteins according to the present invention can be obtained by fusing the nucleic acids for endolysin, fragment and/or derivative thereof and the respective peptide.

As some fusion proteins may either be toxic upon expression in bacteria, or not homogenous due to protein degradation, the strategy might be to express these fusion proteins fused or linked to other additional proteins. Example for these other additional protein is Thioredoxin, which was shown to mediate expression of toxic antimicrobial peptides in *E.coli* (TrxA mediating fusion expression of antimicrobial peptide CM4 from multiple joined genes in Escherichia coli. Zhou L, Zhao Z, Li B, Cai Y, Zhang S. Protein Expr Purif. 2009 Apr;64(2):225-230).

For antimicrobial function of the fusion proteins according to the present invention it may be necessary to remove the additional fusion protein by proteolytic cleavage. Commercially available kits like the pET32 expression system (Novagen), may need to modify e.g. the N-terminus of the fusion depending on the protease used, like from MGS to AMGS, were the remaining alanine residue results from an introduced Enterokinase cleavage site.

In a preferred embodiment of the present invention the fusion protein consists of a peptide according to SEQ ID NO: 11 to 34 and an endolysin according to SEQ ID NO: 1, 3, 4, 5 and/or 6. In a preferred embodiment the fusion protein comprises a peptide selected from the group of peptides according to SEQ ID NO: 11 to 34 and an endolysin selected from the group of endolysins according to SEQ ID NO: 1, 3, 4, 5 and/or 6.

The following table exemplifies the above outlined assembly of specifically preferred fusion proteins according to the present invention listed in the first column starting with the starting methionine residue at the N-terminus in the second column and ending with the optional tag at the C-terminus in the last column:

**Table 4:**

| **Fusion protein according to the present invention (SEQ ID NO:)** | **First amino acid residue (N-term)** | **additional amino acid residues** | **peptide stretch (SEQ ID NO:)** | **additional amino acid residues** | **polypeptide, fragment, derivative according to the present invention (SEQ ID NO:)** | **additional amino acid residues** | **tag (C-term)** |
|---|---|---|---|---|---|---|---|
| SEQ: 7 | | Ala-Met-Gly | SEQ: 12 | - | SEQ: 6 | - | - |
| SEQ: 9 | | Ala-Met-Gly | SEQ: 12 C-terminal | Gly-Ser-Gly | SEQ: 6 | Lys-Gly | - |

The present invention further relates to an isolated nucleic acid molecule encoding the fusion protein according to the present invention. Especially preferred isolated nucleic acid molecules according to the present invention comprise a nucleic acid sequence according to SEQ ID NO: 8 and 10. The present invention further relates to a vector comprising the nucleic acid molecule according to the present invention. Said vector may provide for the constitutive or inducible expression of said fusion protein according to the present invention.

The invention also relates to a method for obtaining said fusion proteins from a micro-organism, such as a genetically modified suitable host cell which expresses said fusion proteins. Said host cell may be a micro-organism such as bacteria or yeast or an animal cell as e.g. a mammalian cell, in particular a human cell. In one embodiment of the present invention the host cell is an *Escherichia coli* cell. The host may be selected due to mere biotechnological reasons, e.g. yield, solubility, costs, etc. but may be also selected from a medical point of view, e.g. a non-pathological bacteria or yeast or human cells. Another aspect of the present invention is related to a method for genetically transforming a suitable host cell in order to obtain the expression of the fusion proteins according to the present invention, wherein the host cell is genetically modified by the introduction of a genetic material encoding said fusion proteins into the host cell and obtain their translation and expression by genetic engineering methods well known by the man skilled in the art.

The fusion proteins may be obtained from a micro-organism, such as a genetically modified suitable host cell which expresses said fusion proteins. Said host cell may be a micro-organism such as bacteria or yeast or fungi or an animal cell as e.g. a mammalian cell, in particular a human cell. In one embodiment of the present invention the yeast cell is a *Pichia pastoris* cell. The host may be selected due to mere biotechnological reasons, e.g. yield, solubility, costs, etc. but may be also selected from a medical point of view, e.g. a non-pathological bacteria or yeast, human cells.

In a further aspect the present invention relates to a composition, preferably a pharmaceutical composition, comprising a fusion protein according to the present invention and/or a host transformed with a nucleic acid molecule or a vector comprising a nucleotide sequence encoding a fusion protein according to the present invention.

The present invention also relates to fusion protein according to the present invention and/or a host transformed with a nucleic acid comprising a nucleotide sequence encoding a fusion protein according to the present invention for use as a medicament. In a further aspect the present invention relates to the use of a fusion protein according to the present invention and/or a host transformed with a vector comprising a nucleic acid molecule comprising a nucleotide sequence encoding a fusion protein according to the present invention in the manufacture of a medicament for the treatment and/or prevention of a disorder, disease or condition associated with Gram-positive bacteria, preferably staphylococcal bacteria, more preferably *Staphylococcus aureus.* In particular the treatment and/or prevention of the disorder, disease or condition may be caused by Gram-positive bacteria of bacterial groups, families, genera or species comprising strains pathogenic for humans or animals like *Staphylococcus aureus.*

In another specific embodiment of the present invention the disorder, disease or condition is caused by *Staphylococcus aureus,* in particular infections of the skin, like pyoderma, particularly folliculitis, furuncle, carbuncle, abscesses of the sweat glands and pemphigus, and like scaled skin syndrome. The scaled skin syndrome can appear in three clinical pictures: dermatitis exfoliativa, impetigo bullosa and scarlatiniform erythroderma. Moreover the disorder, disease or condition caused by *Staphylococcus aureus* is *Staphylococcus* pneumonia, hospitalism, in particular surgical wound infections, mastitis puerperalis and enterokolitis, and food poisonings.

The present invention further relates to a medicament comprising a fusion protein according to the present invention and/or a host transformed with a nucleic acid comprising a nucleotide sequence encoding a fusion protein according to the present invention.

In a further aspect the present invention relates to a method of treating a disorder, disease or condition in a subject in need of treatment and/or prevention, which method comprises administering to said subject an effective amount of a fusion protein according to the present invention and/or an effective amount of a host transformed with a nucleic acid comprising a nucleotide sequence encoding a fusion protein according to the present invention or a composition according to the present invention. The subject may be a human or an animal.

In particular said method of treatment may be for the treatment and/or prevention of infections of the skin, of soft tissues, the respiratory system, the lung, the digestive tract, the eye, the ear, the teeth, the nasopharynx, the mouth, the bones, the vagina, of wounds of bacteraemia and/or endocarditis caused by staphylococcal bacteria, in particular by the staphylococcal bacteria as listed above.

The dosage and route of administration used in a method of treatment or prophylaxis according to the present invention depends on the specific disease/site of infection to be treated. The route of administration may be for example oral, topical, nasopharyngeal, parenteral, intravenous, rectal or any other route of administration.

For application of a fusion protein according to the present invention and/or an effective amount of a host transformed with a nucleic acid comprising a nucleotide sequence encoding a fusion protein according to the present invention or a composition according to the present invention to a site of infection or site endangered to be infected a formulation may be used that protects the active compounds from environmental influences such as proteases, oxidation, immune response etc., until it reaches the site of infection. Therefore, the formulation may be capsule, dragee, pill, powder, suppository, emulsion, suspension, gel, lotion, cream, salve, injectable solution, syrup, spray, inhalant or any other medical reasonable galenic formulation. Preferably, the galenic formulation may comprise suitable carriers, stabilizers, flavourings, buffers or other suitable reagents. For example, for topical application the formulation may be a lotion, cream, gel, salve or plaster, for nasopharyngeal application the formulation may be saline solution to be applied via a spray to the nose. For oral administration in case of the treatment and/or prevention of a specific infection site e.g. in the intestine, it can be necessary to protect a fusion protein according to the present invention from the harsh digestive environment of the gastrointestinal tract until the site of infection is reached. Thus, bacteria as carrier, which survive the initial steps of digestion in the stomach and which secret later on a fusion protein according to the present invention into the intestinal environment can be used.

Preferably, a fusion protein according to the present invention is used for medical treatment, if the infection to be treated or prevented is caused by multiresistant bacterial strains, in particular by strains resistant against one or more of the following antibiotics: streptomycin, tetracycline, cephalothin, gentamicin, cefotaxime, cephalosporin, ceftazidime or imipenem. Furthermore, a fusion protein according to the present invention can be used in methods of treatment by administering it in combination with or in addition to conventional antibacterial agents, such as antibiotics, lantibiotics, bacteriocins or endolysins.

The present invention also relates to a pharmaceutical pack comprising one or more compartments, wherein at least one compartment comprises one or more fusion protein according to the present invention and/or one or more hosts transformed with a nucleic acid comprising a nucleotide sequence encoding a fusion protein according to the present invention or a composition according to the present invention.

In another aspect the present invention relates to a process of preparation of a pharmaceutical composition, said process comprising admixing one or more fusion protein according to the present invention and/or one or more hosts transformed with a nucleic acid comprising a nucleotide sequence encoding a fusion protein according to the present invention with a pharmaceutically acceptable diluent, excipient or carrier.

In an even further aspect the composition according to the present invention is a cosmetic composition. Several bacterial species can cause irritations on environmentally exposed surfaces of the patient's body such as the skin. In order to prevent such irritations or in order to eliminate minor manifestations of said bacterial pathogens, special cosmetic preparations may be employed, which comprise sufficient amounts of the fusion protein according to the present invention in order to degrade already existing or freshly settling pathogenic Gram-positive, preferably staphylococcal bacteria, more preferably *Staphylococcus aureus.*

In a further aspect the present invention relates to fusion protein according to the present invention for use as diagnostic means in medicinal, food or feed or environmental diagnostics, in particular as a diagnostic means for the diagnostic of bacteria infection caused in particular by Gram-positive bacteria, preferably staphylococcal bacteria, more preferably *Staphylococcus aureus.* In this respect the fusion protein according to the present invention may be used as a tool to specifically degrade pathogenic bacteria, in particular Gram-positive pathogenic bacteria, preferably staphylococcal bacteria, more preferably *Staphylococcus aureus.* The degradation of the bacterial cells by the fusion protein according to the present invention can be supported by the addition of detergents like Triton X-100 or other additives which weaken the bacterial cell envelope like polymyxin B. Specific cell degradation is needed as an initial step for subsequent specific detection of bacteria using nucleic acid based methods like PCR, nucleic acid hybridization or NASBA (Nucleic Acid Sequence Based Amplification), immunological methods like IMS, immunofluorescence or ELISA techniques, or other methods relying on the cellular content of the bacterial cells like enzymatic assays using proteins specific for distinct bacterial groups or species (e.g. β-galactosidase for enterobacteria, coagulase for coagulase positive strains).

In a further aspect the present invention relates to the use of the fusion protein according to the present invention for the treatment or prevention of Gram-positive bacterial, preferably staphylococcal contamination of foodstuff, of food processing equipment, of food processing plants, of feed for livestock animals, of surfaces coming into contact with foodstuff such as shelves and food deposit areas and in all other situations, where pathogenic, facultative pathogenic or other undesirable bacteria can potentially infest food material, of medical devices and of all kind of surfaces in hospitals and surgeries.

In particular, a fusion protein of the present invention may be used prophylactically as sanitizing agent. Said sanitizing agent may be used before or after surgery, or for example during hemodialysis. Moreover, premature infants and immunocompromised persons, or those subjects with need for prosthetic devices may be treated with a fusion protein according to the present invention. Said treatment may be either prophylactically or during acute infection. In the same context, nosocomial infections, especially by antibiotic resistant strains like *Methicillin-resistant Staphyloccoccus aureus (MRSA), in particular MRSA from horse, pig and poultry, Methicillin-sensitive Staphyloccoccus aureus and Oxacillin-resistant Staphylococcus aureus (ORSA)* species may be treated prophylactically or during acute phase with a fusion protein of the present invention. Therefore, a fusion protein according to the present invention may be used as a disinfectant also in combination with other ingredients useful in a disinfecting solution like detergents, tensids, solvents, antibiotics, lantibiotics, or bacteriocins.

In a preferred embodiment a fusion protein according to the present invention can be used in methods of treatments by administering it in combination with at least one of the following antibiotics: β-lactams, aminoglycosides, fluoroquinolones, macrolides, novobiocin, rifampicin, oxazolidinones, fusidic acid, mupirocin, pleuromutilins, daptomycin, vancomycin, tetracyclines, sulfonamides, chloramphenicol, trimetoprim, fosfomycin, cycloserine and polymyxin.

In another preferred embodiment a fusion protein according to the present invention can be used in methods of eliminating, reducing or preventing bacterial biofilms of *Staphylococcus aureus* by administering it in combination with at least one of the following antibiotics: β-lactams, aminoglycosides, fluoroquinolones, macrolides, novobiocin, rifampicin, oxazolidinones, fusidic acid, mupirocin, pleuromutilins, daptomycin, vancomycin, tetracyclines, sulfonamides, chloramphenicol, trimetoprim, fosfomycin and cycloserine.

For the use of the fusion protein according to the present invention for eliminating, reducing or preventing bacterial biofilms as a disinfectant e.g. in hospital, dental surgery, veterinary, kitchen or bathroom, the fusion protein can be prepared in a composition in form of e.g. a fluid, a powder, a gel, or an ingredient of a wet wipe or a disinfection sheet product. Said composition may additionally comprise suitable carrier, additives, diluting agents and/or excipients for its respective use and form, respectively, - but also agents that support the antimicrobial activity like EDTA or agents enhance the antimicrobial activity of the fusion proteins. The fusion protein may also be used with common disinfectant agents like, Alcohols, Aldehydes, Oxidizing agents, Phenolics, Quaternary ammonium compounds or UV-light. For disinfecting for example surfaces, objects and/or devices the fusion protein can be applied on said surfaces, objects and/or devices. The application may occur for instance by wetting the disinfecting composition with any means such as a cloth or rag, by spraying, pouring. The fusion proteins may be used in varying concentration depending on the respective application and the ,,reaction time" intended to obtain full antimicrobial activity.

In a further aspect the present invention relates to the use of the fusion protein according to the present invention as a food additive or feed additive for livestock animals.

Further scope of applicability of the present invention will become apparent from the detailed description given hereinafter, however, it should be understood that the detailed description and specific examples, while indicating preferred embodiments of the invention, are given by way of illustration only, since various changes and modifications within the spirit and scope of the invention will become apparent to those skilled in the art from this detailed description. It is to be understood that both the foregoing general description and the following detailed description are exemplary and explanatory only and are not restrictive of the invention, as claimed.

The following examples explain the present invention but are not considered to be limiting. Unless indicated differently, molecular biological standard methods were used, as e.g., described by Sambrock et al., 1989, Molecular Cloning: A Laboratory Manual, 2nd edition, Cold Spring Harbor Laboratory Press, Cold Spring Harbor, New York.

### Example 1: Cloning of the fusion proteins of Staphylococcus aureus phage 2638A

Ply2638A having an amino acid sequence according to SEQ ID NO: 1 is a modular endolysin of 486 amino acid residues originating from *Staphylococcus aureus* phage 2638A with putative N-terminal peptidase/amidase domains and a C-terminal peptidoglycan binding domain.

Codon optimized and synthesized gene of Ply2638A was used as a template in standard PCR reaction with KAPAHiFi™ polymerase (PEQLAB, Erlangen, Germany) using the following PCR parameters and the primers are presented in the table below:

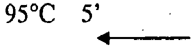

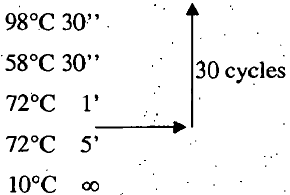

To extend the 5'- or 3'-end of Ply2638A with a fragment encoding the polycationic 9-mer peptide Lys-Arg-Lys-Lys-Arg-Lys-Lys-Arg-Lys (SEQ ID NO: 15) primers presented in table 5 were used for PCR. Ply2638A-PK additionally carries a Gly-Ser-Gly spacer between the Ply2638A and the polycationic peptide.

**Table 5: Primer sequences used for the cloning of the nucleic acids encoding the fusion proteins PK-Ply2638A and Ply2638A-PK**

| Primer | SEQ ID NO: | Nucleic acid sequence | Experiment |
|---|---|---|---|
| Ply2638A forward | SEQ ID NO: 35 | | Amplification of the Ply2638A gene |
| Ply2638A reverse | SEQ ID NO: 36 | | Amplification of the Ply2638A gene |
| PK-Ply2638A forward | SEQ ID NO: 37 | | Extension of the Ply2638A gene |
| PK-Ply2638A reverse | SEQ ID NO: 38 | | Extension of the Ply2638A gene |
| Ply2638A-PK forward | SEQ ID NO: 39 | | Extension of the Ply2638A gene |
| Ply2638A-PK reverse | SEQ ID NO: 40 | | Extension of the Ply2638A gene |

Both extended fragments were digested with NcoI and XhoI and ligated in a linearized pET32b expression vector (Merck KGaA, Darmstadt, Germany) by following standard cloning protocols. By this way a fusion protein with thioredoxin, S-tag, His-tag and thrombin/enterokinase restriction sites (all comprised in the pET32b vector) was generated.

### Example 2: Purification of the fusion proteins of Staphylococcus aureus phase 2638A

Recombinant expression of proteins is performed in exponentially growing E. coli T7 Express lysY/Iq cells (New England Biolabs, Frankfurt.a.M., Germany) after induction with 0.5 mM IPTG (isopropylthiogalactoside) at 37°C for a period of 4 hours. Proteins were pre-purified by Ni²⁺ affinity chromatography (Äkta EXPLORER, GE Healthcare) using the His-tag, encoded by the pET32b expression vector. The Ni2+ affinity chromatography is performed in 4 subsequent steps, all at room temperature:
1. Equilibration of the HiTrap IMAC FF 5 ml column (GE Healthcare) with 5 column volumes of Washing Buffer (20 mM imidazole, 1M NaCl and 20 mM HEPES; pH 7.4) at a flow rate of 5 ml/min.
2. Loading of the total lysate (with wanted endolysin/artilysin) on the HiTrap IMAC FF column at a flow rate of 5 ml/min.
3. Washing of the column with 15 column volumes of Washing Buffer at a flow rate of 5 ml/min.
4. Elution of bound protein is done with a 4 column volume gradient to 100% of Elution Buffer (500 mM imidazole, 0.5 M NaCl and 20 mM HEPES; pH 7.4) at a flow rate of 5ml/min

After pre-purification the fused protein is digested overnight with recombinant enterokinase at room temperature. Ni²⁺ affinity chromatography is performed again, with repeating step 1 - 3 of the protocol with the target protein in the flowthrough collected in step 3 (His-tagged thioredoxin bound to the column).

### Example 3: Antimicrobial activity of the fusion protein of Ply2638A with polykationic stretches on the N-terminus and C-terminus

*Staphylococcus aureus* SP8, *Staphylococcus aureus* SP10, *Staphylococcus aureus* SP17, *Staphylococcus aureus* KS13, *Staphylococcus aureus* S53 (MRSA), *Staphylococcus aureus* AV4 (MRSA), *Staphylococcus aureus* 796N (MRSA) cells (Katholieke Universiteit Leuven, Belgium) as well as *Staphylococcus aureus* DSMZ 346 and *Staphylococcus intermedius ,* DSMZ 20373 (DSMZ, Braunschweig, Germany) were used as test strains.

Overnight cultures were diluted 10-fold in fresh LB medium and grown to OD600=0.6. The culture was spun down and diluted 10-fold in dilution buffer (10 mM HEPES, pH 7.4). Bacteria were incubated at room temperature with each 10 µg fusion protein at a final concentration of 100 µg/ml in buffer (20 mM HEPES 0.5 M NaCl; pH 7.4). After 1 hour cell dilution series were made in PBS and plated on LB. Additionally, a negative control was plated using buffer (20 mM HEPES 0.5 M NaCl; pH 7.4). The residual colonies were counted after an overnight incubation at 37°C. Based on the counted cell numbers the antibacterial activity as logarithmic units (=log 10N₀/Nᵢ with No = number of untreated cells and Nᵢ = number of treated cells) was calculated. All samples were replicated at least in four fold.

**Table 6: Antimicrobial activity of the Ply2638 derivative modified with cationic peptide stretch against Staphylococcal bacteria**

| | PK-Ply2638A | Ply2638A-PK | Ply2638A | Ply2638A-PK | Ply2638A |
|---|---|---|---|---|---|
| *S*. *aureus* SP8 | + | +++ | - | 5 | - |
| *S*. *aureus* SP10 | + | +++ | - | >4 | - |
| *S*. *aureus* SP17 | + | +++ | - | 3 | 0,5 |
| *S*. *aureus* S53 (MRSDA) | n.d. | ++ | - | 3 | - |
| *S*. *aureus* AV4 (MRSDA) | n.d. | +++ | - | 4 | - |
| *S. aureus* 796N (MRSDA) | n.d. | +++ | - | 4 | - |
| *S*. *aureus* KS13 | n.d. | ++ | - | 3 | n.d. |
| *S*_{.} *aureus* DSMZ 346 | - | ++ | - | 2 | - |
| *S. intermedius* DSMZ 20373 | n.d. | - | - | - | - |
| Staphylococcus aureus ATCC 6538 | + | ++ | n.d | 2 | n.d. |

| | | | | | |
|---|---|---|---|---|---|
| Abbreviations: -: no activity measured; +: 0.5 - 1 log; ++: 2-3 log; +++: 4 or more logs; n.d.: not determined; Reduction of cell numbers is displayed in logarithmic units | | | | | |

### EXAMPLE 4: Cloning, expression and purification of Ply2638A modified with various Peptide stretches on the N-terminus or the C-terminus.

The endolysin Ply2638A is encoded by the nucleic acid sequence according to SEQ ID NO:2. The nucleic acid sequence according to SEQ ID NO:2 was synthetically produced with a BamH I (5'-GGA TCC-3') restriction site at the 5'-end of the nucleic acid sequence and an Xho I (5'-CTC GAG-3') restriction site at the 3'-end of the nucleic acid sequence.

The following peptide stretches were used for the generation of fusion proteins with Ply2638A: Pseudin 1 (SEQ ID NO:41), LL-37 (SEQ ID NO: 42), Indolicidin (SEQ ID NO: 43), Magainin 44 (SEQ ID NO:43), Pleurocidin (SEQ ID NO:44), Cecropin A (A. aegypti) (SEQ ID NO:45), Buforin II (SEQ ID NO:46), Sarcotoxin IA (SEQ ID NO:47), SMAP-29 (SEQ ID NO:49), OR-7 (SEQ ID NO:50).

The nucleic acid molecules encoding the respective peptide stretches were synthetically produced with a Nco I restriction site plus two additional nucleotides (5'-CCA TGG GC-3') at the 5'-end of the nucleic acid molecule and a BamH I (5'-GGA TCC-3') restriction site at the 3'-end of the nucleic acid molecule.

Fusion proteins were constructed by linking at least two nucleic acid sequences using standard cloning techniques as described e.g. by Sambrook et al. 2001, Molecular Cloning: A Laboratory Manual. Therefore the nucleic acid molecules encoding the peptide stretches were cleaved in a digest with the respective restriction enzymes Nco I and BamH I. Subsequently the cleaved nucleic acids encoding the peptide stretches were ligated into the pET32 b expression vector (Novagen, Darmstadt, Germany), which was also cleaved in a digest with the respective restriction enzymes Nco I and BamH I as described above. The modification of the pET32b expression vector refers to the deletion of the sequence encoding a S-tag and the central His-tag.

Subsequently, the nucleic acid molecule encoding the enzyme Ply2638A was cleaved in a digest with the restriction enzyme BamH I and Xho I and ligated into the the modified pET32 b expression vector.

The sequence of the endolysin-peptide-fusions was controlled via DNA-sequencing and correct clones were transformed into E.coli BL21(DE3) (Novagen, Darmstadt, Germany) for protein expression.

Recombinant expression of the fusion proteins was performed in *E*. *coli* BL21 (DE3) cells (Novagen, Darmstadt, Germany). The cells were growing until an optical density of OD600nm of 0.5-0.8 was reached. Then the expression of the fusion protein was induced with 1 mM IPTG (isopropylthiogalactoside) and the expression was performed at 37°C for a period of 4 hours.

E.coli BL21 cells were harvested by centrifugation for 20 min at 6000g and disrupted via sonication on ice. Soluble and insoluble fraction of the E.coli crude extract were separated by centrifugation (Sorvall, SS34, 30 min, 15 000 rpm). All proteins were purified by Ni2+ affinity chromatography (Akta FPLC, GE Healthcare) using the C-terminal 6xHis-tag, encoded by the modified pET32b vector (S-tag and central His-tag deleted), which fuses thioredoxin on the N-terminus of the proteins of interest. The vector also contains an enterokinase cleavage site (DDDDK) right before the protein of interest. This site allows the proteolytic cleavage between thioredoxin and the protein of interest, which can purified via the remaining C-terminal His-tag. Expressed fusion proteins were not toxic to the host resulting in high yields of produced protein. For antimicrobial function of the fusion protein it was necessary to remove the thioredoxin by proteolytic cleavage. Therefore the fusion protein was cleaved with 2-4 units/mg recombinant enterokinase (Novagen, Darmstadt, Germany) according to the manufactures instructions to remove the thioredoxin following the protocol provided by the manufacturer. After enterokinase cleavage the fusion protein was purified via His-tag purification as described below.

The Ni²⁺ affinity chromatography is performed in 4 subsequent steps, all at room temperature:
1. *Equilibration* of the *Histrap FF 5 ml* column (GE Healthcare) with up to 10 column volumes of Washing Buffer (20 mM imidazole, 1 M NaCl and 20 mM Hepes on pH 7.4) at a flow rate of 3-5 ml/min.
2. *Loading* of the total lysate (with wanted fusion protein) on the *Histrap FF 5 ml* column at a flow rate of 3-5 ml/min.
3. Washing of the column with up to 10 column volumes of Washing Buffer to remove unbound sample followed by a second washing step with 10% Elution buffer (500 mM imidazole, 0.5 M NaCl and 20 mM Hepes on pH 7.4) at a flow rate of 3-5 ml/min.
4. Elution of bounded fusion proteins from the column with a linear gradient of 4 column volumes of Elution Buffer (500 mM imidazole, 0.5 M NaCl and 20 mM Hepes on pH 7.4) to 100% at a flow rate of 3-5 ml/min.

Purified stock solutions of fusion proteins in Elution Buffer (20 mM Hepes pH 7.4; 0.5 M NaCl; 500 mM imidazole) were at least 90% pure as determined visually on SDS-PAGE gels.

### EXAMPLE 5: Antimicrobial activity of Ply2638A modified with various peptide stretches on the N-terminus or the C-terminus.

### Plating assay:

Exponentially growing cells of Staphylococci were taken (1 ml) harvested and resuspended in 10 mM HEPES (pH 7.4) and diluted 1:10 in 10 mM HEPES (pH7.4). Fusion proteins were diluted in 20 mM HEPES, 0.5 M NaCl (pH7.4) and incubated with the bacteria for 120 minutes at room temperature. Final concentration of the protein was about 100 µg/ml. After incubation serial dilutions (1:10) were made and bacteria were plated on appropriated agar plates. The residual colonies were counted after an overnight incubation at 37°C. Based on the counted cell numbers the antibacterial activity as logarithmic units (=log10N0/Ni with N0 = number of untreated cells and Ni = number of treated cells) was calculated (Table 4). All samples were replicated at least in four fold.

### Lysis test:

Staphylococcal cells of were grown in BHI medium until and optical density at 600nm of 0.7-1 was reached indicating exponential growth. Cells were harvested by centrifugation and resuspended in 20 mM HEPES (pH 7.4), 250 mM NaCl. Cells were resuspended at an optical density at 600nm of 1.0 and incubated with fusion proteins. Activity was measured spectrophotometrically at 600nm.

### Dot assay:

The dot assay was used test the antimicrobial activity of these fusion proteins against Staphylococcus aureus. Therefore, 200 µl overnight culture of Staphylococcus aureus, were mixed with 3 ml Top-Agar (LB with 0,75% Agar) plated and incubated until a solid agar layer is formed. Then 5 µl fusion protein solutions (ca. 500-800 µg/ml) were dotted. After overnight incubation under appropriate conditions formation of halos has to be analyzed.

### Results:

Six strains of Staphylococcus aureus were tested for their susceptibility to unmodified Ply2638A and Ply2638A-PK. Furthermore Ply2638A-PK was tested on 3 MRSA strains and Staphylococcus intermedius. Ply2638A shows no or only minor reduction in cell numbers compared to negative control without protein. Contrary to the endolysin the Artilysin shows good activity in plating assay using standard protocol (see above) with exception of Staphylococcus intermedius which was lysed neither by the endolysin nor by the Artilysin; see Table 6 above.

Table 7 below shows the results of the fusion proteins having the peptide stretch on the N-terminus of Ply2638A.

Table 8 below shows the results of the fusion proteins having the peptide stretch on the C-terminus of Ply2638A.

**Table 7**

| | LL-37-Ply2638A | SMAP-29-Ply2638A | Cecropin A-Ply2638A | Sarcotoxin IA-Ply2638A | OR-7-Ply2638A | Indolicidin-Ply2638A | Pseudinl-Ply2638A | Magainin-Ply2638A | Pleurocidin-Ply2638A | BuforinII-Ply2638A |
|---|---|---|---|---|---|---|---|---|---|---|
| *S*. *aureus* SP8 | + | ++ | - | - | + | + | - | + | + | - |
| *S*. *aureus* SP10 | + | + | + | - | - | + | - | + | - | - |
| *S*. *aureus* SP17 | - | + | + | + | + | + | - | + | + | + |
| *S*. *aureus* S53 | + | - | + | + | - | + | + | + | - | + |
| *S*. *aureus* AV4 | - | + | - | - | - | + | + | + | - | - |
| *S*. *aureus* 796N | + | + | - | - | - | + | - | + | + | - |
| *S*. *aureus* KS13 | + | + | - | - | + | + | - | - | + | + |
| *S*. *aureus* DSMZ 346 | - | + | - | - | + | + | - | - | - | - |
| *S. intermedius* DSMZ 20373 | - | - | - | - | - | - | - | - | - | - |

**Table 8**

| | Ply2638A-LL-37 | Ply2638A-SMAP-29 | Ply2638A-Cecropin A | Ply2638A-Sarcotoxin IA | Ply2638A-OR-7 | Ply2638A-Indolicidin | Ply2638A-Pseudinl | Ply2638A-Magainin | Ply2638A-Pleurocidin | Ply2638A-BuforinII |
|---|---|---|---|---|---|---|---|---|---|---|
| *S. aureus* SP8 | ++ | ++ | ++ | +++ | +++ | +++ | +++ | +++ | +++ | ++ |
| *S. aureus* SP10 | ++ | +++ | ++ | +++ | ++ | +++ | ++ | + | ++ | ++ |
| *S*. *aureus* SP17 | ++ | +++ | ++ | +++ | + | +++ | +++ | +++ | ++ | + |
| S. *aureus* S53 | +++ | ++ | + | +++ | ++ | ++ | + | ++ | +++ | ++ |
| S. *aureus* AV4 | + | +++ | ++ | +++ | + | +++ | +++ | +++ | ++ | ++ |
| *S*. *aureus* 796N | ++ | + | ++ | +++ | ++ | ++ | ++ | ++ | ++ | + |
| *S. aureus* KS13 | ++ | +++ | ++ | ++ | + | ++ | +++ | ++ | +++ | + |
| *S*. *aureus* DSMZ 346 | ++ | ++ | +++ | ++ | + | +++ | ++ | ++ | +++ | + |
| *S. intermedius* DSMZ 20373 | - | - | - | - | - | - | - | - | - | - |

| | | | | | | | | | | |
|---|---|---|---|---|---|---|---|---|---|---|
| Abbreviations: -: no activity measured; +: 0.5 -1 log; ++: 2-3 log; +++: 4 or more logs; n.d.: not determined | | | | | | | | | | |

Furthermore, the comparison of photometric lysis tests (using 30 µg/ml protein) showed that fusion proteins contrary to wild type Ply2638A have lytic activity on the test strain Staphylococcus aureus Sp17. With fusion proteins a well-defined clear cut spot appeared within 16h.

## Claims

1. A fusion protein comprising an endolysin having the amino acid sequence according to SEQ ID NO: 1, a fragment and/or derivative thereof and an additional cationic or polycationic peptide, an amphipatic peptide, a sushi peptide, a defensin, a hydrophobic peptide or an antimicrobial peptide fused to the N- and/or C-terminus of said endolysin, fragment and/or derivative thereof, wherein the fragment comprises an amino acid sequence according to SEQ ID NO: 3, 4 and/or 5, and wherein the derivative has a deletion, addition, insertion and/or substitution in the amino acid sequence according to SEQ ID NO: 1, 3, 4, 5 and/or 6, wherein the deletion is a deletion of 1, 2, 3, 4, or 5 amino acid residues and wherein the substitution is an exchange of an amino acid residue located at a certain position for a different one and wherein the derivative exhibits the lytic activity of Ply2638 endolysin.

2. The fusion protein according to claim 1 comprising additionally a tag, preferably a His₆-tag.

3. The fusion protein according to claim 1 or 2, wherein said cationic or polycationic peptide, amphipatic peptide, sushi peptide, defensin, hydrophobic peptide or antimicrobial peptide comprise about 5 to about 100 amino acid residues, in particular about 5 to 50 amino acid residues, in particular about 5 to 30 amino acid residues.

4. The fusion protein according to any one of the preceding claims wherein at least 70% of the amino acid residues comprised in said cationic and/or polycationic peptide are arginine, histidine and/or lysine residues, in particular arginine and/or lysine residues.

5. The fusion protein according to any one of the preceding claims wherein said cationic and/or polycationic peptide comprises an amino acid sequence according to SEQ ID NO: 11 to 34, said antimicrobial peptide comprises an amino acid sequence according to SEQ ID NO: 41 to 61.

6. The fusion protein according to claim 1 having the amino acid sequence according to SEQ ID NO: 66, 68, 70, 72, 74, 76, 78, 80, 82, 84, 86, 88, 90, 92, 94, 96, 98, 100, 102 or 104.

7. A fusion protein comprising a first amino acid sequence and a second or further amino acid sequences, the first amino acid sequence being a fragment of the endolysin Ply2638A according to SEQ ID NO:1, said fragment comprising an amino acid sequence according to SEQ ID NO: 3, 4 and/or 5.

8. An isolated nucleic acid molecule encoding a fusion protein according to any one of claims 1 to 7.

9. A vector comprising the nucleic acid molecule according to claim 8.

10. A host cell comprising the nucleic acid molecule according to claim 8 or the vector according to claim 9.

11. The fusion protein according to any one of claims 1 to 7 for use as human medical, veterinary medical or diagnostic substance, as an antimicrobial in food, feed additives for livestock animals, cosmetics, as disinfecting agent or in the environmental field or for use in a method for treatment or prevention of staphylococcal bacterial infections.

12. The use of the fusion protein according to any one of claims 1 to 7 for the treatment or prevention of staphylococcal bacterial contamination of foodstuff, of feed for livestock animals, of food processing equipment, of food processing plants, of surfaces coming into contact with foodstuff, of medical devices, of surfaces in hospitals and surgeries.

13. Pharmaceutical composition comprising the fusion protein according to any one of claims 1 to 7.

14. The fusion protein according to claim 1 to 7 for use according to claims 11 to 12, wherein said fusion protein is used in combination with or in addition to antibiotics.

## Patentansprüche

1. Ein Fusionsprotein umfassend ein Endolysin mit der Aminosäuresequenz gemäß SEQ ID NO: 1, eines Fragments und/oder Derivats davon und ein an den N- und/oder C-Terminus des Endolysins, Fragments und/oder Derivats davon fusioniertes zusätzliches kationisches oder polykationisches Peptid, ein amphipathisches Peptid, ein Sushi-Peptid, ein Defensin, ein hydrophobes Peptid oder ein antimikrobielles Peptid, wobei das Fragment eine Aminosäuresequenz gemäß SEQ ID NO: 3, 4 und/oder 5 umfasst, und wobei das Derivat eine Deletion, Addition, Insertion und/oder Substitution in der Aminosäuresequenz gemäß SEQ ID NO:1, 3, 4, 5 und/oder 6 aufweist, wobei die Deletion eine Deletion von 1, 2, 3, 4 oder 5 Aminosäureresten ist und wobei die Substitution ein Austausch eines an einer bestimmten Stelle befindlichen Aminosäurerests gegen einen anderen ist, und wobei das Derivat die lytische Aktivität des Ply2638-Endolysins aufweist.

2. Das Fusionsprotein nach Anspruch 1, zusätzlich umfassend eine Markierung, vorzugsweise eine His₆-Markierung.

3. Das Fusionsprotein nach Anspruch 1 oder 2, wobei das kationische oder polykationische Peptid, amphipathische Peptid, Sushi-Peptid, Defensin, hydrophobe Peptid oder antimikrobielle Peptid etwa 5 bis etwa 100 Aminosäurereste, insbesondere etwa 5 bis etwa 50 Aminosäurereste, insbesondere etwa 5 bis etwa 30 Aminosäurereste umfasst.

4. Das Fusionsprotein nach einem der vorhergehenden Ansprüche, wobei mindestens 70% der in dem kationischen und/oder polykationischen Peptid umfassten Aminosäurereste Arginin-, Histidin- und/oder Lysinreste sind, insbesondere Arginin- und/oder Lysinreste.

5. Das Fusionsprotein nach einem der vorhergehenden Ansprüche, wobei das kationische und/oder polykationische Peptid eine Aminosäuresequenz gemäß SEQ ID NO: 11 bis 34 umfasst, das antimirobielle Peptid eine Aminosäuresequenz gemäß SEQ ID NO: 41 - 61 umfasst.

6. Das Fusionsprotein nach Anspruch 1 mit der Aminosäuresequenz gemäß SEQ ID NO: 66, 68, 70, 72, 74, 76, 78, 80, 82, 84, 86, 88, 90, 92, 94, 96, 98, 100, 102 oder 104.

7. Ein Fusionsprotein, das eine erste Aminosäuresequenz und eine zweite oder weitere Aminosäuresequenzen umfasst, wobei die erste Aminosäuresequenz ein Fragment des Endolysins Ply2638A gemäß SEQ ID NO:1 ist, und das Fragment eine Aminosäuresequenz gemäß SEQ ID NO: 3, 4 und/oder 5 umfasst.

8. Ein isoliertes Nukleinsäuremolekül, das ein Fusionsprotein gemäß einem der Ansprüche 1 bis 7 kodiert.

9. Ein Vektor, der das Nukleinsäuremolekül gemäß Anspruch 8 umfasst.

10. Eine Wirtszelle, die das Nukleinsäuremolekül gemäß Anspruch 8 oder den Vektor gemäß Anspruch 9 umfasst.

11. Das Fusionsprotein nach einem der Ansprüche 1 bis 7 zur Verwendung als humanmedizinische, veterinärmedizinische oder diagnostische Substanz, als Antimikrobiotikum in Lebensmitteln, Futterzusätzen für Nutztiere, Kosmetika, als Desinfektionsmittel oder im Umweltbereich, oder zur Verwendung in einem Verfahren zur Behandlung oder Prävention von Infektionen mit Staphylokokkenbakterien.

12. Die Verwendung des Fusionsproteins nach einem der Ansprüche 1 bis 7 zur Behandlung und/oder Prävention von Kontamination mit Staphylokokkenbakterien von Lebensmitteln, von Futter für Nutztiere, von lebensmittelverarbeitenden Gerätschaften, von lebensmittelverarbeitenden Fabriken, von mit Lebensmitteln in Berührung kommenden Oberflächen, von Medizingeräten, oder von Oberflächen in Krankenhäusern und Operationssälen.

13. Pharmazeutische Zusammensetzung umfassend das Fusionsprotein nach einem der Ansprüche 1 bis 7.

14. Das Fusionsprotein gemäß Anspruch 1 bis 7 zur Verwendung gemäß den Ansprüchen 11 bis 12, wobei das Fusionsprotein in Kombination mit oder zusätzlich zu Antibiotika verwendet wird.

## Revendications

1. Protéine de fusion comprenant une endolysine ayant la séquence en acides aminés selon la SEQ ID NO : 1, un fragment et/ou dérivé de celle-ci et un peptide additionnel cationique ou polycationique, un peptide amphipatique, un peptide sushi, une defensine, un peptide hydrophobe ou un peptide antimicrobien fusionné au N- et/ou au C-terminal de ladite endolysine, fragment et/ou dérivé de celle-ci, dans laquelle le fragment comprend une séquence en acides aminés selon la SEQ ID NO : 3, 4 et/ou 5, et dans laquelle le dérivé a une délétion, addition, insertion et/ou substitution dans la séquence en acides aminés selon la SEQ ID NO: 1, 3, 4, 5 et/ou 6, dans laquelle la délétion est une délétion de 1, 2, 3, 4, ou 5 résidus d'acides aminés et dans laquelle la substitution est un échange d'un résidu d'acide aminé situé à une certaine position par un autre différent, et dans laquelle le dérivé présente l'activité lytique de l'endolysine Ply2638.

2. Protéine de fusion selon la revendication 1 comprenant en outre une étiquette, de préférence une étiquette-His₆.

3. Protéine de fusion selon la revendication 1 ou 2, dans laquelle ledit peptide cationique ou polycationique, peptide amphipathique, peptide sushi, defensine, peptide hydrophobe ou peptide antimicrobien comprend d'environ 5 à environ 100 résidus d'acides aminés, en particulier d'environ 5 à environ 50 résidus d'acides aminés, en particulier d'environ 5 à 30 résidus d'acides aminés.

4. Protéine de fusion selon l'une quelconque des revendications précédentes, dans laquelle au moins 70% des résidus d'acides aminés compris dans ledit peptide cationique et/ou polycationique sont des résidus d'arginine, d'histidine et/ou de lysine, en particulier des résidus d'arginine et/ou de lysine.

5. Protéine de fusion selon l'une quelconque des revendications précédentes, dans laquelle ledit peptide cationique et/ou polycationique comprend une séquence en acides aminés selon la SEQ ID NO : 11 à 34, ledit peptide antimicrobien comprenant une séquence en acides aminés selon la SEQ ID NO : 41 à 61.

6. Protéine de fusion selon la revendication 1 ayant la séquence en acides aminés selon la SEQ ID NO : 66, 68, 70, 72, 74, 76, 78, 80, 82, 84, 86, 88, 90, 92, 94, 96, 98, 100, 102 ou 104.

7. Protéine de fusion comprenant une première séquence en acides aminés et une seconde séquence en acides aminés ou une séquence en acides aminés supplémentaire, la première séquence en acides aminés étant un fragment de l'endolysine Ply2638A selon la SEQ ID NO : 1, ledit fragment comprenant une séquence en acides aminés selon la SEQ ID NO : 3, 4 et/ou 5.

8. Molécule d'acide nucléique isolée codant pour une protéine de fusion selon l'une quelconque des revendications 1 à 7.

9. Vecteur comprenant la molécule d'acide nucléique selon la revendication 8.

10. Cellule hôte comprenant la molécule d'acide nucléique selon la revendication 8 ou le vecteur selon la revendication 9.

11. Protéine de fusion selon l'une quelconque des revendications 1 à 7 pour son utilisation en tant que substance médicale humaine, médicale vétérinaire ou de diagnostic, comme antimicrobien dans les aliments, les additifs alimentaires pour animaux d'élevage, les cosmétiques, comme agent désinfectant ou dans le domaine de l'environnement ou pour son utilisation dans une méthode de traitement ou de prévention des infections bactériennes à staphylocoques.

12. Utilisation de la protéine de fusion selon l'une quelconque des revendications 1 à 7 pour le traitement ou la prévention de contamination bactériennes à staphylocoques de denrées alimentaires, d'aliments pour animaux d'élevage, d'équipements pour le traitement d'aliments, d'usines pour le traitement d'aliments, de surfaces entrant en contact avec des denrées alimentaires, d'appareils médicaux, de surface dans les hôpitaux et salles d'opérations.

13. Composition pharmaceutique comprenant la protéine de fusion selon l'une quelconque des revendications 1 à 7.

14. Protéine de fusion selon la revendication 1 à 7 pour son utilisation selon les revendications 11 à 12, dans laquelle ladite protéine de fusion est utilisée en combinaison ou adjoint à des antibiotiques.


### SEQUENCE LISTING

<110> LYSANDO Holding AG
<120> New Antimicrobial Agents
<130> LYS-009 PCT
<140> unknown
   <141> 2012-04-27
<150> EP 11163897.9
   <151> 2011-04-27
<160> 105
<170> PatentIn version 3.3
<210> 1
   <211> 486
   <212> PRT
   <213> unknown
<220>
   <223> Ply2638 A endolysin
<400> 1
   Met Leu Thr Ala Ile Asp Tyr Leu Thr Lys Lys Gly Trp Lys Ile Ser
   1               5                   10                  15
   Ser Asp Pro Arg Thr Tyr Asp Gly Tyr Pro Lys Asn Tyr Gly Tyr Arg
               20                  25                  30
   Asn Tyr His Glu Asn Gly Ile Asn Tyr Asp Glu Phe Cys Gly Gly Tyr
           35                  40                  45
   His Arg Ala Phe Asp Val Tyr Ser Asn Glu Thr Asn Asp Val Pro Ala
       50                  55                  60
   Val Thr Ser Gly Thr Val Ile Glu Ala Asn Asp Tyr Gly Asn Phe Gly
   65                  70                  75                  80
   Gly Thr Phe Val Ile Arg Asp Ala Asn Asp Asn Asp Trp Ile Tyr Gly
                   85                  90                  95
   His Leu Gln Arg Gly Ser Met Arg Phe Val Val Gly Asp Lys Val Asn
               100                 105                 110
   Gln Gly Asp Ile Ile Gly Leu Gln Gly Asn Ser Asn Tyr Tyr Asp Asn
           115                 120                 125
   Pro Met Ser Val His Leu His Leu Gln Leu Arg Pro Lys Asp Ala Lys
       130                 135                 140
   Lys Asp Glu Lys Ser Gln Val Cys Ser Gly Leu Ala Met Glu Lys Tyr
   145 150 155 160
   Asp Ile Thr Asn Leu Asn Ala Lys Gln Asp Lys Ser Lys Asn Gly Ser
   165 170 175
   Val Lys Glu Leu Lys His Ile Tyr Ser Asn His Ile Lys Gly Asn Lys
   180 185 190
   Ile Thr Ala Pro Lys Pro Ser Ile Gln Gly Val Val Ile His Asn Asp
   195 200 205
   Tyr Gly Ser Met Thr Pro Ser Gln Tyr Leu Pro Trp Leu Tyr Ala Arg
   210 215 220
   Glu Asn Asn Gly Thr His Val Asn Gly Trp Ala Ser Val Tyr Ala Asn
   225 230 235 240
   Arg Asn Glu Val Leu Trp Tyr His Pro Thr Asp Tyr Val Glu Trp His
   245 250 255
   Cys Gly Asn Gln Trp Ala Asn Ala Asn Leu Ile Gly Phe Glu Val Cys
   260 265 270
   Glu Ser Tyr Pro Gly Arg Ile Ser Asp Lys Leu Phe Leu Glu Asn Glu
   275 280 285
   Glu Ala Thr Leu Lys Val Ala Ala Asp Val Met Lys Ser Tyr Gly Leu
   290 295 300
   Pro Val Asn Arg Asn Thr Val Arg Leu His Asn Glu Phe Phe Gly Thr
   305 310 315 320
   Ser Cys Pro His Arg Ser Trp Asp Leu His Val Gly Lys Gly Glu Pro
   325 330 335
   Tyr Thr Thr Thr Asn Ile Asn Lys Met Lys Asp Tyr Phe Ile Lys Arg
   340 345 350
   Ile Lys His Tyr Tyr Asp Gly Gly Lys Leu Glu Val Ser Lys Ala Ala
   355 360 365
   Thr Ile Lys Gln Ser Asp Val Lys Gln Glu Val Lys Lys Gln Glu Ala
   370 375 380
   Lys Gln Ile Val Lys Ala Thr Asp Trp Lys Gln Asn Lys Asp Gly Ile
   385 390 395 400
   Trp Tyr Lys Ala Glu His Ala Ser Phe Thr Val Thr Ala Pro Glu Gly
   405 410 415
   Ile Ile Thr Arg Tyr Lys Gly Pro Trp Thr Gly His Pro Gln Ala Gly
   420 425 430
   Val Leu Gln Lys Gly Gln Thr Ile Lys Tyr Asp Glu Val Gln Lys Phe
   435 440 445
   Asp Gly His Val Trp Val Ser Trp Glu Thr Phe Glu Gly Glu Thr Val
   450 455 460
   Tyr Met Pro Val Arg Thr Trp Asp Ala Lys Thr Gly Lys Val Gly Lys
   465 470 475 480
   Leu Trp Gly Glu Ile Lys
   485
<210> 2
   <211> 1458
   <212> DNA
   <213> unknown
<220>
   <223> Ply2638A endolysin
<400> 2
   atgctgaccg caattgatta cctgaccaaa aaaggctgga aaatctcttc cgatccgcgt 60
   acctatgatg gctatccgaa aaactacggc taccgcaact accacgagaa cggtatcaac 120
   tatgatgaat tttgcggcgg ctatcaccgt gcctttgatg tttactccaa cgaaacgaac 180
   gatgtgccag cagttactag cggtactgtt atcgaggcga acgattacgg taacttcggc 240
   ggtactttcg ttatccgcga cgcgaacgac aacgattgga tctatggcca cctgcagcgc 300
   ggttccatgc gttttgttgt tggcgacaaa gtgaaccagg gcgacattat tggtctgcag 360
   ggtaacagca actattacga caacccgatg agcgttcacc tgcatctgca gctgcgtccg 420
   aaagacgcca aaaaagacga aaaaagccag gtttgctctg gtctggcgat ggagaaatac 480
   gatatcacca acctgaacgc gaaacaggac aaaagcaaaa acggctctgt caaagaactg 540
   aaacacatct actctaacca cattaaaggt aacaaaatca ccgctccgaa accgtctatt 600
   cagggtgtcg tgatccacaa cgattacggt tccatgactc caagccagta cctcccgtgg 660
   ctgtacgctc gtgaaaacaa cggtactcac gttaacggct gggcatctgt atacgccaac 720
   cgcaacgaag tactgtggta ccacccgact gattatgttg aatggcattg cggtaaccag 780
   tgggcaaacg cgaacctgat tggtttcgaa gtctgtgaat cttacccggg tcgtatttct 840
   gacaaactgt tcctggagaa cgaagaggct actctcaaag tggctgcaga cgtgatgaaa 900
   agctacggtc tgccagtaaa ccgtaacacc gttcgcctgc ataacgaatt tttcggcacc 960
   tcctgtccgc accgttcttg ggatctgcac gttggtaaag gcgaaccgta caccactacg 1020
   aacatcaaca aaatgaaaga ctacttcatc aaacgtatca aacactacta tgatggcggc 1080
   aaactggaag tatctaaagc tgcgacgatc aaacagtccg acgtcaaaca ggaggtaaaa 1140
   aaacaggagg cgaaacagat tgttaaagcc accgactgga aacagaacaa agacggcatc 1200
   tggtacaaag ctgaacacgc ttctttcacc gtgactgcac cagagggcat tatcacgcgt 1260
   tataaaggtc cgtggaccgg tcatccacag gctggtgtgc tgcagaaagg tcagacgatc 1320
   aaatatgacg aggttcagaa attcgacggt catgtatggg tatcttggga aacgtttgaa 1380
   ggtgaaacgg tctatatgcc ggtgcgcact tgggatgcga aaaccggtaa agtgggcaaa 1440
   ctgtggggtg aaatcaaa 1458
<210> 3
   <211> 68
   <212> PRT
   <213> unknown
<220>
   <223> peptidoglycan binding domain of Ply2638A endolysin
<400> 3
   Tyr Lys Ala Glu His Ala Ser Phe Thr Val Thr Ala Pro Glu Gly Ile
   1 5 10 15
   Ile Thr Arg Tyr Lys Gly Pro Trp Thr Gly His Pro Gln Ala Gly Val
   20 25 30
   Leu Gln Lys Gly Gln Thr Ile Lys Tyr Asp Glu Val Gln Lys Phe Asp
   35 40 45
   Gly His Val Trp Val Ser Trp Glu Thr Phe Glu Gly Glu Thr Val Tyr
   50 55 60
   Met Pro Val Arg
   65
<210> 4
   <211> 100
   <212> PRT
   <213> unknown
<220>
   <223> M23 peptidase domain of Ply2638A endolysin
<400> 4
   Tyr His Arg Ala Phe Asp Val Tyr Ser Asn Glu Thr Asn Asp Val Pro
   1 5 10 15
   Ala Val Thr Ser Gly Thr Val Ile Glu Ala Asn Asp Tyr Gly Asn Phe
   20 25 30
   Gly Gly Thr Phe Val Ile Arg Asp Ala Asn Asp Asn Asp Trp Ile Tyr
   35 40 45
   Gly His Leu Gln Arg Gly Ser Met Arg Phe Val Val Gly Asp Lys Val
   50 55 60
   Asn Gln Gly Asp Ile Ile Gly Leu Gln Gly Asn Ser Asn Tyr Tyr Asp
   65 70 75 80
   Asn Pro Met Ser Val His Leu His Leu Gln Leu Arg Pro Lys Asp Ala
   85 90 95
   Lys Lys Asp Glu
   100
<210> 5
   <211> 127
   <212> PRT
   <213> unknown
<220>
   <223> putative amidase domain of Ply2638A endolysin
<400> 5
   Ile Gln Gly Val Val Ile His Asn Asp Tyr Gly Ser Met Thr Pro Ser
   1 5 10 15
   Gln Tyr Leu Pro Trp Leu Tyr Ala Arg Glu Asn Asn Gly Thr His Val
   20 25 30
   Asn Gly Trp Ala Ser Val Tyr Ala Asn Arg Asn Glu Val Leu Trp Tyr
   35 40 45
   His Pro Thr Asp Tyr Val Glu Trp His Cys Gly Asn Gln Trp Ala Asn
   50 55 60
   Ala Asn Leu Ile Gly Phe Glu Val Cys Glu Ser Tyr Pro Gly Arg Ile
   65 70 75 80
   Ser Asp Lys Leu Phe Leu Glu Asn Glu Glu Ala Thr Leu Lys Val Ala
   85 90 95
   Ala Asp Val Met Lys Ser Tyr Gly Leu Pro Val Asn Arg Asn Thr Val
   100 105 110
   Arg Leu His Asn Glu Phe Phe Gly Thr Ser Cys Pro His Arg Ser
   115 120 125
<210> 6
   <211> 485
   <212> PRT
   <213> unknown
<220>
   <223> Ply2638A endolysin without Met
<400> 6
   Leu Thr Ala Ile Asp Tyr Leu Thr Lys Lys Gly Trp Lys Ile Ser Ser
   1 5 10 15
   Asp Pro Arg Thr Tyr Asp Gly Tyr Pro Lys Asn Tyr Gly Tyr Arg Asn
   20 25 30
   Tyr His Glu Asn Gly Ile Asn Tyr Asp Glu Phe Cys Gly Gly Tyr His
   35 40 45
   Arg Ala Phe Asp Val Tyr Ser Asn Glu Thr Asn Asp Val Pro Ala Val
   50 55 60
   Thr Ser Gly Thr Val Ile Glu Ala Asn Asp Tyr Gly Asn Phe Gly Gly
   65 70 75 80
   Thr Phe Val Ile Arg Asp Ala Asn Asp Asn Asp Trp Ile Tyr Gly His
   85 90 95
   Leu Gln Arg Gly Ser Met Arg Phe Val Val Gly Asp Lys Val Asn Gln
   100 105 110
   Gly Asp Ile Ile Gly Leu Gln Gly Asn Ser Asn Tyr Tyr Asp Asn Pro
   115 120 125
   Met Ser Val His Leu His Leu Gln Leu Arg Pro Lys Asp Ala Lys Lys
   130 135 140
   Asp Glu Lys Ser Gln Val Cys Ser Gly Leu Ala Met Glu Lys Tyr Asp
   145 150 155 160
   Ile Thr Asn Leu Asn Ala Lys Gln Asp Lys Ser Lys Asn Gly Ser Val
   165 170 175
   Lys Glu Leu Lys His Ile Tyr Ser Asn His Ile Lys Gly Asn Lys Ile
   180 185 190
   Thr Ala Pro Lys Pro Ser Ile Gln Gly Val Val Ile His Asn Asp Tyr
   195 200 205
   Gly Ser Met Thr Pro Ser Gln Tyr Leu Pro Trp Leu Tyr Ala Arg Glu
   210 215 220
   Asn Asn Gly Thr His Val Asn Gly Trp Ala Ser Val Tyr Ala Asn Arg
   225 230 235 240
   Asn Glu Val Leu Trp Tyr His Pro Thr Asp Tyr Val Glu Trp His Cys
   245 250 255
   Gly Asn Gln Trp Ala Asn Ala Asn Leu Ile Gly Phe Glu Val Cys Glu
   260 265 270
   Ser Tyr Pro Gly Arg Ile Ser Asp Lys Leu Phe Leu Glu Asn Glu Glu
   275 280 285
   Ala Thr Leu Lys Val Ala Ala Asp Val Met Lys Ser Tyr Gly Leu Pro
   290 295 300
   Val Asn Arg Asn Thr Val Arg Leu His Asn Glu Phe Phe Gly Thr Ser
   305 310 315 320
   Cys Pro His Arg Ser Trp Asp Leu His Val Gly Lys Gly Glu Pro Tyr
   325 330 335
   Thr Thr Thr Asn Ile Asn Lys Met Lys Asp Tyr Phe Ile Lys Arg Ile
   340 345 350
   Lys His Tyr Tyr Asp Gly Gly Lys Leu Glu Val Ser Lys Ala Ala Thr
   355 360 365
   Ile Lys Gln Ser Asp Val Lys Gln Glu Val Lys Lys Gln Glu Ala Lys
   370 375 380
   Gln Ile Val Lys Ala Thr Asp Trp Lys Gln Asn Lys Asp Gly Ile Trp
   385 390 395 400
   Tyr Lys Ala Glu His Ala Ser Phe Thr Val Thr Ala Pro Glu Gly Ile
   405 410 415
   Ile Thr Arg Tyr Lys Gly Pro Trp Thr Gly His Pro Gln Ala Gly Val
   420 425 430
   Leu Gln Lys Gly Gln Thr Ile Lys Tyr Asp Glu Val Gln Lys Phe Asp
   435 440 445
   Gly His Val Trp Val Ser Trp Glu Thr Phe Glu Gly Glu Thr Val Tyr
   450 455 460
   Met Pro Val Arg Thr Trp Asp Ala Lys Thr Gly Lys Val Gly Lys Leu
   465 470 475 480
   Trp Gly Glu Ile Lys
   485
<210> 7
   <211> 497
   <212> PRT
   <213> unknown
<220>
   <223> PK-Ply2638A
<400> 7
   Ala Met Gly Lys Arg Lys Lys Arg Lys Lys Arg Lys Leu Thr Ala Ile
   1               5                   10                  15
   Asp Tyr Leu Thr Lys Lys Gly Trp Lys Ile Ser Ser Asp Pro Arg Thr
               20                  25                  30
   Tyr Asp Gly Tyr Pro Lys Asn Tyr Gly Tyr Arg Asn Tyr His Glu Asn
           35                  40                  45
   Gly Ile Asn Tyr Asp Glu Phe Cys Gly Gly Tyr His Arg Ala Phe Asp
       50                  55                  60
   Val Tyr Ser Asn Glu Thr Asn Asp Val Pro Ala Val Thr Ser Gly Thr
   65                  70                  75                  80
   Val Ile Glu Ala Asn Asp Tyr Gly Asn Phe Gly Gly Thr Phe Val Ile
                   85                  90                  95
   Arg Asp Ala Asn Asp Asn Asp Trp Ile Tyr Gly His Leu Gln Arg Gly
               100                 105                 110
   Ser Met Arg Phe Val Val Gly Asp Lys Val Asn Gln Gly Asp Ile Ile
           115                 120                 125
   Gly Leu Gln Gly Asn Ser Asn Tyr Tyr Asp Asn Pro Met Ser Val His
       130                 135                 140
   Leu His Leu Gln Leu Arg Pro Lys Asp Ala Lys Lys Asp Glu Lys Ser
   145                 150                 155                 160
   Gln Val Cys Ser Gly Leu Ala Met Glu Lys Tyr Asp Ile Thr Asn Leu
                   165                 170                 175
   Asn Ala Lys Gln Asp Lys Ser Lys Asn Gly Ser Val Lys Glu Leu Lys
               180                 185                 190
   His Ile Tyr Ser Asn His Ile Lys Gly Asn Lys Ile Thr Ala Pro Lys
           195                 200                 205
   Pro Ser Ile Gln Gly Val Val Ile His Asn Asp Tyr Gly Ser Met Thr
       210                 215                 220
   Pro Ser Gln Tyr Leu Pro Trp Leu Tyr Ala Arg Glu Asn Asn Gly Thr
   225                 230                 235                 240
   His Val Asn Gly Trp Ala Ser Val Tyr Ala Asn Arg Asn Glu Val Leu
                   245                 250                 255
   Trp Tyr His Pro Thr Asp Tyr Val Glu Trp His Cys Gly Asn Gln Trp
               260                 265                 270
   Ala Asn Ala Asn Leu Ile Gly Phe Glu Val Cys Glu Ser Tyr Pro Gly
           275                 280                 285
   Arg Ile Ser Asp Lys Leu Phe Leu Glu Asn Glu Glu Ala Thr Leu Lys
       290                 295                 300
   Val Ala Ala Asp Val Met Lys Ser Tyr Gly Leu Pro Val Asn Arg Asn
   305                 310                 315                 320
   Thr Val Arg Leu His Asn Glu Phe Phe Gly Thr Ser Cys Pro His Arg
                   325                 330                 335
   Ser Trp Asp Leu His Val Gly Lys Gly Glu Pro Tyr Thr Thr Thr Asn
               340                 345                 350
   Ile Asn Lys Met Lys Asp Tyr Phe Ile Lys Arg Ile Lys His Tyr Tyr
           355                 360                 365
   Asp Gly Gly Lys Leu Glu Val Ser Lys Ala Ala Thr Ile Lys Gln Ser
       370                 375                 380
   Asp Val Lys Gln Glu Val Lys Lys Gln Glu Ala Lys Gln Ile Val Lys
   385                 390                 395                 400
   Ala Thr Asp Trp Lys Gln Asn Lys Asp Gly Ile Trp Tyr Lys Ala Glu
   405 410 415
   His Ala Ser Phe Thr Val Thr Ala Pro Glu Gly Ile Ile Thr Arg Tyr
   420 425 430
   Lys Gly Pro Trp Thr Gly His Pro Gln Ala Gly Val Leu Gln Lys Gly
   435 440 445
   Gln Thr Ile Lys Tyr Asp Glu Val Gln Lys Phe Asp Gly His Val Trp
   450 455 460
   Val Ser Trp Glu Thr Phe Glu Gly Glu Thr Val Tyr Met Pro Val Arg
   465 470 475 480
   Thr Trp Asp Ala Lys Thr Gly Lys Val Gly Lys Leu Trp Gly Glu Ile
   485 490 495
   Lys
<210> 8
   <211> 1491
   <212> DNA
   <213> unknown
<220>
   <223> PK-Ply2638A
<400> 8
   gccatgggca agcgtaagaa acgcaaaaaa cgcaaactga ccgcaattga ttacctgacc 60
   aaaaaaggct ggaaaatctc ttccgatccg cgtacctatg atggctatcc gaaaaactac 120
   ggctaccgca actaccacga gaacggtatc aactatgatg aattttgcgg cggctatcac 180
   cgtgcctttg atgtttactc caacgaaacg aacgatgtgc cagcagttac tagcggtact 240
   gttatcgagg cgaacgatta cggtaacttc ggcggtactt tcgttatccg cgacgcgaac 300
   gacaacgatt ggatctatgg ccacctgcag cgcggttcca tgcgttttgt tgttggcgac 360
   aaagtgaacc agggcgacat tattggtctg cagggtaaca gcaactatta cgacaacccg 420
   atgagcgttc acctgcatct gcagctgcgt ccgaaagacg ccaaaaaaga cgaaaaaagc 480
   caggtttgct ctggtctggc gatggagaaa tacgatatca ccaacctgaa cgcgaaacag 540
   gacaaaagca aaaacggctc tgtcaaagaa ctgaaacaca tctactctaa ccacattaaa 600
   ggtaacaaaa tcaccgctcc gaaaccgtct attcagggtg tcgtgatcca caacgattac 660
   ggttccatga ctccaagcca gtacctcccg tggctgtacg ctcgtgaaaa caacggtact 720
   cacgttaacg gctgggcatc tgtatacgcc aaccgcaacg aagtactgtg gtaccacccg 780
   actgattatg ttgaatggca ttgcggtaac cagtgggcaa acgcgaacct gattggtttc 840
   gaagtctgtg aatcttaccc gggtcgtatt tctgacaaac tgttcctgga gaacgaagag 900
   gctactctca aagtggctgc agacgtgatg aaaagctacg gtctgccagt aaaccgtaac 960
   accgttcgcc tgcataacga atttttcggc acctcctgtc cgcaccgttc ttgggatctg 1020
   cacgttggta aaggcgaacc gtacaccact acgaacatca acaaaatgaa agactacttc 1080
   atcaaacgta tcaaacacta ctatgatggc ggcaaactgg aagtatctaa agctgcgacg 1140
   atcaaacagt ccgacgtcaa acaggaggta aaaaaacagg aggcgaaaca gattgttaaa 1200
   gccaccgact ggaaacagaa caaagacggc atctggtaca aagctgaaca cgcttctttc 1260
   accgtgactg caccagaggg cattatcacg cgttataaag gtccgtggac cggtcatcca 1320
   caggctggtg tgctgcagaa aggtcagacg atcaaatatg acgaggttca gaaattcgac 1380
   ggtcatgtat gggtatcttg ggaaacgttt gaaggtgaaa cggtctatat gccggtgcgc 1440
   acttgggatg cgaaaaccgg taaagtgggc aaactgtggg gtgaaatcaa a 1491
<210> 9
   <211> 500
   <212> PRT
   <213> unknown
<220>
   <223> Ply2638A-PK
<400> 9
   Ala Met Gly Leu Thr Ala Ile Asp Tyr Leu Thr Lys Lys Gly Trp Lys
   1               5                   10                  15
   Ile Ser Ser Asp Pro Arg Thr Tyr Asp Gly Tyr Pro Lys Asn Tyr Gly
               20                  25                  30
   Tyr Arg Asn Tyr His Glu Asn Gly Ile Asn Tyr Asp Glu Phe Cys Gly
           35                  40                  45
   Gly Tyr His Arg Ala Phe Asp Val Tyr Ser Asn Glu Thr Asn Asp Val
       50                  55                  60
   Pro Ala Val Thr Ser Gly Thr Val Ile Glu Ala Asn Asp Tyr Gly Asn
   65                  70                  75                  80
   Phe Gly Gly Thr Phe Val Ile Arg Asp Ala Asn Asp Asn Asp Trp Ile
                   85                  90                  95
   Tyr Gly His Leu Gln Arg Gly Ser Met Arg Phe Val Val Gly Asp Lys
               100                 105                 110
   Val Asn Gln Gly Asp Ile Ile Gly Leu Gln Gly Asn Ser Asn Tyr Tyr
   115 120 125
   Asp Asn Pro Met Ser Val His Leu His Leu Gln Leu Arg Pro Lys Asp
   130 135 140
   Ala Lys Lys Asp Glu Lys Ser Gln Val Cys Ser Gly Leu Ala Met Glu
   145 150 155 160
   Lys Tyr Asp Ile Thr Asn Leu Asn Ala Lys Gln Asp Lys Ser Lys Asn
   165 170 175
   Gly Ser Val Lys Glu Leu Lys His Ile Tyr Ser Asn His Ile Lys Gly
   180 185 190
   Asn Lys Ile Thr Ala Pro Lys Pro Ser Ile Gln Gly Val Val Ile His
   195 200 205
   Asn Asp Tyr Gly Ser Met Thr Pro Ser Gln Tyr Leu Pro Trp Leu Tyr
   210 215 220
   Ala Arg Glu Asn Asn Gly Thr His Val Asn Gly Trp Ala Ser Val Tyr
   225 230 235 240
   Ala Asn Arg Asn Glu Val Leu Trp Tyr His Pro Thr Asp Tyr Val Glu
   245 250 255
   Trp His Cys Gly Asn Gln Trp Ala Asn Ala Asn Leu Ile Gly Phe Glu
   260 265 270
   Val Cys Glu Ser Tyr Pro Gly Arg Ile Ser Asp Lys Leu Phe Leu Glu
   275 280 285
   Asn Glu Glu Ala Thr Leu Lys Val Ala Ala Asp Val Met Lys Ser Tyr
   290 295 300
   Gly Leu Pro Val Asn Arg Asn Thr Val Arg Leu His Asn Glu Phe Phe
   305 310 315 320
   Gly Thr Ser Cys Pro His Arg Ser Trp Asp Leu His Val Gly Lys Gly
   325 330 335
   Glu Pro Tyr Thr Thr Thr Asn Ile Asn Lys Met Lys Asp Tyr Phe Ile
   340 345 350
   Lys Arg Ile Lys His Tyr Tyr Asp Gly Gly Lys Leu Glu Val Ser Lys
   355 360 365
   Ala Ala Thr Ile Lys Gln Ser Asp Val Lys Gln Glu Val Lys Lys Gln
   370 375 380
   Glu Ala Lys Gln Ile Val Lys Ala Thr Asp Trp Lys Gln Asn Lys Asp
   385 390 395 400
   Gly Ile Trp Tyr Lys Ala Glu His Ala Ser Phe Thr Val Thr Ala Pro
   405 410 415
   Glu Gly Ile Ile Thr Arg Tyr Lys Gly Pro Trp Thr Gly His Pro Gln
   420 425 430
   Ala Gly Val Leu Gln Lys Gly Gln Thr Ile Lys Tyr Asp Glu Val Gln
   435 440 445
   Lys Phe Asp Gly His Val Trp Val Ser Trp Glu Thr Phe Glu Gly Glu
   450 455 460
   Thr Val Tyr Met Pro Val Arg Thr Trp Asp Ala Lys Thr Gly Lys Val
   465 470 475 480
   Gly Lys Leu Trp Gly Glu Ile Lys Gly Ser Gly Lys Arg Lys Lys Arg
   485 490 495
   Lys Lys Arg Lys
   500
<210> 10
   <211> 1500
   <212> DNA
   <213> unknown
<220>
   <223> Ply2638A-PK
<400> 10
   gccatgggcc tgaccgcaat tgattacctg accaaaaaag gctggaaaat ctcttccgat 60
   ccgcgtacct atgatggcta tccgaaaaac tacggctacc gcaactacca cgagaacggt 120
   atcaactatg atgaattttg cggcggctat caccgtgcct ttgatgttta ctccaacgaa 180
   acgaacgatg tgccagcagt tactagcggt actgttatcg aggcgaacga ttacggtaac 240
   ttcggcggta ctttcgttat ccgcgacgcg aacgacaacg attggatcta tggccacctg 300
   cagcgcggtt ccatgcgttt tgttgttggc gacaaagtga accagggcga cattattggt 360
   ctgcagggta acagcaacta ttacgacaac ccgatgagcg ttcacctgca tctgcagctg 420
   cgtccgaaag acgccaaaaa agacgaaaaa agccaggttt gctctggtct ggcgatggag 480
   aaatacgata tcaccaacct gaacgcgaaa caggacaaaa gcaaaaacgg ctctgtcaaa 540
   gaactgaaac acatctactc taaccacatt aaaggtaaca aaatcaccgc tccgaaaccg 600
   tctattcagg gtgtcgtgat ccacaacgat tacggttcca tgactccaag ccagtacctc 660
   ccgtggctgt acgctcgtga aaacaacggt actcacgtta acggctgggc atctgtatac 720
   gccaaccgca acgaagtact gtggtaccac ccgactgatt atgttgaatg gcattgcggt 780
   aaccagtggg caaacgcgaa cctgattggt ttcgaagtct gtgaatctta cccgggtcgt 840
   atttctgaca aactgttcct ggagaacgaa gaggctactc tcaaagtggc tgcagacgtg 900
   atgaaaagct acggtctgcc agtaaaccgt aacaccgttc gcctgcataa cgaatttttc 960
   ggcacctcct gtccgcaccg ttcttgggat ctgcacgttg gtaaaggcga accgtacacc 1020
   actacgaaca tcaacaaaat gaaagactac ttcatcaaac gtatcaaaca ctactatgat 1080
   ggcggcaaac tggaagtatc taaagctgcg acgatcaaac agtccgacgt caaacaggag 1140
   gtaaaaaaac aggaggcgaa acagattgtt aaagccaccg actggaaaca gaacaaagac 1200
   ggcatctggt acaaagctga acacgcttct ttcaccgtga ctgcaccaga gggcattatc 1260
   acgcgttata aaggtccgtg gaccggtcat ccacaggctg gtgtgctgca gaaaggtcag 1320
   acgatcaaat atgacgaggt tcagaaattc gacggtcatg tatgggtatc ttgggaaacg 1380
   tttgaaggtg aaacggtcta tatgccggtg cgcacttggg atgcgaaaac cggtaaagtg 1440
   ggcaaactgt ggggtgaaat caaagggtcg ggaaagcgta agaaacgcaa aaaacgcaaa 1500
<210> 11
   <211> 6
   <212> PRT
   <213> unknown
<220>
   <223> synthetic peptide
<400> 11
   Lys Arg Lys Lys Arg Lys
   1 5
<210> 12
   <211> 9
   <212> PRT
   <213> unknown
<220>
   <223> synthetic peptide
<400> 12
   Lys Arg Lys Lys Arg Lys Lys Arg Lys
   1 5
<210> 13
   <211> 9
   <212> PRT
   <213> unknown
<220>
   <223> synthetic peptide
<400> 13
   Arg Arg Arg Arg Arg Arg Arg Arg Arg
   1 5
<210> 14
   <211> 8
   <212> PRT
   <213> unknown
<220>
   <223> synthetic peptide
<400> 14
   Lys Lys Lys Lys Lys Lys Lys Lys
   1 5
<210> 15
   <211> 10
   <212> PRT
   <213> unknown
<220>
   <223> synthetic peptide
<400> 15
   Lys Arg Lys Lys Arg Lys Lys Arg Lys Lys
   1 5 10
<210> 16
   <211> 12
   <212> PRT
   <213> unknown
<220>
   <223> synthetic peptide
<400> 16
   Lys Arg Lys Lys Arg Lys Lys Arg Lys Lys Arg Lys
   1 5 10
<210> 17
   <211> 14
   <212> PRT
   <213> unknown
<220>
   <223> synthetic peptide
<400> 17
   Lys Arg Lys Lys Arg Lys Lys Arg Lys Lys Arg Lys Lys Arg
   1               5                   10
<210> 18
   <211> 16
   <212> PRT
   <213> unknown
<220>
   <223> synthetic peptide
<400> 18
   Lys Lys Lys Lys Lys Lys Lys Lys Lys Lys Lys Lys Lys Lys Lys Lys
   1               5                   10                  15
<210> 19
   <211> 19
   <212> PRT
   <213> unknown
<220>
   <223> synthetic peptide
<400> 19
   Lys Arg Lys Lys Arg Lys Lys Arg Lys Lys Arg Lys Lys Arg Lys Lys
   1               5                   10                  15
   Arg Lys Lys
<210> 20
   <211> 19
   <212> PRT
   <213> unknown
<220>
   <223> synthetic peptide
<400> 20
   Arg Arg Arg Arg Arg Arg Arg Arg Arg Arg Arg Arg Arg Arg Arg Arg
   1               5                   10                  15
   Arg Arg Arg
<210> 21
   <211> 19
   <212> PRT
   <213> unknown
<220>
   <223> synthetic peptide
<400> 21
   Lys Lys Lys Lys Lys Lys Lys Lys Lys Lys Lys Lys Lys Lys Lys Lys
   1 5 10 15
   Lys Lys Lys
<210> 22
   <211> 20
   <212> PRT
   <213> unknown
<220>
   <223> synthetic peptide
<400> 22
   Lys Arg Lys Lys Arg Lys Lys Arg Lys Arg Ser Lys Arg Lys Lys Arg
   1 5 10 15
   Lys Lys Arg Lys
   20
<210> 23
   <211> 21
   <212> PRT
   <213> unknown
<220>
   <223> synthetic peptide
<400> 23
   Lys Arg Lys Lys Arg Lys Lys Arg Lys Arg Ser Lys Arg Lys Lys Arg
   1 5 10 15
   Lys Lys Arg Lys Lys
   20
<210> 24
   <211> 21
   <212> PRT
   <213> unknown
<220>
   <223> synthetic peptide
<400> 24
   Lys Arg Lys Lys Arg Lys Lys Arg Lys Lys Arg Lys Lys Arg Lys Lys
   1 5 10 15
   Arg Lys Lys Arg Lys
   20
<210> 25
   <211> 22
   <212> PRT
   <213> unknown
<220>
   <223> synthetic peptide
<400> 25
   Lys Arg Lys Lys Arg Lys Lys Arg Lys Arg Gly Ser Gly Lys Arg Lys
   1               5                   10                  15
   Lys Arg Lys Lys Arg Lys
               20
<210> 26
   <211> 24
   <212> PRT
   <213> unknown
<220>
   <223> synthetic peptide
<400> 26
   Lys Arg Lys Lys Arg Lys Lys Arg Lys Arg Gly Ser Gly Ser Gly Lys
   1               5                   10                  15
   Arg Lys Lys Arg Lys Lys Arg Lys
               20
<210> 27
   <211> 25
   <212> PRT
   <213> unknown
<220>
   <223> synthetic peptide
<400> 27
   Lys Arg Lys Lys Arg Lys Lys Arg Lys Lys Arg Lys Lys Arg Lys Lys
   1               5                   10                  15
   Arg Lys Lys Arg Lys Lys Arg Lys Lys
               20                  25
<210> 28
   <211> 31
   <212> PRT
   <213> unknown
<220>
   <223> synthetic peptide
<400> 28
   Lys Arg Lys Lys Arg Lys Lys Arg Lys Arg Ser Lys Arg Lys Lys Arg
   1 5 10 15
   Lys Lys Arg Lys Arg Ser Lys Arg Lys Lys Arg Lys Lys Arg Lys
   20 25 30
<210> 29
   <211> 38
   <212> PRT
   <213> unknown
<220>
   <223> synthetic peptide
<400> 29
   Lys Arg Lys Lys Arg Lys Lys Arg Lys Arg Gly Ser Gly Ser Gly Lys
   1 5 10 15
   Arg Lys Lys Arg Lys Lys Arg Lys Gly Ser Gly Ser Gly Lys Arg Lys
   20 25 30
   Lys Arg Lys Lys Arg Lys
   35
<210> 30
   <211> 39
   <212> PRT
   <213> unknown
<220>
   <223> synthetic peptide
<400> 30
   Lys Arg Lys Lys Arg Lys Lys Arg Lys Lys Arg Lys Lys Arg Lys Lys
   1 5 10 15
   Arg Lys Lys Arg Lys Lys Arg Lys Lys Arg Lys Lys Arg Lys Lys Arg
   20 25 30
   Lys Lys Arg Lys Lys Arg Lys
   35
<210> 31
   <211> 42
   <212> PRT
   <213> unknown
<220>
   <223> synthetic peptide
<400> 31
   Lys Arg Lys Lys Arg Lys Lys Arg Lys Arg Ser Lys Arg Lys Lys Arg
   1               5                   10                  15
   Lys Lys Arg Lys Arg Ser Lys Arg Lys Lys Arg Lys Lys Arg Lys Arg
               20                  25                  30
   Ser Lys Arg Lys Lys Arg Lys Lys Arg Lys
           35                  40
<210> 32
   <211> 5
   <212> PRT
   <213> unknown
<220>
   <223> synthetic peptide
<220>
   <221> misc_feature
   <222> (3)..(3)
   <223> wherein Xaa is any other amino acid residue than lysine, arginine and histidine
<400> 32
   Lys Arg Xaa Lys Arg
   1               5
<210> 33
   <211> 5
   <212> PRT
   <213> unknown
<220>
   <223> synthetic peptide
<400> 33
   Lys Arg Ser Lys Arg
   1               5
<210> 34
   <211> 5
   <212> PRT
   <213> unknown
<220>
   <223> synthetic peptide
<400> 34
   Lys Arg Gly Ser Gly
   1               5
<210> 35
   <211> 36
   <212> DNA
   <213> unknown
<220>
   <223> Ply2638A forward primer
<400> 35
   catgccatgg gcctgaccgc aattgattac ctgacc 36
<210> 36
   <211> 40
   <212> DNA
   <213> unknown
<220>
   <223> Ply2638A reverse primer
<400> 36
   ccgctcgagt catcatttga tttcacccca cagtttgccc 40
<210> 37
   <211> 63
   <212> DNA
   <213> unknown
<220>
   <223> PK-Ply2638A forward primer
<400> 37
   catgccatgg gcaagcgtaa gaaacgcaaa aaacgcaaac tgaccgcaat tgattacctg 60
   acc 63
<210> 38
   <211> 40
   <212> DNA
   <213> unknown
<220>
   <223> PK-Ply2638A reverse primer
<400> 38
   ccgctcgagt catcatttga tttcacccca cagtttgccc 40
<210> 39
   <211> 36
   <212> DNA
   <213> unknown
<220>
   <223> Ply2638A-PK forward primer
<400> 39
   catgccatgg gcctgaccgc aattgattac ctgacc 36
<210> 40
   <211> 74
   <212> DNA
   <213> unknown
<220>
   <223> Ply2638A-PK reverse primer
<400> 40
   ccgctcgagt catcatttgc gttttttgcg tttcttacgc tttcccgacc ctttgatttc 60
   accccacagt ttgc 74
<210> 41
   <211> 24
   <212> PRT
   <213> unknown
<220>
   <223> Pseudin 1
<400> 41
   Gly Leu Asn Thr Leu Lys Lys Val Phe Gln Gly Leu His Glu Ala Ile
   1 5 10 15
   Lys Leu Ile Asn Asn His Val Gln
   20
<210> 42
   <211> 37
   <212> PRT
   <213> unknown
<220>
   <223> All-37
<400> 42
   Leu Leu Gly Asp Phe Phe Arg Lys Ser Lys Glu Lys Ile Gly Lys Glu
   1 5 10 15
   Phe Lys Arg Ile Val Gln Arg Ile Lys Asp Phe Leu Arg Asn Leu Val
   20 25 30
   Pro Arg Thr Glu Ser
   35
<210> 43
   <211> 13
   <212> PRT
   <213> unknown
<220>
   <223> Indolicidin
<400> 43
   Ile Leu Pro Trp Lys Trp Pro Trp Trp Pro Trp Arg Arg
   1 5 10
<210> 44
   <211> 23
   <212> PRT
   <213> unknown
<220>
   <223> Magainin
<400> 44
   Gly Ile Gly Lys Phe Leu His Ser Ala Lys Lys Phe Gly Lys Ala Phe
   1 5 10 15
   Val Gly Glu Ile Met Asn Ser
   20
<210> 45
   <211> 25
   <212> PRT
   <213> unknown
<220>
   <223> Pleurocidin
<400> 45
   Gly Trp Gly Ser Phe Phe Lys Lys Ala Ala His Val Gly Lys His Val
   1 5 10 15
   Gly Lys Ala Ala Leu Thr His Tyr Leu
   20 25
<210> 46
   <211> 36
   <212> PRT
   <213> unknown
<220>
   <223> Cecropin A (A.aegypti)
<400> 46
   Gly Gly Leu Lys Lys Leu Gly Lys Lys Leu Glu Gly Ala Gly Lys Arg
   1 5 10 15
   Val Phe Asn Ala Ala Glu Lys Ala Leu Pro Val Val Ala Gly Ala Lys
   20 25 30
   Ala Leu Arg Lys
   35
<210> 47
   <211> 21
   <212> PRT
   <213> unknown
<220>
   <223> Buforin II
<400> 47
   Thr Arg Ser Ser Arg Ala Gly Leu Gln Phe Pro Val Gly Arg Val His
   1 5 10 15
   Arg Leu Leu Arg Lys
   20
<210> 48
   <211> 39
   <212> PRT
   <213> unknown
<220>
   <223> Sarcotoxin IA
<400> 48
   Gly Trp Leu Lys Lys Ile Gly Lys Lys Ile Glu Arg Val Gly Gln His
   1 5 10 15
   Thr Arg Asp Ala Thr Ile Gln Gly Leu Gly Ile Ala Gln Gln Ala Ala
   20 25 30
   Asn Val Ala Ala Thr Ala Arg
   35
<210> 49
   <211> 29
   <212> PRT
   <213> unknown
<220>
   <223> SMAP-29
<400> 49
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly
   20 25
<210> 50
   <211> 54
   <212> PRT
   <213> unknown
<220>
   <223> OR-7
<400> 50
   Lys Thr Tyr Tyr Gly Thr Asn Gly Val His Cys Thr Lys Asn Ser Leu
   1 5 10 15
   Trp Gly Lys Val Arg Leu Lys Asn Met Lys Tyr Asp Gln Asn Thr Thr
   20 25 30
   Tyr Met Gly Arg Leu Gln Asp Ile Leu Leu Gly Trp Ala Thr Gly Ala
   35 40 45
   Phe Gly Lys Thr Phe His
   50
<210> 51
   <211> 18
   <212> PRT
   <213> unknown
<220>
   <223> Protegrin
<400> 51
   Arg Gly Gly Arg Leu Cys Tyr Cys Arg Arg Arg Phe Cys Val Cys Val
   1 5 10 15
   Gly Arg
<210> 52
   <211> 31
   <212> PRT
   <213> unknown
<220>
   <223> Cecropin P1
<400> 52
   Ser Trp Leu Ser Lys Thr Ala Lys Lys Leu Glu Asn Ser Ala Lys Lys
   1 5 10 15
   Arg Ile Ser Glu Gly Ile Ala Ile Ala Ile Gln Gly Gly Pro Arg
   20 25 30
<210> 53
   <211> 40
   <212> PRT
   <213> unknown
<220>
   <223> Cecropin A (D. melanogaster)
<400> 53
   Gly Trp Leu Lys Lys Ile Gly Lys Lys Ile Glu Arg Val Gly Gln His
   1 5 10 15
   Thr Arg Asp Ala Thr Ile Gln Gly Leu Gly Ile Pro Gln Gln Ala Ala
   20 25 30
   Asn Val Ala Ala Thr Ala Arg Gly
   35 40
<210> 54
   <211> 24
   <212> PRT
   <213> unknown
<220>
   <223> Ascaphine
<400> 54
   Gly Ile Lys Asp Trp Ile Lys Gly Ala Ala Lys Lys Leu Ile Lys Thr
   1 5 10 15
   Val Ala Ser His Ile Ala Asn Gln
   20
<210> 55
   <211> 17
   <212> PRT
   <213> unknown
<220>
   <223> Apidaecine
<400> 55
   Ala Asn Arg Pro Val Tyr Ile Pro Pro Pro Arg Pro Pro His Pro Arg
   1 5 10 15
   Leu
<210> 56
   <211> 22
   <212> PRT
   <213> unknown
<220>
   <223> Nigrocine
<400> 56
   Gly Leu Leu Ser Lys Val Leu Gly Val Gly Lys Lys Val Leu Cys Gly
   1 5 10 15
   Val Ser Gly Leu Val Cys
   20
<210> 57
   <211> 19
   <212> PRT
   <213> unknown
<220>
   <223> Parasin 1
<400> 57
   Lys Gly Arg Gly Lys Gln Gly Gly Lys Val Arg Ala Lys Ala Lys Thr
   1 5 10 15
   Arg Ser Ser
<210> 58
   <211> 25
   <212> PRT
   <213> unknown
<220>
   <223> Lycotoxin
<400> 58
   Ile Trp Leu Thr Ala Leu Lys Phe Leu Gly Lys His Ala Ala Lys Lys
   1 5 10 15
   Leu Ala Lys Gln Gln Leu Ser Lys Leu
   20 25
<210> 59
   <211> 18
   <212> PRT
   <213> unknown
<220>
   <223> Ranalexin
<400> 59
   Phe Leu Gly Gly Leu Ile Val Pro Ala Met Ile Cys Ala Val Thr Lys
   1 5 10 15
   Lys Cys
<210> 60
   <211> 26
   <212> PRT
   <213> unknown
<220>
   <223> Melittin
<400> 60
   Gly Ile Gly Ala Val Leu Lys Val Leu Thr Thr Gly Leu Pro Ala Leu
   1 5 10 15
   Ile Ser Trp Ile Lys Arg Lys Arg Gln Gln
   20 25
<210> 61
   <211> 38
   <212> PRT
   <213> unknown
<220>
   <223> Buforin I
<400> 61
   Gly Arg Gly Lys Gln Gly Gly Lys Val Arg Ala Lys Ala Lys Thr Arg
   1 5 10 15
   Ser Ser Arg Ala Gly Leu Gln Phe Pro Val Gly Arg Val His Arg Leu
   20 25 30
   Leu Arg Lys Gly Asn Tyr
   35
<210> 62
   <211> 13
   <212> PRT
   <213> unknown
<220>
   <223> Alpha 4
<400> 62
   Pro Asn Arg Ala Lys Arg Val Ile Thr Thr Phe Arg Thr
   1 5 10
<210> 63
   <211> 18
   <212> PRT
   <213> unknown
<220>
   <223> Artilysinl
<400> 63
   Gly Phe Phe Ile Pro Ala Val Ile Leu Pro Ser Ile Ala Phe Leu Ile
   1 5 10 15
   Val Pro
<210> 64
   <211> 25
   <212> PRT
   <213> unknown
<220>
   <223> Artilysin2
<400> 64
   Gly Lys Pro Gly Trp Leu Ile Lys Lys Ala Leu Val Phe Lys Lys Leu
   1 5 10 15
   Ile Arg Arg Pro Leu Lys Arg Leu Ala
   20 25
<210> 65
   <211> 27
   <212> PRT
   <213> unknown
<220>
   <223> WLBU2 variant
<400> 65
   Lys Arg Trp Val Lys Arg Val Lys Arg Val Lys Arg Trp Val Lys Arg
   1 5 10 15
   Val Val Arg Val Val Lys Arg Trp Val Lys Arg
   20 25
<210> 66
   <211> 526
   <212> PRT
   <213> artificial
<220>
   <223> LL-37-Ply2638A
<400> 66
   Met Leu Leu Gly Asp Phe Phe Arg Lys Ser Lys Glu Lys Ile Gly Lys
   1 5 10 15
   Glu Phe Lys Arg Ile Val Gln Arg Ile Lys Asp Phe Leu Arg Asn Leu
   20 25 30
   Val Pro Arg Thr Glu Ser Gly Ser Met Leu Thr Ala Ile Asp Tyr Leu
   35 40 45
   Thr Lys Lys Gly Trp Lys Ile Ser Ser Asp Pro Arg Thr Tyr Asp Gly
       50                  55                  60
   Tyr Pro Lys Asn Tyr Gly Tyr Arg Asn Tyr His Glu Asn Gly Ile Asn
   65                  70                  75                  80
   Tyr Asp Glu Phe Cys Gly Gly Tyr His Arg Ala Phe Asp Val Tyr Ser
                   85                  90                  95
   Asn Glu Thr Asn Asp Val Pro Ala Val Thr Ser Gly Thr Val Ile Glu
               100                 105                 110
   Ala Asn Asp Tyr Gly Asn Phe Gly Gly Thr Phe Val Ile Arg Asp Ala
           115                 120                 125
   Asn Asp Asn Asp Trp Ile Tyr Gly His Leu Gln Arg Gly Ser Met Arg
       130                 135                 140
   Phe Val Val Gly Asp Lys Val Asn Gln Gly Asp Ile Ile Gly Leu Gln
   145                 150                 155                 160
   Gly Asn Ser Asn Tyr Tyr Asp Asn Pro Met Ser Val His Leu His Leu
                   165                 170                 175
   Gln Leu Arg Pro Lys Asp Ala Lys Lys Asp Glu Lys Ser Gln Val Cys
               180                 185                 190
   Ser Gly Leu Ala Met Glu Lys Tyr Asp Ile Thr Asn Leu Asn Ala Lys
           195                 200                 205
   Gln Asp Lys Ser Lys Asn Gly Ser Val Lys Glu Leu Lys His Ile Tyr
       210                 215                 220
   Ser Asn His Ile Lys Gly Asn Lys Ile Thr Ala Pro Lys Pro Ser Ile
   225                 230                 235                 240
   Gln Gly Val Val Ile His Asn Asp Tyr Gly Ser Met Thr Pro Ser Gln
                   245                 250                 255
   Tyr Leu Pro Trp Leu Tyr Ala Arg Glu Asn Asn Gly Thr His Val Asn
               260                 265                 270
   Gly Trp Ala Ser Val Tyr Ala Asn Arg Asn Glu Val Leu Trp Tyr His
           275                 280                 285
   Pro Thr Asp Tyr Val Glu Trp His Cys Gly Asn Gln Trp Ala Asn Ala
       290                 295                 300
   Asn Leu Ile Gly Phe Glu Val Cys Glu Ser Tyr Pro Gly Arg Ile Ser
   305 310 315 320
   Asp Lys Leu Phe Leu Glu Asn Glu Glu Ala Thr Leu Lys Val Ala Ala
   325 330 335
   Asp Val Met Lys Ser Tyr Gly Leu Pro Val Asn Arg Asn Thr Val Arg
   340 345 350
   Leu His Asn Glu Phe Phe Gly Thr Ser Cys Pro His Arg Ser Trp Asp
   355 360 365
   Leu His Val Gly Lys Gly Glu Pro Tyr Thr Thr Thr Asn Ile Asn Lys
   370 375 380
   Met Lys Asp Tyr Phe Ile Lys Arg Ile Lys His Tyr Tyr Asp Gly Gly
   385 390 395 400
   Lys Leu Glu Val Ser Lys Ala Ala Thr Ile Lys Gln Ser Asp Val Lys
   405 410 415
   Gln Glu Val Lys Lys Gln Glu Ala Lys Gln Ile Val Lys Ala Thr Asp
   420 425 430
   Trp Lys Gln Asn Lys Asp Gly Ile Trp Tyr Lys Ala Glu His Ala Ser
   435 440 445
   Phe Thr Val Thr Ala Pro Glu Gly Ile Ile Thr Arg Tyr Lys Gly Pro
   450 455 460
   Trp Thr Gly His Pro Gln Ala Gly Val Leu Gln Lys Gly Gln Thr Ile
   465 470 475 480
   Lys Tyr Asp Glu Val Gln Lys Phe Asp Gly His Val Trp Val Ser Trp
   485 490 495
   Glu Thr Phe Glu Gly Glu Thr Val Tyr Met Pro Val Arg Thr Trp Asp
   500 505 510
   Ala Lys Thr Gly Lys Val Gly Lys Leu Trp Gly Glu Ile Lys
   515 520 525
<210> 67
   <211> 1581
   <212> DNA
   <213> artificial
<220>
   <223> LL-37-Ply2638A
<400> 67
   atgctcctgg gtgacttctt tcgcaaatcc aaagagaaaa tcggcaaaga gttcaaacgt      60
   atcgtgcagc gcattaaaga ctttctgcgt aacctggttc cgcgtaccga atctggatcc     120
   atgctaactg ctattgacta tcttacgaaa aaaggttgga aaatatcatc tgaccctcgc     180
   acttacgatg gttaccctaa aaactacggc tacagaaatt accatgaaaa cggcattaat     240
   tatgatgagt tttgtggtgg ttatcataga gcttttgatg tttacagtaa cgaaactaac     300
   gacgtgcctg ctgttactag cggaacagtt attgaagcaa acgattacgg taattttggt     360
   ggtacattcg ttattagaga cgctaacgat aacgattgga tatatgggca tctacaacgt     420
   ggctcaatgc gatttgttgt aggcgacaaa gtcaatcaag gtgacattat tggtttacaa     480
   ggtaatagca actattacga caatcctatg agtgtacatt tacatttaca attacgccct     540
   aaagacgcaa agaaagatga aaaatcacaa gtatgtagtg gtttggctat ggaaaaatat     600
   gacattacaa atttaaatgc taaacaagat aaatcaaaga atgggagcgt gaaagagttg     660
   aaacatatct attcaaacca tattaaaggt aacaagatta cagcaccaaa acctagtatt     720
   caaggtgtgg tcatccacaa tgattatggt agtatgacac ctagtcaata cttaccatgg     780
   ttatatgcac gtgagaataa cggtacacac gttaacggtt gggctagtgt ttatgcaaat     840
   agaaacgaag tgctttggta tcatccgaca gactacgtag agtggcattg tggtaatcaa     900
   tgggcaaatg ctaacttaat cggatttgaa gtgtgtgagt cgtatcctgg tagaatctcg     960
   gacaaattat tcttagaaaa tgaagaagcg acattgaaag tagctgcgga tgtgatgaag    1020
   tcgtacggat taccagttaa tcgcaacact gtacgtctgc ataacgaatt cttcggaact    1080
   tcttgtccac atcgttcgtg ggacttgcat gttggcaaag gtgagcctta cacaactact    1140
   aatattaata aaatgaaaga ctacttcatc aaacgcatca aacattatta tgacggtgga    1200
   aagctagaag taagcaaagc agcaactatc aaacaatctg acgttaagca agaagttaaa    1260
   aagcaagaag caaaacaaat tgtgaaagca acagattgga aacagaataa agatggcatt    1320
   tggtataaag ctgaacatgc ttcgttcaca gtgacagcac cagagggaat tatcacaaga    1380
   tacaaaggtc cttggactgg tcacccacaa gctggtgtat tacaaaaagg tcaaacgatt    1440
   aaatatgatg aggttcaaaa atttgacggt catgtttggg tatcgtggga aacgtttgag    1500
   ggcgaaactg tatacatgcc ggtacgcaca tgggacgcta aaactggtaa agttggtaag    1560
   ttgtggggcg aaattaaatg a                                              1581
<210> 68
   <211> 518
   <212> PRT
   <213> artificial
<220>
   <223> SMAP-29-Ply2638A
<400> 68
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Gly Ser
               20                  25                  30
   Met Leu Thr Ala Ile Asp Tyr Leu Thr Lys Lys Gly Trp Lys Ile Ser
           35                  40                  45
   Ser Asp Pro Arg Thr Tyr Asp Gly Tyr Pro Lys Asn Tyr Gly Tyr Arg
       50                  55                  60
   Asn Tyr His Glu Asn Gly Ile Asn Tyr Asp Glu Phe Cys Gly Gly Tyr
   65                  70                  75                  80
   His Arg Ala Phe Asp Val Tyr Ser Asn Glu Thr Asn Asp Val Pro Ala
                   85                  90                  95
   Val Thr Ser Gly Thr Val Ile Glu Ala Asn Asp Tyr Gly Asn Phe Gly
               100                 105                 110
   Gly Thr Phe Val Ile Arg Asp Ala Asn Asp Asn Asp Trp Ile Tyr Gly
           115                 120                 125
   His Leu Gln Arg Gly Ser Met Arg Phe Val Val Gly Asp Lys Val Asn
       130                 135                 140
   Gln Gly Asp Ile Ile Gly Leu Gln Gly Asn Ser Asn Tyr Tyr Asp Asn
   145                 150                 155                 160
   Pro Met Ser Val His Leu His Leu Gln Leu Arg Pro Lys Asp Ala Lys
                   165                 170                 175
   Lys Asp Glu Lys Ser Gln Val Cys Ser Gly Leu Ala Met Glu Lys Tyr
               180                 185                 190
   Asp Ile Thr Asn Leu Asn Ala Lys Gln Asp Lys Ser Lys Asn Gly Ser
           195                 200                 205
   Val Lys Glu Leu Lys His Ile Tyr Ser Asn His Ile Lys Gly Asn Lys
       210                 215                 220
   Ile Thr Ala Pro Lys Pro Ser Ile Gln Gly Val Val Ile His Asn Asp
   225                 230                 235                 240
   Tyr Gly Ser Met Thr Pro Ser Gln Tyr Leu Pro Trp Leu Tyr Ala Arg
   245 250 255
   Glu Asn Asn Gly Thr His Val Asn Gly Trp Ala Ser Val Tyr Ala Asn
   260 265 270
   Arg Asn Glu Val Leu Trp Tyr His Pro Thr Asp Tyr Val Glu Trp His
   275 280 285
   Cys Gly Asn Gln Trp Ala Asn Ala Asn Leu Ile Gly Phe Glu Val Cys
   290 295 300
   Glu Ser Tyr Pro Gly Arg Ile Ser Asp Lys Leu Phe Leu Glu Asn Glu
   305 310 315 320
   Glu Ala Thr Leu Lys Val Ala Ala Asp Val Met Lys Ser Tyr Gly Leu
   325 330 335
   Pro Val Asn Arg Asn Thr Val Arg Leu His Asn Glu Phe Phe Gly Thr
   340 345 350
   Ser Cys Pro His Arg Ser Trp Asp Leu His Val Gly Lys Gly Glu Pro
   355 360 365
   Tyr Thr Thr Thr Asn Ile Asn Lys Met Lys Asp Tyr Phe Ile Lys Arg
   370 375 380
   Ile Lys His Tyr Tyr Asp Gly Gly Lys Leu Glu Val Ser Lys Ala Ala
   385 390 395 400
   Thr Ile Lys Gln Ser Asp Val Lys Gln Glu Val Lys Lys Gln Glu Ala
   405 410 415
   Lys Gln Ile Val Lys Ala Thr Asp Trp Lys Gln Asn Lys Asp Gly Ile
   420 425 430
   Trp Tyr Lys Ala Glu His Ala Ser Phe Thr Val Thr Ala Pro Glu Gly
   435 440 445
   Ile Ile Thr Arg Tyr Lys Gly Pro Trp Thr Gly His Pro Gln Ala Gly
   450 455 460
   Val Leu Gln Lys Gly Gln Thr Ile Lys Tyr Asp Glu Val Gln Lys Phe
   465 470 475 480
   Asp Gly His Val Trp Val Ser Trp Glu Thr Phe Glu Gly Glu Thr Val
   485 490 495
   Tyr Met Pro Val Arg Thr Trp Asp Ala Lys Thr Gly Lys Val Gly Lys
   500 505 510
   Leu Trp Gly Glu Ile Lys
   515
<210> 69
   <211> 1557
   <212> DNA
   <213> artificial
<220>
   <223> SMAP-29-Ply2638A
<400> 69
   atgcgtggtc tgcgtcgcct gggtcgcaaa attgcgcacg gcgtcaaaaa atacggcccg 60
   accgtgctgc gcattatccg catcgctggt ggatccatgc taactgctat tgactatctt 120
   acgaaaaaag gttggaaaat atcatctgac cctcgcactt acgatggtta ccctaaaaac 180
   tacggctaca gaaattacca tgaaaacggc attaattatg atgagttttg tggtggttat 240
   catagagctt ttgatgttta cagtaacgaa actaacgacg tgcctgctgt tactagcgga 300
   acagttattg aagcaaacga ttacggtaat tttggtggta cattcgttat tagagacgct 360
   aacgataacg attggatata tgggcatcta caacgtggct caatgcgatt tgttgtaggc 420
   gacaaagtca atcaaggtga cattattggt ttacaaggta atagcaacta ttacgacaat 480
   cctatgagtg tacatttaca tttacaatta cgccctaaag acgcaaagaa agatgaaaaa 540
   tcacaagtat gtagtggttt ggctatggaa aaatatgaca ttacaaattt aaatgctaaa 600
   caagataaat caaagaatgg gagcgtgaaa gagttgaaac atatctattc aaaccatatt 660
   aaaggtaaca agattacagc accaaaacct agtattcaag gtgtggtcat ccacaatgat 720
   tatggtagta tgacacctag tcaatactta ccatggttat atgcacgtga gaataacggt 780
   acacacgtta acggttgggc tagtgtttat gcaaatagaa acgaagtgct ttggtatcat 840
   ccgacagact acgtagagtg gcattgtggt aatcaatggg caaatgctaa cttaatcgga 900
   tttgaagtgt gtgagtcgta tcctggtaga atctcggaca aattattctt agaaaatgaa 960
   gaagcgacat tgaaagtagc tgcggatgtg atgaagtcgt acggattacc agttaatcgc 1020
   aacactgtac gtctgcataa cgaattcttc ggaacttctt gtccacatcg ttcgtgggac 1080
   ttgcatgttg gcaaaggtga gccttacaca actactaata ttaataaaat gaaagactac 1140
   ttcatcaaac gcatcaaaca ttattatgac ggtggaaagc tagaagtaag caaagcagca 1200
   actatcaaac aatctgacgt taagcaagaa gttaaaaagc aagaagcaaa acaaattgtg 1260
   aaagcaacag attggaaaca gaataaagat ggcatttggt ataaagctga acatgcttcg 1320
   ttcacagtga cagcaccaga gggaattatc acaagataca aaggtccttg gactggtcac 1380
   ccacaagctg gtgtattaca aaaaggtcaa acgattaaat atgatgaggt tcaaaaattt 1440
   gacggtcatg tttgggtatc gtgggaaacg tttgagggcg aaactgtata catgccggta 1500
   cgcacatggg acgctaaaac tggtaaagtt ggtaagttgt ggggcgaaat taaatga 1557
<210> 70
   <211> 525
   <212> PRT
   <213> artificial
<220>
   <223> Cecropin A (A.aegypti)-Ply2638A
<400> 70
   Met Gly Gly Leu Lys Lys Leu Gly Lys Lys Leu Glu Gly Ala Gly Lys
   1 5 10 15
   Arg Val Phe Asn Ala Ala Glu Lys Ala Leu Pro Val Val Ala Gly Ala
   20 25 30
   Lys Ala Leu Arg Lys Gly Ser Met Leu Thr Ala Ile Asp Tyr Leu Thr
   35 40 45
   Lys Lys Gly Trp Lys Ile Ser Ser Asp Pro Arg Thr Tyr Asp Gly Tyr
   50 55 60
   Pro Lys Asn Tyr Gly Tyr Arg Asn Tyr His Glu Asn Gly Ile Asn Tyr
   65 70 75 80
   Asp Glu Phe Cys Gly Gly Tyr His Arg Ala Phe Asp Val Tyr Ser Asn
   85 90 95
   Glu Thr Asn Asp Val Pro Ala Val Thr Ser Gly Thr Val Ile Glu Ala
   100 105 110
   Asn Asp Tyr Gly Asn Phe Gly Gly Thr Phe Val Ile Arg Asp Ala Asn
   115 120 125
   Asp Asn Asp Trp Ile Tyr Gly His Leu Gln Arg Gly Ser Met Arg Phe
   130 135 140
   Val Val Gly Asp Lys Val Asn Gln Gly Asp Ile Ile Gly Leu Gln Gly
   145 150 155 160
   Asn Ser Asn Tyr Tyr Asp Asn Pro Met Ser Val His Leu His Leu Gln
   165 170 175
   Leu Arg Pro Lys Asp Ala Lys Lys Asp Glu Lys Ser Gln Val Cys Ser
               180                 185                 190
   Gly Leu Ala Met Glu Lys Tyr Asp Ile Thr Asn Leu Asn Ala Lys Gln
           195                 200                 205
   Asp Lys Ser Lys Asn Gly Ser Val Lys Glu Leu Lys His Ile Tyr Ser
       210                 215                 220
   Asn His Ile Lys Gly Asn Lys Ile Thr Ala Pro Lys Pro Ser Ile Gln
   225                 230                 235                 240
   Gly Val Val Ile His Asn Asp Tyr Gly Ser Met Thr Pro Ser Gln Tyr
                   245                 250                 255
   Leu Pro Trp Leu Tyr Ala Arg Glu Asn Asn Gly Thr His Val Asn Gly
               260                 265                 270
   Trp Ala Ser Val Tyr Ala Asn Arg Asn Glu Val Leu Trp Tyr His Pro
           275                 280                 285
   Thr Asp Tyr Val Glu Trp His Cys Gly Asn Gln Trp Ala Asn Ala Asn
       290                 295                 300
   Leu Ile Gly Phe Glu Val Cys Glu Ser Tyr Pro Gly Arg Ile Ser Asp
   305                 310                 315                 320
   Lys Leu Phe Leu Glu Asn Glu Glu Ala Thr Leu Lys Val Ala Ala Asp
                   325                 330                 335
   Val Met Lys Ser Tyr Gly Leu Pro Val Asn Arg Asn Thr Val Arg Leu
               340                 345                 350
   His Asn Glu Phe Phe Gly Thr Ser Cys Pro His Arg Ser Trp Asp Leu
           355                 360                 365
   His Val Gly Lys Gly Glu Pro Tyr Thr Thr Thr Asn Ile Asn Lys Met
       370                 375                 380
   Lys Asp Tyr Phe Ile Lys Arg Ile Lys His Tyr Tyr Asp Gly Gly Lys
   385                 390                 395                 400
   Leu Glu Val Ser Lys Ala Ala Thr Ile Lys Gln Ser Asp Val Lys Gln
                   405                 410                 415
   Glu Val Lys Lys Gln Glu Ala Lys Gln Ile Val Lys Ala Thr Asp Trp
               420                 425                 430
   Lys Gln Asn Lys Asp Gly Ile Trp Tyr Lys Ala Glu His Ala Ser Phe
   435 440 445
   Thr Val Thr Ala Pro Glu Gly Ile Ile Thr Arg Tyr Lys Gly Pro Trp
   450 455 460
   Thr Gly His Pro Gln Ala Gly Val Leu Gln Lys Gly Gln Thr Ile Lys
   465 470 475 480
   Tyr Asp Glu Val Gln Lys Phe Asp Gly His Val Trp Val Ser Trp Glu
   485 490 495
   Thr Phe Glu Gly Glu Thr Val Tyr Met Pro Val Arg Thr Trp Asp Ala
   500 505 510
   Lys Thr Gly Lys Val Gly Lys Leu Trp Gly Glu Ile Lys
   515 520 525
<210> 71
   <211> 1578
   <212> DNA
   <213> artificial
<220>
   <223> Cecropin A (A.aegypti)-Ply2638A
<400> 71
   atgggcggcc tgaaaaaact gggcaaaaaa ctggaaggtg ccggcaaacg tgtgttcaac 60
   gctgcagaaa aagcactgcc ggttgtagct ggtgctaaag ctctccgtaa aggatccatg 120
   ctaactgcta ttgactatct tacgaaaaaa ggttggaaaa tatcatctga ccctcgcact 180
   tacgatggtt accctaaaaa ctacggctac agaaattacc atgaaaacgg cattaattat 240
   gatgagtttt gtggtggtta tcatagagct tttgatgttt acagtaacga aactaacgac 300
   gtgcctgctg ttactagcgg aacagttatt gaagcaaacg attacggtaa ttttggtggt 360
   acattcgtta ttagagacgc taacgataac gattggatat atgggcatct acaacgtggc 420
   tcaatgcgat ttgttgtagg cgacaaagtc aatcaaggtg acattattgg tttacaaggt 480
   aatagcaact attacgacaa tcctatgagt gtacatttac atttacaatt acgccctaaa 540
   gacgcaaaga aagatgaaaa atcacaagta tgtagtggtt tggctatgga aaaatatgac 600
   attacaaatt taaatgctaa acaagataaa tcaaagaatg ggagcgtgaa agagttgaaa 660
   catatctatt caaaccatat taaaggtaac aagattacag caccaaaacc tagtattcaa 720
   ggtgtggtca tccacaatga ttatggtagt atgacaccta gtcaatactt accatggtta 780
   tatgcacgtg agaataacgg tacacacgtt aacggttggg ctagtgttta tgcaaataga 840
   aacgaagtgc tttggtatca tccgacagac tacgtagagt ggcattgtgg taatcaatgg 900
   gcaaatgcta acttaatcgg atttgaagtg tgtgagtcgt atcctggtag aatctcggac 960
   aaattattct tagaaaatga agaagcgaca ttgaaagtag ctgcggatgt gatgaagtcg 1020
   tacggattac cagttaatcg caacactgta cgtctgcata acgaattctt cggaacttct 1080
   tgtccacatc gttcgtggga cttgcatgtt ggcaaaggtg agccttacac aactactaat 1140
   attaataaaa tgaaagacta cttcatcaaa cgcatcaaac attattatga cggtggaaag 1200
   ctagaagtaa gcaaagcagc aactatcaaa caatctgacg ttaagcaaga agttaaaaag 1260
   caagaagcaa aacaaattgt gaaagcaaca gattggaaac agaataaaga tggcatttgg 1320
   tataaagctg aacatgcttc gttcacagtg acagcaccag agggaattat cacaagatac 1380
   aaaggtcctt ggactggtca cccacaagct ggtgtattac aaaaaggtca aacgattaaa 1440
   tatgatgagg ttcaaaaatt tgacggtcat gtttgggtat cgtgggaaac gtttgagggc 1500
   gaaactgtat acatgccggt acgcacatgg gacgctaaaa ctggtaaagt tggtaagttg 1560
   tggggcgaaa ttaaatga 1578
<210> 72
   <211> 528
   <212> PRT
   <213> artificial
<220>
   <223> Sarcotoxin IA-Ply2638A
<400> 72
   Met Gly Trp Leu Lys Lys Ile Gly Lys Lys Ile Glu Arg Val Gly Gln
   1               5                   10                  15
   His Thr Arg Asp Ala Thr Ile Gln Gly Leu Gly Ile Ala Gln Gln Ala
               20                  25                  30
   Ala Asn Val Ala Ala Thr Ala Arg Gly Ser Met Leu Thr Ala Ile Asp
           35                  40                  45
   Tyr Leu Thr Lys Lys Gly Trp Lys Ile Ser Ser Asp Pro Arg Thr Tyr
       50                  55                  60
   Asp Gly Tyr Pro Lys Asn Tyr Gly Tyr Arg Asn Tyr His Glu Asn Gly
   65                  70                  75                  80
   Ile Asn Tyr Asp Glu Phe Cys Gly Gly Tyr His Arg Ala Phe Asp Val
                   85                  90                  95
   Tyr Ser Asn Glu Thr Asn Asp Val Pro Ala Val Thr Ser Gly Thr Val
               100                 105                 110
   Ile Glu Ala Asn Asp Tyr Gly Asn Phe Gly Gly Thr Phe Val Ile Arg
   115 120 125
   Asp Ala Asn Asp Asn Asp Trp Ile Tyr Gly His Leu Gln Arg Gly Ser
   130 135 140
   Met Arg Phe Val Val Gly Asp Lys Val Asn Gln Gly Asp Ile Ile Gly
   145 150 155 160
   Leu Gln Gly Asn Ser Asn Tyr Tyr Asp Asn Pro Met Ser Val His Leu
   165 170 175
   His Leu Gln Leu Arg Pro Lys Asp Ala Lys Lys Asp Glu Lys Ser Gln
   180 185 190
   Val Cys Ser Gly Leu Ala Met Glu Lys Tyr Asp Ile Thr Asn Leu Asn
   195 200 205
   Ala Lys Gln Asp Lys Ser Lys Asn Gly Ser Val Lys Glu Leu Lys His
   210 215 220
   Ile Tyr Ser Asn His Ile Lys Gly Asn Lys Ile Thr Ala Pro Lys Pro
   225 230 235 240
   Ser Ile Gln Gly Val Val Ile His Asn Asp Tyr Gly Ser Met Thr Pro
   245 250 255
   Ser Gln Tyr Leu Pro Trp Leu Tyr Ala Arg Glu Asn Asn Gly Thr His
   260 265 270
   Val Asn Gly Trp Ala Ser Val Tyr Ala Asn Arg Asn Glu Val Leu Trp
   275 280 285
   Tyr His Pro Thr Asp Tyr Val Glu Trp His Cys Gly Asn Gln Trp Ala
   290 295 300
   Asn Ala Asn Leu Ile Gly Phe Glu Val Cys Glu Ser Tyr Pro Gly Arg
   305 310 315 320
   Ile Ser Asp Lys Leu Phe Leu Glu Asn Glu Glu Ala Thr Leu Lys Val
   325 330 335
   Ala Ala Asp Val Met Lys Ser Tyr Gly Leu Pro Val Asn Arg Asn Thr
   340 345 350
   Val Arg Leu His Asn Glu Phe Phe Gly Thr Ser Cys Pro His Arg Ser
   355 360 365
   Trp Asp Leu His Val Gly Lys Gly Glu Pro Tyr Thr Thr Thr Asn Ile
   370 375 380
   Asn Lys Met Lys Asp Tyr Phe Ile Lys Arg Ile Lys His Tyr Tyr Asp
   385 390 395 400
   Gly Gly Lys Leu Glu Val Ser Lys Ala Ala Thr Ile Lys Gln Ser Asp
   405 410 415
   Val Lys Gln Glu Val Lys Lys Gln Glu Ala Lys Gln Ile Val Lys Ala
   420 425 430
   Thr Asp Trp Lys Gln Asn Lys Asp Gly Ile Trp Tyr Lys Ala Glu His
   435 440 445
   Ala Ser Phe Thr Val Thr Ala Pro Glu Gly Ile Ile Thr Arg Tyr Lys
   450 455 460
   Gly Pro Trp Thr Gly His Pro Gln Ala Gly Val Leu Gln Lys Gly Gln
   465 470 475 480
   Thr Ile Lys Tyr Asp Glu Val Gln Lys Phe Asp Gly His Val Trp Val
   485 490 495
   Ser Trp Glu Thr Phe Glu Gly Glu Thr Val Tyr Met Pro Val Arg Thr
   500 505 510
   Trp Asp Ala Lys Thr Gly Lys Val Gly Lys Leu Trp Gly Glu Ile Lys
   515 520 525
<210> 73
   <211> 1587
   <212> DNA
   <213> artificial
<220>
   <223> Sarcotoxin IA-Ply2638A
<400> 73
   atgggatggc tcaaaaagat tggcaagaaa atcgagcgag tcggtcagca tacgcgtgat 60
   gcaactatcc agggtttagg tatcgcacag caagcagcta atgtagcagc tactgctcgg 120
   ggatccatgc taactgctat tgactatctt acgaaaaaag gttggaaaat atcatctgac 180
   cctcgcactt acgatggtta ccctaaaaac tacggctaca gaaattacca tgaaaacggc 240
   attaattatg atgagttttg tggtggttat catagagctt ttgatgttta cagtaacgaa 300
   actaacgacg tgcctgctgt tactagcgga acagttattg aagcaaacga ttacggtaat 360
   tttggtggta cattcgttat tagagacgct aacgataacg attggatata tgggcatcta 420
   caacgtggct caatgcgatt tgttgtaggc gacaaagtca atcaaggtga cattattggt 480
   ttacaaggta atagcaacta ttacgacaat cctatgagtg tacatttaca tttacaatta 540
   cgccctaaag acgcaaagaa agatgaaaaa tcacaagtat gtagtggttt ggctatggaa 600
   aaatatgaca ttacaaattt aaatgctaaa caagataaat caaagaatgg gagcgtgaaa 660
   gagttgaaac atatctattc aaaccatatt aaaggtaaca agattacagc accaaaacct 720
   agtattcaag gtgtggtcat ccacaatgat tatggtagta tgacacctag tcaatactta 780
   ccatggttat atgcacgtga gaataacggt acacacgtta acggttgggc tagtgtttat 840
   gcaaatagaa acgaagtgct ttggtatcat ccgacagact acgtagagtg gcattgtggt 900
   aatcaatggg caaatgctaa cttaatcgga tttgaagtgt gtgagtcgta tcctggtaga 960
   atctcggaca aattattctt agaaaatgaa gaagcgacat tgaaagtagc tgcggatgtg 1020
   atgaagtcgt acggattacc agttaatcgc aacactgtac gtctgcataa cgaattcttc 1080
   ggaacttctt gtccacatcg ttcgtgggac ttgcatgttg gcaaaggtga gccttacaca 1140
   actactaata ttaataaaat gaaagactac ttcatcaaac gcatcaaaca ttattatgac 1200
   ggtggaaagc tagaagtaag caaagcagca actatcaaac aatctgacgt taagcaagaa 1260
   gttaaaaagc aagaagcaaa acaaattgtg aaagcaacag attggaaaca gaataaagat 1320
   ggcatttggt ataaagctga acatgcttcg ttcacagtga cagcaccaga gggaattatc 1380
   acaagataca aaggtccttg gactggtcac ccacaagctg gtgtattaca aaaaggtcaa 1440
   acgattaaat atgatgaggt tcaaaaattt gacggtcatg tttgggtatc gtgggaaacg 1500
   tttgagggcg aaactgtata catgccggta cgcacatggg acgctaaaac tggtaaagtt 1560
   ggtaagttgt ggggcgaaat taaatga 1587
<210> 74
   <211> 543
   <212> PRT
   <213> artificial
<220>
   <223> OR-7-Ply2638A
<400> 74
   Met Lys Thr Tyr Tyr Gly Thr Asn Gly Val His Cys Thr Lys Asn Ser
   1               5                   10                  15
   Leu Trp Gly Lys Val Arg Leu Lys Asn Met Lys Tyr Asp Gln Asn Thr
               20                  25                  30
   Thr Tyr Met Gly Arg Leu Gln Asp Ile Leu Leu Gly Trp Ala Thr Gly
   35 40 45
   Ala Phe Gly Lys Thr Phe His Gly Ser Met Leu Thr Ala Ile Asp Tyr
   50 55 60
   Leu Thr Lys Lys Gly Trp Lys Ile Ser Ser Asp Pro Arg Thr Tyr Asp
   65 70 75 80
   Gly Tyr Pro Lys Asn Tyr Gly Tyr Arg Asn Tyr His Glu Asn Gly Ile
   85 90 95
   Asn Tyr Asp Glu Phe Cys Gly Gly Tyr His Arg Ala Phe Asp Val Tyr
   100 105 110
   Ser Asn Glu Thr Asn Asp Val Pro Ala Val Thr Ser Gly Thr Val Ile
   115 120 125
   Glu Ala Asn Asp Tyr Gly Asn Phe Gly Gly Thr Phe Val Ile Arg Asp
   130 135 140
   Ala Asn Asp Asn Asp Trp Ile Tyr Gly His Leu Gln Arg Gly Ser Met
   145 150 155 160
   Arg Phe Val Val Gly Asp Lys Val Asn Gln Gly Asp Ile Ile Gly Leu
   165 170 175
   Gln Gly Asn Ser Asn Tyr Tyr Asp Asn Pro Met Ser Val His Leu His
   180 185 190
   Leu Gln Leu Arg Pro Lys Asp Ala Lys Lys Asp Glu Lys Ser Gln Val
   195 200 205
   Cys Ser Gly Leu Ala Met Glu Lys Tyr Asp Ile Thr Asn Leu Asn Ala
   210 215 220
   Lys Gln Asp Lys Ser Lys Asn Gly Ser Val Lys Glu Leu Lys His Ile
   225 230 235 240
   Tyr Ser Asn His Ile Lys Gly Asn Lys Ile Thr Ala Pro Lys Pro Ser
   245 250 255
   Ile Gln Gly Val Val Ile His Asn Asp Tyr Gly Ser Met Thr Pro Ser
   260 265 270
   Gln Tyr Leu Pro Trp Leu Tyr Ala Arg Glu Asn Asn Gly Thr His Val
   275 280 285
   Asn Gly Trp Ala Ser Val Tyr Ala Asn Arg Asn Glu Val Leu Trp Tyr
   290 295 300
   His Pro Thr Asp Tyr Val Glu Trp His Cys Gly Asn Gln Trp Ala Asn
   305 310 315 320
   Ala Asn Leu Ile Gly Phe Glu Val Cys Glu Ser Tyr Pro Gly Arg Ile
   325 330 335
   Ser Asp Lys Leu Phe Leu Glu Asn Glu Glu Ala Thr Leu Lys Val Ala
   340 345 350
   Ala Asp Val Met Lys Ser Tyr Gly Leu Pro Val Asn Arg Asn Thr Val
   355 360 365
   Arg Leu His Asn Glu Phe Phe Gly Thr Ser Cys Pro His Arg Ser Trp
   370 375 380
   Asp Leu His Val Gly Lys Gly Glu Pro Tyr Thr Thr Thr Asn Ile Asn
   385 390 395 400
   Lys Met Lys Asp Tyr Phe Ile Lys Arg Ile Lys His Tyr Tyr Asp Gly
   405 410 415
   Gly Lys Leu Glu Val Ser Lys Ala Ala Thr Ile Lys Gln Ser Asp Val
   420 425 430
   Lys Gln Glu Val Lys Lys Gln Glu Ala Lys Gln Ile Val Lys Ala Thr
   435 440 445
   Asp Trp Lys Gln Asn Lys Asp Gly Ile Trp Tyr Lys Ala Glu His Ala
   450 455 460
   Ser Phe Thr Val Thr Ala Pro Glu Gly Ile Ile Thr Arg Tyr Lys Gly
   465 470 475 480
   Pro Trp Thr Gly His Pro Gln Ala Gly Val Leu Gln Lys Gly Gln Thr
   485 490 495
   Ile Lys Tyr Asp Glu Val Gln Lys Phe Asp Gly His Val Trp Val Ser
   500 505 510
   Trp Glu Thr Phe Glu Gly Glu Thr Val Tyr Met Pro Val Arg Thr Trp
   515 520 525
   Asp Ala Lys Thr Gly Lys Val Gly Lys Leu Trp Gly Glu Ile Lys
   530 535 540
<210> 75
   <211> 1632
   <212> DNA
   <213> artificial
<220>
   <223> OR-7-Ply2638A
<400> 75
   atgaaaacct actatggcac caacggcgta cactgcacca aaaacagcct gtggggtaaa 60
   gtgcgtctga aaaacatgaa atacgaccag aacaccacgt atatgggccg tctccaggat 120
   atcctgctgg gttgggcaac tggtgccttc ggcaaaacct ttcacggatc catgctaact 180
   gctattgact atcttacgaa aaaaggttgg aaaatatcat ctgaccctcg cacttacgat 240
   ggttacccta aaaactacgg ctacagaaat taccatgaaa acggcattaa ttatgatgag 300
   ttttgtggtg gttatcatag agcttttgat gtttacagta acgaaactaa cgacgtgcct 360
   gctgttacta gcggaacagt tattgaagca aacgattacg gtaattttgg tggtacattc 420
   gttattagag acgctaacga taacgattgg atatatgggc atctacaacg tggctcaatg 480
   cgatttgttg taggcgacaa agtcaatcaa ggtgacatta ttggtttaca aggtaatagc 540
   aactattacg acaatcctat gagtgtacat ttacatttac aattacgccc taaagacgca 600
   aagaaagatg aaaaatcaca agtatgtagt ggtttggcta tggaaaaata tgacattaca 660
   aatttaaatg ctaaacaaga taaatcaaag aatgggagcg tgaaagagtt gaaacatatc 720
   tattcaaacc atattaaagg taacaagatt acagcaccaa aacctagtat tcaaggtgtg 780
   gtcatccaca atgattatgg tagtatgaca cctagtcaat acttaccatg gttatatgca 840
   cgtgagaata acggtacaca cgttaacggt tgggctagtg tttatgcaaa tagaaacgaa 900
   gtgctttggt atcatccgac agactacgta gagtggcatt gtggtaatca atgggcaaat 960
   gctaacttaa tcggatttga agtgtgtgag tcgtatcctg gtagaatctc ggacaaatta 1020
   ttcttagaaa atgaagaagc gacattgaaa gtagctgcgg atgtgatgaa gtcgtacgga 1080
   ttaccagtta atcgcaacac tgtacgtctg cataacgaat tcttcggaac ttcttgtcca 1140
   catcgttcgt gggacttgca tgttggcaaa ggtgagcctt acacaactac taatattaat 1200
   aaaatgaaag actacttcat caaacgcatc aaacattatt atgacggtgg aaagctagaa 1260
   gtaagcaaag cagcaactat caaacaatct gacgttaagc aagaagttaa aaagcaagaa 1320
   gcaaaacaaa ttgtgaaagc aacagattgg aaacagaata aagatggcat ttggtataaa 1380
   gctgaacatg cttcgttcac agtgacagca ccagagggaa ttatcacaag atacaaaggt 1440
   ccttggactg gtcacccaca agctggtgta ttacaaaaag gtcaaacgat taaatatgat 1500
   gaggttcaaa aatttgacgg tcatgtttgg gtatcgtggg aaacgtttga gggcgaaact 1560
   gtatacatgc cggtacgcac atgggacgct aaaactggta aagttggtaa gttgtggggc 1620
   gaaattaaat ga 1632
<210> 76
   <211> 502
   <212> PRT
   <213> artificial
<220>
   <223> Indolicidin-Ply2638A
<400> 76
   Met Ile Leu Pro Trp Lys Trp Pro Trp Trp Pro Trp Arg Arg Gly Ser
   1               5                   10                  15
   Met Leu Thr Ala Ile Asp Tyr Leu Thr Lys Lys Gly Trp Lys Ile Ser
               20                  25                  30
   Ser Asp Pro Arg Thr Tyr Asp Gly Tyr Pro Lys Asn Tyr Gly Tyr Arg
           35                  40                  45
   Asn Tyr His Glu Asn Gly Ile Asn Tyr Asp Glu Phe Cys Gly Gly Tyr
       50                  55                  60
   His Arg Ala Phe Asp Val Tyr Ser Asn Glu Thr Asn Asp Val Pro Ala
   65                  70                  75                  80
   Val Thr Ser Gly Thr Val Ile Glu Ala Asn Asp Tyr Gly Asn Phe Gly
                   85                  90                  95
   Gly Thr Phe Val Ile Arg Asp Ala Asn Asp Asn Asp Trp Ile Tyr Gly
               100                 105                 110
   His Leu Gln Arg Gly Ser Met Arg Phe Val Val Gly Asp Lys Val Asn
           115                 120                 125
   Gln Gly Asp Ile Ile Gly Leu Gln Gly Asn Ser Asn Tyr Tyr Asp Asn
       130                 135                 140
   Pro Met Ser Val His Leu His Leu Gln Leu Arg Pro Lys Asp Ala Lys
   145                 150                 155                 160
   Lys Asp Glu Lys Ser Gln Val Cys Ser Gly Leu Ala Met Glu Lys Tyr
                   165                 170                 175
   Asp Ile Thr Asn Leu Asn Ala Lys Gln Asp Lys Ser Lys Asn Gly Ser
               180                 185                 190
   Val Lys Glu Leu Lys His Ile Tyr Ser Asn His Ile Lys Gly Asn Lys
   195 200 205
   Ile Thr Ala Pro Lys Pro Ser Ile Gln Gly Val Val Ile His Asn Asp
   210 215 220
   Tyr Gly Ser Met Thr Pro Ser Gln Tyr Leu Pro Trp Leu Tyr Ala Arg
   225 230 235 240
   Glu Asn Asn Gly Thr His Val Asn Gly Trp Ala Ser Val Tyr Ala Asn
   245 250 255
   Arg Asn Glu Val Leu Trp Tyr His Pro Thr Asp Tyr Val Glu Trp His
   260 265 270
   Cys Gly Asn Gln Trp Ala Asn Ala Asn Leu Ile Gly Phe Glu Val Cys
   275 280 285
   Glu Ser Tyr Pro Gly Arg Ile Ser Asp Lys Leu Phe Leu Glu Asn Glu
   290 295 300
   Glu Ala Thr Leu Lys Val Ala Ala Asp Val Met Lys Ser Tyr Gly Leu
   305 310 315 320
   Pro Val Asn Arg Asn Thr Val Arg Leu His Asn Glu Phe Phe Gly Thr
   325 330 335
   Ser Cys Pro His Arg Ser Trp Asp Leu His Val Gly Lys Gly Glu Pro
   340 345 350
   Tyr Thr Thr Thr Asn Ile Asn Lys Met Lys Asp Tyr Phe Ile Lys Arg
   355 360 365
   Ile Lys His Tyr Tyr Asp Gly Gly Lys Leu Glu Val Ser Lys Ala Ala
   370 375 380
   Thr Ile Lys Gln Ser Asp Val Lys Gln Glu Val Lys Lys Gln Glu Ala
   385 390 395 400
   Lys Gln Ile Val Lys Ala Thr Asp Trp Lys Gln Asn Lys Asp Gly Ile
   405 410 415
   Trp Tyr Lys Ala Glu His Ala Ser Phe Thr Val Thr Ala Pro Glu Gly
   420 425 430
   Ile Ile Thr Arg Tyr Lys Gly Pro Trp Thr Gly His Pro Gln Ala Gly
   435 440 445
   Val Leu Gln Lys Gly Gln Thr Ile Lys Tyr Asp Glu Val Gln Lys Phe
   450 455 460
   Asp Gly His Val Trp Val Ser Trp Glu Thr Phe Glu Gly Glu Thr Val
   465 470 475 480
   Tyr Met Pro Val Arg Thr Trp Asp Ala Lys Thr Gly Lys Val Gly Lys
   485 490 495
   Leu Trp Gly Glu Ile Lys
   500
<210> 77
   <211> 1509
   <212> DNA
   <213> artificial
<220>
   <223> Indolicidin-Ply2638A
<400> 77
   atgatcctgc cgtggaaatg gccgtggtgg ccatggcgtc gcggatccat gctaactgct     60
   attgactatc ttacgaaaaa aggttggaaa atatcatctg accctcgcac ttacgatggt    120
   taccctaaaa actacggcta cagaaattac catgaaaacg gcattaatta tgatgagttt    180
   tgtggtggtt atcatagagc ttttgatgtt tacagtaacg aaactaacga cgtgcctgct    240
   gttactagcg gaacagttat tgaagcaaac gattacggta attttggtgg tacattcgtt    300
   attagagacg ctaacgataa cgattggata tatgggcatc tacaacgtgg ctcaatgcga    360
   tttgttgtag gcgacaaagt caatcaaggt gacattattg gtttacaagg taatagcaac    420
   tattacgaca atcctatgag tgtacattta catttacaat tacgccctaa agacgcaaag    480
   aaagatgaaa aatcacaagt atgtagtggt ttggctatgg aaaaatatga cattacaaat    540
   ttaaatgcta aacaagataa atcaaagaat gggagcgtga aagagttgaa acatatctat    600
   tcaaaccata ttaaaggtaa caagattaca gcaccaaaac ctagtattca aggtgtggtc    660
   atccacaatg attatggtag tatgacacct agtcaatact taccatggtt atatgcacgt    720
   gagaataacg gtacacacgt taacggttgg gctagtgttt atgcaaatag aaacgaagtg    780
   ctttggtatc atccgacaga ctacgtagag tggcattgtg gtaatcaatg ggcaaatgct    840
   aacttaatcg gatttgaagt gtgtgagtcg tatcctggta gaatctcgga caaattattc    900
   ttagaaaatg aagaagcgac attgaaagta gctgcggatg tgatgaagtc gtacggatta    960
   ccagttaatc gcaacactgt acgtctgcat aacgaattct tcggaacttc ttgtccacat   1020
   cgttcgtggg acttgcatgt tggcaaaggt gagccttaca caactactaa tattaataaa   1080
   atgaaagact acttcatcaa acgcatcaaa cattattatg acggtggaaa gctagaagta   1140
   agcaaagcag caactatcaa acaatctgac gttaagcaag aagttaaaaa gcaagaagca 1200
   aaacaaattg tgaaagcaac agattggaaa cagaataaag atggcatttg gtataaagct 1260
   gaacatgctt cgttcacagt gacagcacca gagggaatta tcacaagata caaaggtcct 1320
   tggactggtc acccacaagc tggtgtatta caaaaaggtc aaacgattaa atatgatgag 1380
   gttcaaaaat ttgacggtca tgtttgggta tcgtgggaaa cgtttgaggg cgaaactgta 1440
   tacatgccgg tacgcacatg ggacgctaaa actggtaaag ttggtaagtt gtggggcgaa 1500
   attaaatga 1509
<210> 78
   <211> 513
   <212> PRT
   <213> artificial
<220>
   <223> Pseudinl-Ply2638A
<400> 78
   Met Gly Leu Asn Thr Leu Lys Lys Val Phe Gln Gly Leu His Glu Ala
   1               5                   10                  15
   Ile Lys Leu Ile Asn Asn His Val Gln Gly Ser Met Leu Thr Ala Ile
               20                  25                  30
   Asp Tyr Leu Thr Lys Lys Gly Trp Lys Ile Ser Ser Asp Pro Arg Thr
           35                  40                  45
   Tyr Asp Gly Tyr Pro Lys Asn Tyr Gly Tyr Arg Asn Tyr His Glu Asn
       50                  55                  60
   Gly Ile Asn Tyr Asp Glu Phe Cys Gly Gly Tyr His Arg Ala Phe Asp
   65                  70                  75                  80
   Val Tyr Ser Asn Glu Thr Asn Asp Val Pro Ala Val Thr Ser Gly Thr
                   85                  90                  95
   Val Ile Glu Ala Asn Asp Tyr Gly Asn Phe Gly Gly Thr Phe Val Ile
               100                 105                 110
   Arg Asp Ala Asn Asp Asn Asp Trp Ile Tyr Gly His Leu Gln Arg Gly
           115                 120                 125
   Ser Met Arg Phe Val Val Gly Asp Lys Val Asn Gln Gly Asp Ile Ile
       130                 135                 140
   Gly Leu Gln Gly Asn Ser Asn Tyr Tyr Asp Asn Pro Met Ser Val His
   145                 150                 155                 160
   Leu His Leu Gln Leu Arg Pro Lys Asp Ala Lys Lys Asp Glu Lys Ser
   165 170 175
   Gln Val Cys Ser Gly Leu Ala Met Glu Lys Tyr Asp Ile Thr Asn Leu
   180 185 190
   Asn Ala Lys Gln Asp Lys Ser Lys Asn Gly Ser Val Lys Glu Leu Lys
   195 200 205
   His Ile Tyr Ser Asn His Ile Lys Gly Asn Lys Ile Thr Ala Pro Lys
   210 215 220
   Pro Ser Ile Gln Gly Val Val Ile His Asn Asp Tyr Gly Ser Met Thr
   225 230 235 240
   Pro Ser Gln Tyr Leu Pro Trp Leu Tyr Ala Arg Glu Asn Asn Gly Thr
   245 250 255
   His Val Asn Gly Trp Ala Ser Val Tyr Ala Asn Arg Asn Glu Val Leu
   260 265 270
   Trp Tyr His Pro Thr Asp Tyr Val Glu Trp His Cys Gly Asn Gln Trp
   275 280 285
   Ala Asn Ala Asn Leu Ile Gly Phe Glu Val Cys Glu Ser Tyr Pro Gly
   290 295 300
   Arg Ile Ser Asp Lys Leu Phe Leu Glu Asn Glu Glu Ala Thr Leu Lys
   305 310 315 320
   Val Ala Ala Asp Val Met Lys Ser Tyr Gly Leu Pro Val Asn Arg Asn
   325 330 335
   Thr Val Arg Leu His Asn Glu Phe Phe Gly Thr Ser Cys Pro His Arg
   340 345 350
   Ser Trp Asp Leu His Val Gly Lys Gly Glu Pro Tyr Thr Thr Thr Asn
   355 360 365
   Ile Asn Lys Met Lys Asp Tyr Phe Ile Lys Arg Ile Lys His Tyr Tyr
   370 375 380
   Asp Gly Gly Lys Leu Glu Val Ser Lys Ala Ala Thr Ile Lys Gln Ser
   385 390 395 400
   Asp Val Lys Gln Glu Val Lys Lys Gln Glu Ala Lys Gln Ile Val Lys
   405 410 415
   Ala Thr Asp Trp Lys Gln Asn Lys Asp Gly Ile Trp Tyr Lys Ala Glu
   420 425 430
   His Ala Ser Phe Thr Val Thr Ala Pro Glu Gly Ile Ile Thr Arg Tyr
   435 440 445
   Lys Gly Pro Trp Thr Gly His Pro Gln Ala Gly Val Leu Gln Lys Gly
   450 455 460
   Gln Thr Ile Lys Tyr Asp Glu Val Gln Lys Phe Asp Gly His Val Trp
   465 470 475 480
   Val Ser Trp Glu Thr Phe Glu Gly Glu Thr Val Tyr Met Pro Val Arg
   485 490 495
   Thr Trp Asp Ala Lys Thr Gly Lys Val Gly Lys Leu Trp Gly Glu Ile
   500 505 510
   Lys
<210> 79
   <211> 1542
   <212> DNA.
   <213> artificial
<220>
   <223> Pseudinl-Ply2638A
<400> 79
   atgggtctga acaccctgaa aaaagtattc cagggtctgc atgaagcgat taaactgatc 60
   aacaaccacg ttcagggatc catgctaact gctattgact atcttacgaa aaaaggttgg 120
   aaaatatcat ctgaccctcg cacttacgat ggttacccta aaaactacgg ctacagaaat 180
   taccatgaaa acggcattaa ttatgatgag ttttgtggtg gttatcatag agcttttgat 240
   gtttacagta acgaaactaa cgacgtgcct gctgttacta gcggaacagt tattgaagca 300
   aacgattacg gtaattttgg tggtacattc gttattagag acgctaacga taacgattgg 360
   atatatgggc atctacaacg tggctcaatg cgatttgttg taggcgacaa agtcaatcaa 420
   ggtgacatta ttggtttaca aggtaatagc aactattacg acaatcctat gagtgtacat 480
   ttacatttac aattacgccc taaagacgca aagaaagatg aaaaatcaca agtatgtagt 540
   ggtttggcta tggaaaaata tgacattaca aatttaaatg ctaaacaaga taaatcaaag 600
   aatgggagcg tgaaagagtt gaaacatatc tattcaaacc atattaaagg taacaagatt 660
   acagcaccaa aacctagtat tcaaggtgtg gtcatccaca atgattatgg tagtatgaca 720
   cctagtcaat acttaccatg gttatatgca cgtgagaata acggtacaca cgttaacggt 780
   tgggctagtg tttatgcaaa tagaaacgaa gtgctttggt atcatccgac agactacgta 840
   gagtggcatt gtggtaatca atgggcaaat gctaacttaa tcggatttga agtgtgtgag 900
   tcgtatcctg gtagaatctc ggacaaatta ttcttagaaa atgaagaagc gacattgaaa 960
   gtagctgcgg atgtgatgaa gtcgtacgga ttaccagtta atcgcaacac tgtacgtctg 1020
   cataacgaat tcttcggaac ttcttgtcca catcgttcgt gggacttgca tgttggcaaa 1080
   ggtgagcctt acacaactac taatattaat aaaatgaaag actacttcat caaacgcatc 1140
   aaacattatt atgacggtgg aaagctagaa gtaagcaaag cagcaactat caaacaatct 1200
   gacgttaagc aagaagttaa aaagcaagaa gcaaaacaaa ttgtgaaagc aacagattgg 1260
   aaacagaata aagatggcat ttggtataaa gctgaacatg cttcgttcac agtgacagca 1320
   ccagagggaa ttatcacaag atacaaaggt ccttggactg gtcacccaca agctggtgta 1380
   ttacaaaaag gtcaaacgat taaatatgat gaggttcaaa aatttgacgg tcatgtttgg 1440
   gtatcgtggg aaacgtttga gggcgaaact gtatacatgc cggtacgcac atgggacgct 1500
   aaaactggta aagttggtaa gttgtggggc gaaattaaat ga 1542
<210> 80
   <211> 512
   <212> PRT
   <213> artificial
<220>
   <223> Magainin-Ply2638A
<400> 80
   Met Gly Ile Gly Lys Phe Leu His Ser Ala Lys Lys Phe Gly Lys Ala
   1               5                   10                  15
   Phe Val Gly Glu Ile Met Asn Ser Gly Ser Met Leu Thr Ala Ile Asp
               20                  25                  30
   Tyr Leu Thr Lys Lys Gly Trp Lys Ile Ser Ser Asp Pro Arg Thr Tyr
           35                  40                  45
   Asp Gly Tyr Pro Lys Asn Tyr Gly Tyr Arg Asn Tyr His Glu Asn Gly
       50                  55                  60
   Ile Asn Tyr Asp Glu Phe Cys Gly Gly Tyr His Arg Ala Phe Asp Val
   65                  70                  75                  80
   Tyr Ser Asn Glu Thr Asn Asp Val Pro Ala Val Thr Ser Gly Thr Val
                   85                  90                  95
   Ile Glu Ala Asn Asp Tyr Gly Asn Phe Gly Gly Thr Phe Val Ile Arg
   100 105 110
   Asp Ala Asn Asp Asn Asp Trp Ile Tyr Gly His Leu Gln Arg Gly Ser
   115 120 125
   Met Arg Phe Val Val Gly Asp Lys Val Asn Gln Gly Asp Ile Ile Gly
   130 135 140
   Leu Gln Gly Asn Ser Asn Tyr Tyr Asp Asn Pro Met Ser Val His Leu
   145 150 155 160
   His Leu Gln Leu Arg Pro Lys Asp Ala Lys Lys Asp Glu Lys Ser Gln
   165 170 175
   Val Cys Ser Gly Leu Ala Met Glu Lys Tyr Asp Ile Thr Asn Leu Asn
   180 185 190
   Ala Lys Gln Asp Lys Ser Lys Asn Gly Ser Val Lys Glu Leu Lys His
   195 200 205
   Ile Tyr Ser Asn His Ile Lys Gly Asn Lys Ile Thr Ala Pro Lys Pro
   210 215 220
   Ser Ile Gln Gly Val Val Ile His Asn Asp Tyr Gly Ser Met Thr Pro
   225 230 235 240
   Ser Gln Tyr Leu Pro Trp Leu Tyr Ala Arg Glu Asn Asn Gly Thr His
   245 250 255
   Val Asn Gly Trp Ala Ser Val Tyr Ala Asn Arg Asn Glu Val Leu Trp
   260 265 270
   Tyr His Pro Thr Asp Tyr Val Glu Trp His Cys Gly Asn Gln Trp Ala
   275 280 285
   Asn Ala Asn Leu Ile Gly Phe Glu Val Cys Glu Ser Tyr Pro Gly Arg
   290 295 300
   Ile Ser Asp Lys Leu Phe Leu Glu Asn Glu Glu Ala Thr Leu Lys Val
   305 310 315 320
   Ala Ala Asp Val Met Lys Ser Tyr Gly Leu Pro Val Asn Arg Asn Thr
   325 330 335
   Val Arg Leu His Asn Glu Phe Phe Gly Thr Ser Cys Pro His Arg Ser
   340 345 350
   Trp Asp Leu His Val Gly Lys Gly Glu Pro Tyr Thr Thr Thr Asn Ile
   355 360 365
   Asn Lys Met Lys Asp Tyr Phe Ile Lys Arg Ile Lys His Tyr Tyr Asp
   370 375 380
   Gly Gly Lys Leu Glu Val Ser Lys Ala Ala Thr Ile Lys Gln Ser Asp
   385 390 395 400
   Val Lys Gln Glu Val Lys Lys Gln Glu Ala Lys Gln Ile Val Lys Ala
   405 410 415
   Thr Asp Trp Lys Gln Asn Lys Asp Gly Ile Trp Tyr Lys Ala Glu His
   420 425 430
   Ala Ser Phe Thr Val Thr Ala Pro Glu Gly Ile Ile Thr Arg Tyr Lys
   435 440 445
   Gly Pro Trp Thr Gly His Pro Gln Ala Gly Val Leu Gln Lys Gly Gln
   450 455 460
   Thr Ile Lys Tyr Asp Glu Val Gln Lys Phe Asp Gly His Val Trp Val
   465 470 475 480
   Ser Trp Glu Thr Phe Glu Gly Glu Thr Val Tyr Met Pro Val Arg Thr
   485 490 495
   Trp Asp Ala Lys Thr Gly Lys Val Gly Lys Leu Trp Gly Glu Ile Lys
   500 505 510
<210> 81
   <211> 1539
   <212> **DNA**
   <213> artificial
<220>
   <223> Magainin-Ply2638A
<400> 81
   atgggtatcg gcaaattcct gcactccgca aaaaaattcg gcaaagcttt cgtgggcgaa 60
   attatgaact ctggatccat gctaactgct attgactatc ttacgaaaaa aggttggaaa 120
   atatcatctg accctcgcac ttacgatggt taccctaaaa actacggcta cagaaattac 180
   catgaaaacg gcattaatta tgatgagttt tgtggtggtt atcatagagc ttttgatgtt 240
   tacagtaacg aaactaacga cgtgcctgct gttactagcg gaacagttat tgaagcaaac 300
   gattacggta attttggtgg tacattcgtt attagagacg ctaacgataa cgattggata 360
   tatgggcatc tacaacgtgg ctcaatgcga tttgttgtag gcgacaaagt caatcaaggt    420
   gacattattg gtttacaagg taatagcaac tattacgaca atcctatgag tgtacattta    480
   catttacaat tacgccctaa agacgcaaag aaagatgaaa aatcacaagt atgtagtggt    540
   ttggctatgg aaaaatatga cattacaaat ttaaatgcta aacaagataa atcaaagaat    600
   gggagcgtga aagagttgaa acatatctat tcaaaccata ttaaaggtaa caagattaca    660
   gcaccaaaac ctagtattca aggtgtggtc atccacaatg attatggtag tatgacacct    720
   agtcaatact taccatggtt atatgcacgt gagaataacg gtacacacgt taacggttgg    780
   gctagtgttt atgcaaatag aaacgaagtg ctttggtatc atccgacaga ctacgtagag    840
   tggcattgtg gtaatcaatg ggcaaatgct aacttaatcg gatttgaagt gtgtgagtcg    900
   tatcctggta gaatctcgga caaattattc ttagaaaatg aagaagcgac attgaaagta    960
   gctgcggatg tgatgaagtc gtacggatta ccagttaatc gcaacactgt acgtctgcat   1020
   aacgaattct tcggaacttc ttgtccacat cgttcgtggg acttgcatgt tggcaaaggt   1080
   gagccttaca caactactaa tattaataaa atgaaagact acttcatcaa acgcatcaaa   1140
   cattattatg acggtggaaa gctagaagta agcaaagcag caactatcaa acaatctgac   1200
   gttaagcaag aagttaaaaa gcaagaagca aaacaaattg tgaaagcaac agattggaaa   1260
   cagaataaag atggcatttg gtataaagct gaacatgctt cgttcacagt gacagcacca   1320
   gagggaatta tcacaagata caaaggtcct tggactggtc acccacaagc tggtgtatta   1380
   caaaaaggtc aaacgattaa atatgatgag gttcaaaaat ttgacggtca tgtttgggta   1440
   tcgtgggaaa cgtttgaggg cgaaactgta tacatgccgg tacgcacatg ggacgctaaa   1500
   actggtaaag ttggtaagtt gtggggcgaa attaaatga                          1539
<210> 82
   <211> 514
   <212> PRT
   <213> artificial
<220>
   <223> Pleurocidin-Ply2638A
<400> 82
   Met Gly Trp Gly Ser Phe Phe Lys Lys Ala Ala His Val Gly Lys His
   1               5                   10                  15
   Val Gly Lys Ala Ala Leu Thr His Tyr Leu Gly Ser Met Leu Thr Ala
               20                  25                  30
   Ile Asp Tyr Leu Thr Lys Lys Gly Trp Lys Ile Ser Ser Asp Pro Arg
           35                  40                  45
   Thr Tyr Asp Gly Tyr Pro Lys Asn Tyr Gly Tyr Arg Asn Tyr His Glu
   50 55 60
   Asn Gly Ile Asn Tyr Asp Glu Phe Cys Gly Gly Tyr His Arg Ala Phe
   65 70 75 80
   Asp Val Tyr Ser Asn Glu Thr Asn Asp Val Pro Ala Val Thr Ser Gly
   85 90 95
   Thr Val Ile Glu Ala Asn Asp Tyr Gly Asn Phe Gly Gly Thr Phe Val
   100 105 110
   Ile Arg Asp Ala Asn Asp Asn Asp Trp Ile Tyr Gly His Leu Gln Arg
   115 120 125
   Gly Ser Met Arg Phe Val Val Gly Asp Lys Val Asn Gln Gly Asp Ile
   130 135 140
   Ile Gly Leu Gln Gly Asn Ser Asn Tyr Tyr Asp Asn Pro Met Ser Val
   145 150 155 160
   His Leu His Leu Gln Leu Arg Pro Lys Asp Ala Lys Lys Asp Glu Lys
   165 170 175
   Ser Gln Val Cys Ser Gly Leu Ala Met Glu Lys Tyr Asp Ile Thr Asn
   180 185 190
   Leu Asn Ala Lys Gln Asp Lys Ser Lys Asn Gly Ser Val Lys Glu Leu
   195 200 205
   Lys His Ile Tyr Ser Asn His Ile Lys Gly Asn Lys Ile Thr Ala Pro
   210 215 220
   Lys Pro Ser Ile Gln Gly Val Val Ile His Asn Asp Tyr Gly Ser Met
   225 230 235 240
   Thr Pro Ser Gln Tyr Leu Pro Trp Leu Tyr Ala Arg Glu Asn Asn Gly
   245 250 255
   Thr His Val Asn Gly Trp Ala Ser Val Tyr Ala Asn Arg Asn Glu Val
   260 265 270
   Leu Trp Tyr His Pro Thr Asp Tyr Val Glu Trp His Cys Gly Asn Gln
   275 280 285
   Trp Ala Asn Ala Asn Leu Ile Gly Phe Glu Val Cys Glu Ser Tyr Pro
   290 295 300
   Gly Arg Ile Ser Asp Lys Leu Phe Leu Glu Asn Glu Glu Ala Thr Leu
   305 310 315 320
   Lys Val Ala Ala Asp Val Met Lys Ser Tyr Gly Leu Pro Val Asn Arg
   325 330 335
   Asn Thr Val Arg Leu His Asn Glu Phe Phe Gly Thr Ser Cys Pro His
   340 345 350
   Arg Ser Trp Asp Leu His Val Gly Lys Gly Glu Pro Tyr Thr Thr Thr
   355 360 365
   Asn Ile Asn Lys Met Lys Asp Tyr Phe Ile Lys Arg Ile Lys His Tyr
   370 375 380
   Tyr Asp Gly Gly Lys Leu Glu Val Ser Lys Ala Ala Thr Ile Lys Gln
   385 390 395 400
   Ser Asp Val Lys Gln Glu Val Lys Lys Gln Glu Ala Lys Gln Ile Val
   405 410 415
   Lys Ala Thr Asp Trp Lys Gln Asn Lys Asp Gly Ile Trp Tyr Lys Ala
   420 425 430
   Glu His Ala Ser Phe Thr Val Thr Ala Pro Glu Gly Ile Ile Thr Arg
   435 440 445
   Tyr Lys Gly Pro Trp Thr Gly His Pro Gln Ala Gly Val Leu Gln Lys
   450 455 460
   Gly Gln Thr Ile Lys Tyr Asp Glu Val Gln Lys Phe Asp Gly His Val
   465 470 475 480
   Trp Val Ser Trp Glu Thr Phe Glu Gly Glu Thr Val Tyr Met Pro Val
   485 490 495
   Arg Thr Trp Asp Ala Lys Thr Gly Lys Val Gly Lys Leu Trp Gly Glu
   500 505 510
   Ile Lys
<210> 83
   <211> 1545
   <212> DNA
   <213> artificial
<220>
   <223> Pleurocidin-Ply2638A
<400> 83
   atgggctggg gttctttctt taaaaaagcg gctcacgttg gcaaacatgt aggtaaagca     60
   gctctgaccc actatctggg atccatgcta actgctattg actatcttac gaaaaaaggt    120
   tggaaaatat catctgaccc tcgcacttac gatggttacc ctaaaaacta cggctacaga    180
   aattaccatg aaaacggcat taattatgat gagttttgtg gtggttatca tagagctttt    240
   gatgtttaca gtaacgaaac taacgacgtg cctgctgtta ctagcggaac agttattgaa    300
   gcaaacgatt acggtaattt tggtggtaca ttcgttatta gagacgctaa cgataacgat    360
   tggatatatg ggcatctaca acgtggctca atgcgatttg ttgtaggcga caaagtcaat    420
   caaggtgaca ttattggttt acaaggtaat agcaactatt acgacaatcc tatgagtgta    480
   catttacatt tacaattacg ccctaaagac gcaaagaaag atgaaaaatc acaagtatgt    540
   agtggtttgg ctatggaaaa atatgacatt acaaatttaa atgctaaaca agataaatca    600
   aagaatggga gcgtgaaaga gttgaaacat atctattcaa accatattaa aggtaacaag    660
   attacagcac caaaacctag tattcaaggt gtggtcatcc acaatgatta tggtagtatg    720
   acacctagtc aatacttacc atggttatat gcacgtgaga ataacggtac acacgttaac    780
   ggttgggcta gtgtttatgc aaatagaaac gaagtgcttt ggtatcatcc gacagactac    840
   gtagagtggc attgtggtaa tcaatgggca aatgctaact taatcggatt tgaagtgtgt    900
   gagtcgtatc ctggtagaat ctcggacaaa ttattcttag aaaatgaaga agcgacattg    960
   aaagtagctg cggatgtgat gaagtcgtac ggattaccag ttaatcgcaa cactgtacgt   1020
   ctgcataacg aattcttcgg aacttcttgt ccacatcgtt cgtgggactt gcatgttggc   1080
   aaaggtgagc cttacacaac tactaatatt aataaaatga aagactactt catcaaacgc   1140
   atcaaacatt attatgacgg tggaaagcta gaagtaagca aagcagcaac tatcaaacaa   1200
   tctgacgtta agcaagaagt taaaaagcaa gaagcaaaac aaattgtgaa agcaacagat   1260
   tggaaacaga ataaagatgg catttggtat aaagctgaac atgcttcgtt cacagtgaca   1320
   gcaccagagg gaattatcac aagatacaaa ggtccttgga ctggtcaccc acaagctggt   1380
   gtattacaaa aaggtcaaac gattaaatat gatgaggttc aaaaatttga cggtcatgtt   1440
   tgggtatcgt gggaaacgtt tgagggcgaa actgtataca tgccggtacg cacatgggac   1500
   gctaaaactg gtaaagttgg taagttgtgg ggcgaaatta aatga                    1545
<210> 84
   <211> 510
   <212> PRT
   <213> artificial
<220>
   <223> BuforinII-Ply2638A
<400> 84
   Met Thr Arg Ser Ser Arg Ala Gly Leu Gln Phe Pro Val Gly Arg Val
   1               5                   10                  15
   His Arg Leu Leu Arg Lys Gly Ser Met Leu Thr Ala Ile Asp Tyr Leu
               20                  25                  30
   Thr Lys Lys Gly Trp Lys Ile Ser Ser Asp Pro Arg Thr Tyr Asp Gly
           35                  40                  45
   Tyr Pro Lys Asn Tyr Gly Tyr Arg Asn Tyr His Glu Asn Gly Ile Asn
       50                  55                  60
   Tyr Asp Glu Phe Cys Gly Gly Tyr His Arg Ala Phe Asp Val Tyr Ser
   65                  70                  75                  80
   Asn Glu Thr Asn Asp Val Pro Ala Val Thr Ser Gly Thr Val Ile Glu
                   85                  90                  95
   Ala Asn Asp Tyr Gly Asn Phe Gly Gly Thr Phe Val Ile Arg Asp Ala
               100                 105                 110
   Asn Asp Asn Asp Trp Ile Tyr Gly His Leu Gln Arg Gly Ser Met Arg
           115                 120                 125
   Phe Val Val Gly Asp Lys Val Asn Gln Gly Asp Ile Ile Gly Leu Gln
       130                 135                 140
   Gly Asn Ser Asn Tyr Tyr Asp Asn Pro Met Ser Val His Leu His Leu
   145                 150                 155                 160
   Gln Leu Arg Pro Lys Asp Ala Lys Lys Asp Glu Lys Ser Gln Val Cys
                   165                 170                 175
   Ser Gly Leu Ala Met Glu Lys Tyr Asp Ile Thr Asn Leu Asn Ala Lys
               180                 185                 190
   Gln Asp Lys Ser Lys Asn Gly Ser Val Lys Glu Leu Lys His Ile Tyr
           195                 200                 205
   Ser Asn His Ile Lys Gly Asn Lys Ile Thr Ala Pro Lys Pro Ser Ile
       210                 215                 220
   Gln Gly Val Val Ile His Asn Asp Tyr Gly Ser Met Thr Pro Ser Gln
   225                 230                 235                 240
   Tyr Leu Pro Trp Leu Tyr Ala Arg Glu Asn Asn Gly Thr His Val Asn
                   245                 250                 255
   Gly Trp Ala Ser Val Tyr Ala Asn Arg Asn Glu Val Leu Trp Tyr His
               260                 265                 270
   Pro Thr Asp Tyr Val Glu Trp His Cys Gly Asn Gln Trp Ala Asn Ala
           275                 280                 285
   Asn Leu Ile Gly Phe Glu Val Cys Glu Ser Tyr Pro Gly Arg Ile Ser
       290                 295                 300
   Asp Lys Leu Phe Leu Glu Asn Glu Glu Ala Thr Leu Lys Val Ala Ala
   305                 310                 315                 320
   Asp Val Met Lys Ser Tyr Gly Leu Pro Val Asn Arg Asn Thr Val Arg
                   325                 330                 335
   Leu His Asn Glu Phe Phe Gly Thr Ser Cys Pro His Arg Ser Trp Asp
               340                 345                 350
   Leu His Val Gly Lys Gly Glu Pro Tyr Thr Thr Thr Asn Ile Asn Lys
           355                 360                 365
   Met Lys Asp Tyr Phe Ile Lys Arg Ile Lys His Tyr Tyr Asp Gly Gly
       370                 375                 380
   Lys Leu Glu Val Ser Lys Ala Ala Thr Ile Lys Gln Ser Asp Val Lys
   385                 390                 395                 400
   Gln Glu Val Lys Lys Gln Glu Ala Lys Gln Ile Val Lys Ala Thr Asp
                   405                 410                 415
   Trp Lys Gln Asn Lys Asp Gly Ile Trp Tyr Lys Ala Glu His Ala Ser
               420                 425                 430
   Phe Thr Val Thr Ala Pro Glu Gly Ile Ile Thr Arg Tyr Lys Gly Pro
           435                 440                 445
   Trp Thr Gly His Pro Gln Ala Gly Val Leu Gln Lys Gly Gln Thr Ile
       450                 455                 460
   Lys Tyr Asp Glu Val Gln Lys Phe Asp Gly His Val Trp Val Ser Trp
   465                 470                 475                 480
   Glu Thr Phe Glu Gly Glu Thr Val Tyr Met Pro Val Arg Thr Trp Asp
                   485                 490                 495
   Ala Lys Thr Gly Lys Val Gly Lys Leu Trp Gly Glu Ile Lys
   500 505 510
<210> 85
   <211> 1533
   <212> DNA
   <213> artificial
<220>
   <223> BuforinII-Ply2638A
<400> 85
   atgacccgta gctctcgtgc tggcctgcag tttccggttg gtcgcgtgca ccgtctgctc     60
   cgcaaaggat ccatgctaac tgctattgac tatcttacga aaaaaggttg gaaaatatca    120
   tctgaccctc gcacttacga tggttaccct aaaaactacg gctacagaaa ttaccatgaa    180
   aacggcatta attatgatga gttttgtggt ggttatcata gagcttttga tgtttacagt    240
   aacgaaacta acgacgtgcc tgctgttact agcggaacag ttattgaagc aaacgattac    300
   ggtaattttg gtggtacatt cgttattaga gacgctaacg ataacgattg gatatatggg    360
   catctacaac gtggctcaat gcgatttgtt gtaggcgaca aagtcaatca aggtgacatt    420
   attggtttac aaggtaatag caactattac gacaatccta tgagtgtaca tttacattta    480
   caattacgcc ctaaagacgc aaagaaagat gaaaaatcac aagtatgtag tggtttggct    540
   atggaaaaat atgacattac aaatttaaat gctaaacaag ataaatcaaa gaatgggagc    600
   gtgaaagagt tgaaacatat ctattcaaac catattaaag gtaacaagat tacagcacca    660
   aaacctagta ttcaaggtgt ggtcatccac aatgattatg gtagtatgac acctagtcaa    720
   tacttaccat ggttatatgc acgtgagaat aacggtacac acgttaacgg ttgggctagt    780
   gtttatgcaa atagaaacga agtgctttgg tatcatccga cagactacgt agagtggcat    840
   tgtggtaatc aatgggcaaa tgctaactta atcggatttg aagtgtgtga gtcgtatcct    900
   ggtagaatct cggacaaatt attcttagaa aatgaagaag cgacattgaa agtagctgcg    960
   gatgtgatga agtcgtacgg attaccagtt aatcgcaaca ctgtacgtct gcataacgaa   1020
   ttcttcggaa cttcttgtcc acatcgttcg tgggacttgc atgttggcaa aggtgagcct   1080
   tacacaacta ctaatattaa taaaatgaaa gactacttca tcaaacgcat caaacattat   1140
   tatgacggtg gaaagctaga agtaagcaaa gcagcaacta tcaaacaatc tgacgttaag   1200
   caagaagtta aaaagcaaga agcaaaacaa attgtgaaag caacagattg gaaacagaat   1260
   aaagatggca tttggtataa agctgaacat gcttcgttca cagtgacagc accagaggga   1320
   attatcacaa gatacaaagg tccttggact ggtcacccac aagctggtgt attacaaaaa   1380
   ggtcaaacga ttaaatatga tgaggttcaa aaatttgacg gtcatgtttg ggtatcgtgg   1440
   gaaacgtttg agggcgaaac tgtatacatg ccggtacgca catggacgca taaaactggt   1500
   aaagttggta agttgtgggg cgaaattaaa tga                                1533
<210> 86
   <211> 524
   <212> PRT
   <213> artificial
<220>
   <223> Ply2638A-LL-37
<400> 86
   Met Leu Thr Ala Ile Asp Tyr Leu Thr Lys Lys Gly Trp Lys Ile Ser
   1               5                   10                  15
   Ser Asp Pro Arg Thr Tyr Asp Gly Tyr Pro Lys Asn Tyr Gly Tyr Arg
               20                  25                  30
   Asn Tyr His Glu Asn Gly Ile Asn Tyr Asp Glu Phe Cys Gly Gly Tyr
           35                  40                  45
   His Arg Ala Phe Asp Val Tyr Ser Asn Glu Thr Asn Asp Val Pro Ala
       50                  55                  60
   Val Thr Ser Gly Thr Val Ile Glu Ala Asn Asp Tyr Gly Asn Phe Gly
   65                  70                  75                  80
   Gly Thr Phe Val Ile Arg Asp Ala Asn Asp Asn Asp Trp Ile Tyr Gly
                   85                  90                  95
   His Leu Gln Arg Gly Ser Met Arg Phe Val Val Gly Asp Lys Val Asn
               100                 105                 110
   Gln Gly Asp Ile Ile Gly Leu Gln Gly Asn Ser Asn Tyr Tyr Asp Asn
           115                 120                 125
   Pro Met Ser Val His Leu His Leu Gln Leu Arg Pro Lys Asp Ala Lys
       130                 135                 140
   Lys Asp Glu Lys Ser Gln Val Cys Ser Gly Leu Ala Met Glu Lys Tyr
   145                 150                 155                 160
   Asp Ile Thr Asn Leu Asn Ala Lys Gln Asp Lys Ser Lys Asn Gly Ser
                   165                 170                 175
   Val Lys Glu Leu Lys His Ile Tyr Ser Asn His Ile Lys Gly Asn Lys
               180                 185                 190
   Ile Thr Ala Pro Lys Pro Ser Ile Gln Gly Val Val Ile His Asn Asp
   195 200 205
   Tyr Gly Ser Met Thr Pro Ser Gln Tyr Leu Pro Trp Leu Tyr Ala Arg
   210 215 220
   Glu Asn Asn Gly Thr His Val Asn Gly Trp Ala Ser Val Tyr Ala Asn
   225 230 235 240
   Arg Asn Glu Val Leu Trp Tyr His Pro Thr Asp Tyr Val Glu Trp His
   245 250 255
   Cys Gly Asn Gln Trp Ala Asn Ala Asn Leu Ile Gly Phe Glu Val Cys
   260 265 270
   Glu Ser Tyr Pro Gly Arg Ile Ser Asp Lys Leu Phe Leu Glu Asn Glu
   275 280 285
   Glu Ala Thr Leu Lys Val Ala Ala Asp Val Met Lys Ser Tyr Gly Leu
   290 295 300
   Pro Val Asn Arg Asn Thr Val Arg Leu His Asn Glu Phe Phe Gly Thr
   305 310 315 320
   Ser Cys Pro His Arg Ser Trp Asp Leu His Val Gly Lys Gly Glu Pro
   325 330 335
   Tyr Thr Thr Thr Asn Ile Asn Lys Met Lys Asp Tyr Phe Ile Lys Arg
   340 345 350
   Ile Lys His Tyr Tyr Asp Gly Gly Lys Leu Glu Val Ser Lys Ala Ala
   355 360 365
   Thr Ile Lys Gln Ser Asp Val Lys Gln Glu Val Lys Lys Gln Glu Ala
   370 375 380
   Lys Gln Ile Val Lys Ala Thr Asp Trp Lys Gln Asn Lys Asp Gly Ile
   385 390 395 400
   Trp Tyr Lys Ala Glu His Ala Ser Phe Thr Val Thr Ala Pro Glu Gly
   405 410 415
   Ile Ile Thr Arg Tyr Lys Gly Pro Trp Thr Gly His Pro Gln Ala Gly
   420 425 430
   Val Leu Gln Lys Gly Gln Thr Ile Lys Tyr Asp Glu Val Gln Lys Phe
   435 440 445
   Asp Gly His Val Trp Val Ser Trp Glu Thr Phe Glu Gly Glu Thr Val
   450 455 460
   Tyr Met Pro Val Arg Thr Trp Asp Ala Lys Thr Gly Lys Val Gly Lys
   465 470 475 480
   Leu Trp Gly Glu Ile Lys Ser Leu Leu Gly Asp Phe Phe Arg Lys Ser
   485 490 495
   Lys Glu Lys Ile Gly Lys Glu Phe Lys Arg Ile Val Gln Arg Ile Lys
   500 505 510
   Asp Phe Leu Arg Asn Leu Val Pro Arg Thr Glu Ser
   515 520
<210> 87
   <211> 1575
   <212> DNA
   <213> artificial
<220>
   <223> P1y2638A-LL-37
<400> 87
   atgctaactg ctattgacta tcttacgaaa aaaggttgga aaatatcatc tgaccctcgc 60
   acttacgatg gttaccctaa aaactacggc tacagaaatt accatgaaaa cggcattaat 120
   tatgatgagt tttgtggtgg ttatcataga gcttttgatg tttacagtaa cgaaactaac 180
   gacgtgcctg ctgttactag cggaacagtt attgaagcaa acgattacgg taattttggt 240
   ggtacattcg ttattagaga cgctaacgat aacgattgga tatatgggca tctacaacgt 300
   ggctcaatgc gatttgttgt aggcgacaaa gtcaatcaag gtgacattat tggtttacaa 360
   ggtaatagca actattacga caatcctatg agtgtacatt tacatttaca attacgccct 420
   aaagacgcaa agaaagatga aaaatcacaa gtatgtagtg gtttggctat ggaaaaatat 480
   gacattacaa atttaaatgc taaacaagat aaatcaaaga atgggagcgt gaaagagttg 540
   aaacatatct attcaaacca tattaaaggt aacaagatta cagcaccaaa acctagtatt 600
   caaggtgtgg tcatccacaa tgattatggt agtatgacac ctagtcaata cttaccatgg 660
   ttatatgcac gtgagaataa cggtacacac gttaacggtt gggctagtgt ttatgcaaat 720
   agaaacgaag tgctttggta tcatccgaca gactacgtag agtggcattg tggtaatcaa 780
   tgggcaaatg ctaacttaat cggatttgaa gtgtgtgagt cgtatcctgg tagaatctcg 840
   gacaaattat tcttagaaaa tgaagaagcg acattgaaag tagctgcgga tgtgatgaag 900
   tcgtacggat taccagttaa tcgcaacact gtacgtctgc ataacgaatt cttcggaact 960
   tcttgtccac atcgttcgtg ggacttgcat gttggcaaag gtgagcctta cacaactact 1020
   aatattaata aaatgaaaga ctacttcatc aaacgcatca aacattatta tgacggtgga 1080
   aagctagaag taagcaaagc agcaactatc aaacaatctg acgttaagca agaagttaaa 1140
   aagcaagaag caaaacaaat tgtgaaagca acagattgga aacagaataa agatggcatt 1200
   tggtataaag ctgaacatgc ttcgttcaca gtgacagcac cagagggaat tatcacaaga 1260
   tacaaaggtc cttggactgg tcacccacaa gctggtgtat tacaaaaagg tcaaacgatt 1320
   aaatatgatg aggttcaaaa atttgacggt catgtttggg tatcgtggga aacgtttgag 1380
   ggcgaaactg tatacatgcc ggtacgcaca tgggacgcta aaactggtaa agttggtaag 1440
   ttgtggggcg aaattaaatc cctcctgggt gacttctttc gcaaatccaa agagaaaatc 1500
   ggcaaagagt tcaaacgtat cgtgcagcgc attaaagact ttctgcgtaa cctggttccg 1560
   cgtaccgaat cttaa 1575
<210> 88
   <211> 516
   <212> PRT
   <213> artificial
<220>
   <223> Ply2638A-SMAP-29
<400> 88
   Met Leu Thr Ala Ile Asp Tyr Leu Thr Lys Lys Gly Trp Lys Ile Ser
   1 5 10 15
   Ser Asp Pro Arg Thr Tyr Asp Gly Tyr Pro Lys Asn Tyr Gly Tyr Arg
   20 25 30
   Asn Tyr His Glu Asn Gly Ile Asn Tyr Asp Glu Phe Cys Gly Gly Tyr
   35 40 45
   His Arg Ala Phe Asp Val Tyr Ser Asn Glu Thr Asn Asp Val Pro Ala
   50 55 60
   Val Thr Ser Gly Thr Val Ile Glu Ala Asn Asp Tyr Gly Asn Phe Gly
   65 70 75 80
   Gly Thr Phe Val Ile Arg Asp Ala Asn Asp Asn Asp Trp Ile Tyr Gly
   85 90 95
   His Leu Gln Arg Gly Ser Met Arg Phe Val Val Gly Asp Lys Val Asn
   100 105 110
   Gln Gly Asp Ile Ile Gly Leu Gln Gly Asn Ser Asn Tyr Tyr Asp Asn
   115 120 125
   Pro Met Ser Val His Leu His Leu Gln Leu Arg Pro Lys Asp Ala Lys
   130 135 140
   Lys Asp Glu Lys Ser Gln Val Cys Ser Gly Leu Ala Met Glu Lys Tyr
   145 150 155 160
   Asp Ile Thr Asn Leu Asn Ala Lys Gln Asp Lys Ser Lys Asn Gly Ser
   165 170 175
   Val Lys Glu Leu Lys His Ile Tyr Ser Asn His Ile Lys Gly Asn Lys
   180 185 190
   Ile Thr Ala Pro Lys Pro Ser Ile Gln Gly Val Val Ile His Asn Asp
   195 200 205
   Tyr Gly Ser Met Thr Pro Ser Gln Tyr Leu Pro Trp Leu Tyr Ala Arg
   210 215 220
   Glu Asn Asn Gly Thr His Val Asn Gly Trp Ala Ser Val Tyr Ala Asn
   225 230 235 240
   Arg Asn Glu Val Leu Trp Tyr His Pro Thr Asp Tyr Val Glu Trp His
   245 250 255
   Cys Gly Asn Gln Trp Ala Asn Ala Asn Leu Ile Gly Phe Glu Val Cys
   260 265 270
   Glu Ser Tyr Pro Gly Arg Ile Ser Asp Lys Leu Phe Leu Glu Asn Glu
   275 280 285
   Glu Ala Thr Leu Lys Val Ala Ala Asp Val Met Lys Ser Tyr Gly Leu
   290 295 300
   Pro Val Asn Arg Asn Thr Val Arg Leu His Asn Glu Phe Phe Gly Thr
   305 310 315 320
   Ser Cys Pro His Arg Ser Trp Asp Leu His Val Gly Lys Gly Glu Pro
   325 330 335
   Tyr Thr Thr Thr Asn Ile Asn Lys Met Lys Asp Tyr Phe Ile Lys Arg
   340 345 350
   Ile Lys His Tyr Tyr Asp Gly Gly Lys Leu Glu Val Ser Lys Ala Ala
   355 360 365
   Thr Ile Lys Gln Ser Asp Val Lys Gln Glu Val Lys Lys Gln Glu Ala
   370 375 380
   Lys Gln Ile Val Lys Ala Thr Asp Trp Lys Gln Asn Lys Asp Gly Ile
   385 390 395 400
   Trp Tyr Lys Ala Glu His Ala Ser Phe Thr Val Thr Ala Pro Glu Gly
   405 410 415
   Ile Ile Thr Arg Tyr Lys Gly Pro Trp Thr Gly His Pro Gln Ala Gly
   420 425 430
   Val Leu Gln Lys Gly Gln Thr Ile Lys Tyr Asp Glu Val Gln Lys Phe
   435 440 445
   Asp Gly His Val Trp Val Ser Trp Glu Thr Phe Glu Gly Glu Thr Val
   450 455 460
   Tyr Met Pro Val Arg Thr Trp Asp Ala Lys Thr Gly Lys Val Gly Lys
   465 470 475 480
   Leu Trp Gly Glu Ile Lys Ser Arg Gly Leu Arg Arg Leu Gly Arg Lys
   485 490 495
   Ile Ala His Gly Val Lys Lys Tyr Gly Pro Thr Val Leu Arg Ile Ile
   500 505 510
   Arg Ile Ala Gly
   515
<210> 89
   <211> 1551
   <212> DNA
   <213> artificial
<220>
   <223> Ply2638A-SMAP-29
<400> 89
   atgctaactg ctattgacta tcttacgaaa aaaggttgga aaatatcatc tgaccctcgc 60
   acttacgatg gttaccctaa aaactacggc tacagaaatt accatgaaaa cggcattaat 120
   tatgatgagt tttgtggtgg ttatcataga gcttttgatg tttacagtaa cgaaactaac 180
   gacgtgcctg ctgttactag cggaacagtt attgaagcaa acgattacgg taattttggt 240
   ggtacattcg ttattagaga cgctaacgat aacgattgga tatatgggca tctacaacgt 300
   ggctcaatgc gatttgttgt aggcgacaaa gtcaatcaag gtgacattat tggtttacaa 360
   ggtaatagca actattacga caatcctatg agtgtacatt tacatttaca attacgccct 420
   aaagacgcaa agaaagatga aaaatcacaa gtatgtagtg gtttggctat ggaaaaatat 480
   gacattacaa atttaaatgc taaacaagat aaatcaaaga atgggagcgt gaaagagttg 540
   aaacatatct attcaaacca tattaaaggt aacaagatta cagcaccaaa acctagtatt     600
   caaggtgtgg tcatccacaa tgattatggt agtatgacac ctagtcaata cttaccatgg     660
   ttatatgcac gtgagaataa cggtacacac gttaacggtt gggctagtgt ttatgcaaat     720
   agaaacgaag tgctttggta tcatccgaca gactacgtag agtggcattg tggtaatcaa     780
   tgggcaaatg ctaacttaat cggatttgaa gtgtgtgagt cgtatcctgg tagaatctcg     840
   gacaaattat tcttagaaaa tgaagaagcg acattgaaag tagctgcgga tgtgatgaag     900
   tcgtacggat taccagttaa tcgcaacact gtacgtctgc ataacgaatt cttcggaact     960
   tcttgtccac atcgttcgtg ggacttgcat gttggcaaag gtgagcctta cacaactact    1020
   aatattaata aaatgaaaga ctacttcatc aaacgcatca aacattatta tgacggtgga    1080
   aagctagaag taagcaaagc agcaactatc aaacaatctg acgttaagca agaagttaaa    1140
   aagcaagaag caaaacaaat tgtgaaagca acagattgga aacagaataa agatggcatt    1200
   tggtataaag ctgaacatgc ttcgttcaca gtgacagcac cagagggaat tatcacaaga    1260
   tacaaaggtc cttggactgg tcacccacaa gctggtgtat tacaaaaagg tcaaacgatt    1320
   aaatatgatg aggttcaaaa atttgacggt catgtttggg tatcgtggga aacgtttgag    1380
   ggcgaaactg tatacatgcc ggtacgcaca tgggacgcta aaactggtaa agttggtaag    1440
   ttgtggggcg aaattaaatc ccgtggtctg cgtcgcctgg gtcgcaaaat tgcgcacggc    1500
   gtcaaaaaat acggcccgac cgtgctgcgc attatccgca tcgctggtta a             1551
<210> 90
   <211> 523
   <212> PRT
   <213> artificial
<220>
   <223> Ply2638A-Cecropin A (A.aegypti)
<400> 90
   Met Leu Thr Ala Ile Asp Tyr Leu Thr Lys Lys Gly Trp Lys Ile Ser
   1               5                   10                  15
   Ser Asp Pro Arg Thr Tyr Asp Gly Tyr Pro Lys Asn Tyr Gly Tyr Arg
               20                  25                  30
   Asn Tyr His Glu Asn Gly Ile Asn Tyr Asp Glu Phe Cys Gly Gly Tyr
           35                  40                  45
   His Arg Ala Phe Asp Val Tyr Ser Asn Glu Thr Asn Asp Val Pro Ala
       50                  55                  60
   Val Thr Ser Gly Thr Val Ile Glu Ala Asn Asp Tyr Gly Asn Phe Gly
   65                  70                  75                  80
   Gly Thr Phe Val Ile Arg Asp Ala Asn Asp Asn Asp Trp Ile Tyr Gly
                   85                  90                  95
   His Leu Gln Arg Gly Ser Met Arg Phe Val Val Gly Asp Lys Val Asn
               100                 105                 110
   Gln Gly Asp Ile Ile Gly Leu Gln Gly Asn Ser Asn Tyr Tyr Asp Asn
           115                 120                 125
   Pro Met Ser Val His Leu His Leu Gln Leu Arg Pro Lys Asp Ala Lys
       130                 135                 140
   Lys Asp Glu Lys Ser Gln Val Cys Ser Gly Leu Ala Met Glu Lys Tyr
   145                 150                 155                 160
   Asp Ile Thr Asn Leu Asn Ala Lys Gln Asp Lys Ser Lys Asn Gly Ser
                   165                 170                 175
   Val Lys Glu Leu Lys His Ile Tyr Ser Asn His Ile Lys Gly Asn Lys
               180                 185                 190
   Ile Thr Ala Pro Lys Pro Ser Ile Gln Gly Val Val Ile His Asn Asp
           195                 200                 205
   Tyr Gly Ser Met Thr Pro Ser Gln Tyr Leu Pro Trp Leu Tyr Ala Arg
       210                 215                 220
   Glu Asn Asn Gly Thr His Val Asn Gly Trp Ala Ser Val Tyr Ala Asn
   225                 230                 235                 240
   Arg Asn Glu Val Leu Trp Tyr His Pro Thr Asp Tyr Val Glu Trp His
                   245                 250                 255
   Cys Gly Asn Gln Trp Ala Asn Ala Asn Leu Ile Gly Phe Glu Val Cys
               260                 265                 270
   Glu Ser Tyr Pro Gly Arg Ile Ser Asp Lys Leu Phe Leu Glu Asn Glu
           275                 280                 285
   Glu Ala Thr Leu Lys Val Ala Ala Asp Val Met Lys Ser Tyr Gly Leu
       290                 295                 300
   Pro Val Asn Arg Asn Thr Val Arg Leu His Asn Glu Phe Phe Gly Thr
   305                 310                 315                 320
   Ser Cys Pro His Arg Ser Trp Asp Leu His Val Gly Lys Gly Glu Pro
   325 330 335
   Tyr Thr Thr Thr Asn Ile Asn Lys Met Lys Asp Tyr Phe Ile Lys Arg
   340 345 350
   Ile Lys His Tyr Tyr Asp Gly Gly Lys Leu Glu Val Ser Lys Ala Ala
   355 360 365
   Thr Ile Lys Gln Ser Asp Val Lys Gln Glu Val Lys Lys Gln Glu Ala
   370 375 380
   Lys Gln Ile Val Lys Ala Thr Asp Trp Lys Gln Asn Lys Asp Gly Ile
   385 390 395 400
   Trp Tyr Lys Ala Glu His Ala Ser Phe Thr Val Thr Ala Pro Glu Gly
   405 410 415
   Ile Ile Thr Arg Tyr Lys Gly Pro Trp Thr Gly His Pro Gln Ala Gly
   420 425 430
   Val Leu Gln Lys Gly Gln Thr Ile Lys Tyr Asp Glu Val Gln Lys Phe
   435 440 445
   Asp Gly His Val Trp Val Ser Trp Glu Thr Phe Glu Gly Glu Thr Val
   450 455 460
   Tyr Met Pro Val Arg Thr Trp Asp Ala Lys Thr Gly Lys Val Gly Lys
   465 470 475 480
   Leu Trp Gly Glu Ile Lys Ser Gly Gly Leu Lys Lys Leu Gly Lys Lys
   485 490 495
   Leu Glu Gly Ala Gly Lys Arg Val Phe Asn Ala Ala Glu Lys Ala Leu
   500 505 510
   Pro Val Val Ala Gly Ala Lys Ala Leu Arg Lys
   515 520
<210> 91
   <211> 1572
   <212> DNA
   <213> artificial
<220>
   <223> Ply2638A-Cecropin A (A.aegypti)
<400> 91
   atgctaactg ctattgacta tcttacgaaa aaaggttgga aaatatcatc tgaccctcgc      60
   acttacgatg gttaccctaa aaactacggc tacagaaatt accatgaaaa cggcattaat     120
   tatgatgagt tttgtggtgg ttatcataga gcttttgatg tttacagtaa cgaaactaac     180
   gacgtgcctg ctgttactag cggaacagtt attgaagcaa acgattacgg taattttggt     240
   ggtacattcg ttattagaga cgctaacgat aacgattgga tatatgggca tctacaacgt     300
   ggctcaatgc gatttgttgt aggcgacaaa gtcaatcaag gtgacattat tggtttacaa     360
   ggtaatagca actattacga caatcctatg agtgtacatt tacatttaca attacgccct     420
   aaagacgcaa agaaagatga aaaatcacaa gtatgtagtg gtttggctat ggaaaaatat     480
   gacattacaa atttaaatgc taaacaagat aaatcaaaga atgggagcgt gaaagagttg     540
   aaacatatct attcaaacca tattaaaggt aacaagatta cagcaccaaa acctagtatt     600
   caaggtgtgg tcatccacaa tgattatggt agtatgacac ctagtcaata cttaccatgg     660
   ttatatgcac gtgagaataa cggtacacac gttaacggtt gggctagtgt ttatgcaaat     720
   agaaacgaag tgctttggta tcatccgaca gactacgtag agtggcattg tggtaatcaa     780
   tgggcaaatg ctaacttaat cggatttgaa gtgtgtgagt cgtatcctgg tagaatctcg     840
   gacaaattat tcttagaaaa tgaagaagcg acattgaaag tagctgcgga tgtgatgaag     900
   tcgtacggat taccagttaa tcgcaacact gtacgtctgc ataacgaatt cttcggaact     960
   tcttgtccac atcgttcgtg ggacttgcat gttggcaaag gtgagcctta cacaactact    1020
   aatattaata aaatgaaaga ctacttcatc aaacgcatca aacattatta tgacggtgga    1080
   aagctagaag taagcaaagc agcaactatc aaacaatctg acgttaagca agaagttaaa    1140
   aagcaagaag caaaacaaat tgtgaaagca acagattgga aacagaataa agatggcatt    1200
   tggtataaag ctgaacatgc ttcgttcaca gtgacagcac cagagggaat tatcacaaga    1260
   tacaaaggtc cttggactgg tcacccacaa gctggtgtat tacaaaaagg tcaaacgatt    1320
   aaatatgatg aggttcaaaa atttgacggt catgtttggg tatcgtggga aacgtttgag    1380
   ggcgaaactg tatacatgcc ggtacgcaca tgggacgcta aaactggtaa agttggtaag    1440
   ttgtggggcg aaattaaatc cggcggcctg aaaaaactgg gcaaaaaact ggaaggtgcc    1500
   ggcaaacgtg tgttcaacgc tgcagaaaaa gcactgccgg ttgtagctgg tgctaaagct    1560
   ctccgtaaat aa                                                        1572
<210> 92
   <211> 526
   <212> PRT
   <213> artificial
<220>
   <223> Ply2638A-Sarcotoxin IA
<400> 92
   Met Leu Thr Ala Ile Asp Tyr Leu Thr Lys Lys Gly Trp Lys Ile Ser
   1               5                   10                  15
   Ser Asp Pro Arg Thr Tyr Asp Gly Tyr Pro Lys Asn Tyr Gly Tyr Arg
               20                  25                  30
   Asn Tyr His Glu Asn Gly Ile Asn Tyr Asp Glu Phe Cys Gly Gly Tyr
           35                  40                  45
   His Arg Ala Phe Asp Val Tyr Ser Asn Glu Thr Asn Asp Val Pro Ala
       50                  55                  60
   Val Thr Ser Gly Thr Val Ile Glu Ala Asn Asp Tyr Gly Asn Phe Gly
   65                  70                  75                  80
   Gly Thr Phe Val Ile Arg Asp Ala Asn Asp Asn Asp Trp Ile Tyr Gly
                   85                  90                  95
   His Leu Gln Arg Gly Ser Met Arg Phe Val Val Gly Asp Lys Val Asn
               100                 105                 110
   Gln Gly Asp Ile Ile Gly Leu Gln Gly Asn Ser Asn Tyr Tyr Asp Asn
           115                 120                 125
   Pro Met Ser Val His Leu His Leu Gln Leu Arg Pro Lys Asp Ala Lys
       130                 135                 140
   Lys Asp Glu Lys Ser Gln Val Cys Ser Gly Leu Ala Met Glu Lys Tyr
   145                 150                 155                 160
   Asp Ile Thr Asn Leu Asn Ala Lys Gln Asp Lys Ser Lys Asn Gly Ser
                   165                 170                 175
   Val Lys Glu Leu Lys His Ile Tyr Ser Asn His Ile Lys Gly Asn Lys
               180                 185                 190
   Ile Thr Ala Pro Lys Pro Ser Ile Gln Gly Val Val Ile His Asn Asp
           195                 200                 205
   Tyr Gly Ser Met Thr Pro Ser Gln Tyr Leu Pro Trp Leu Tyr Ala Arg
       210                 215                 220
   Glu Asn Asn Gly Thr His Val Asn Gly Trp Ala Ser Val Tyr Ala Asn
   225                 230                 235                 240
   Arg Asn Glu Val Leu Trp Tyr His Pro Thr Asp Tyr Val Glu Trp His
                   245                 250                 255
   Cys Gly Asn Gln Trp Ala Asn Ala Asn Leu Ile Gly Phe Glu Val Cys
   260 265 270
   Glu Ser Tyr Pro Gly Arg Ile Ser Asp Lys Leu Phe Leu Glu Asn Glu
   275 280 285
   Glu Ala Thr Leu Lys Val Ala Ala Asp Val Met Lys Ser Tyr Gly Leu
   290 295 300
   Pro Val Asn Arg Asn Thr Val Arg Leu His Asn Glu Phe Phe Gly Thr
   305 310 315 320
   Ser Cys Pro His Arg Ser Trp Asp Leu His Val Gly Lys Gly Glu Pro
   325 330 335
   Tyr Thr Thr Thr Asn Ile Asn Lys Met Lys Asp Tyr Phe Ile Lys Arg
   340 345 350
   Ile Lys His Tyr Tyr Asp Gly Gly Lys Leu Glu Val Ser Lys Ala Ala
   355 360 365
   Thr Ile Lys Gln Ser Asp Val Lys Gln Glu Val Lys Lys Gln Glu Ala
   370 375 380
   Lys Gln Ile Val Lys Ala Thr Asp Trp Lys Gln Asn Lys Asp Gly Ile
   385 390 395 400
   Trp Tyr Lys Ala Glu His Ala Ser Phe Thr Val Thr Ala Pro Glu Gly
   405 410 415
   Ile Ile Thr Arg Tyr Lys Gly Pro Trp Thr Gly His Pro Gln Ala Gly
   420 425 430
   Val Leu Gln Lys Gly Gln Thr Ile Lys Tyr Asp Glu Val Gln Lys Phe
   435 440 445
   Asp Gly His Val Trp Val Ser Trp Glu Thr Phe Glu Gly Glu Thr Val
   450 455 460
   Tyr Met Pro Val Arg Thr Trp Asp Ala Lys Thr Gly Lys Val Gly Lys
   465 470 475 480
   Leu Trp Gly Glu Ile Lys Ser Gly Trp Leu Lys Lys Ile Gly Lys Lys
   485 490 495
   Ile Glu Arg Val Gly Gln His Thr Arg Asp Ala Thr Ile Gln Gly Leu
   500 505 510
   Gly Ile Ala Gln Gln Ala Ala Asn Val Ala Ala Thr Ala Arg
   515 520 525
<210> 93
   <211> 1581
   <212> DNA
   <213> artificial
<220>
   <223> Ply2638A-Sarcotoxin IA
<400> 93
   atgctaactg ctattgacta tcttacgaaa aaaggttgga aaatatcatc tgaccctcgc 60
   acttacgatg gttaccctaa aaactacggc tacagaaatt accatgaaaa cggcattaat 120
   tatgatgagt tttgtggtgg ttatcataga gcttttgatg tttacagtaa cgaaactaac 180
   gacgtgcctg ctgttactag cggaacagtt attgaagcaa acgattacgg taattttggt 240
   ggtacattcg ttattagaga cgctaacgat aacgattgga tatatgggca tctacaacgt 300
   ggctcaatgc gatttgttgt aggcgacaaa gtcaatcaag gtgacattat tggtttacaa 360
   ggtaatagca actattacga caatcctatg agtgtacatt tacatttaca attacgccct 420
   aaagacgcaa agaaagatga aaaatcacaa gtatgtagtg gtttggctat ggaaaaatat 480
   gacattacaa atttaaatgc taaacaagat aaatcaaaga atgggagcgt gaaagagttg 540
   aaacatatct attcaaacca tattaaaggt aacaagatta cagcaccaaa acctagtatt 600
   caaggtgtgg tcatccacaa tgattatggt agtatgacac ctagtcaata cttaccatgg 660
   ttatatgcac gtgagaataa cggtacacac gttaacggtt gggctagtgt ttatgcaaat 720
   agaaacgaag tgctttggta tcatccgaca gactacgtag agtggcattg tggtaatcaa 780
   tgggcaaatg ctaacttaat cggatttgaa gtgtgtgagt cgtatcctgg tagaatctcg 840
   gacaaattat tcttagaaaa tgaagaagcg acattgaaag tagctgcgga tgtgatgaag 900
   tcgtacggat taccagttaa tcgcaacact gtacgtctgc ataacgaatt cttcggaact 960
   tcttgtccac atcgttcgtg ggacttgcat gttggcaaag gtgagcctta cacaactact 1020
   aatattaata aaatgaaaga ctacttcatc aaacgcatca aacattatta tgacggtgga 1080
   aagctagaag taagcaaagc agcaactatc aaacaatctg acgttaagca agaagttaaa 1140
   aagcaagaag caaaacaaat tgtgaaagca acagattgga aacagaataa agatggcatt 1200
   tggtataaag ctgaacatgc ttcgttcaca gtgacagcac cagagggaat tatcacaaga 1260
   tacaaaggtc cttggactgg tcacccacaa gctggtgtat tacaaaaagg tcaaacgatt 1320
   aaatatgatg aggttcaaaa atttgacggt catgtttggg tatcgtggga aacgtttgag 1380
   ggcgaaactg tatacatgcc ggtacgcaca tgggacgcta aaactggtaa agttggtaag 1440
   ttgtggggcg aaattaaatc cggatggctc aaaaagattg gcaagaaaat cgagcgagtc 1500
   ggtcagcata cgcgtgatgc aactatccag ggtttaggta tcgcacagca agcagctaat 1560
   gtagcagcta ctgctcggta a 1581
<210> 94
   <211> 541
   <212> PRT
   <213> artificial
<220>
   <223> Ply2638A-OR-7
<400> 94
   Met Leu Thr Ala Ile Asp Tyr Leu Thr Lys Lys Gly Trp Lys Ile Ser
   1 5 10 15
   Ser Asp Pro Arg Thr Tyr Asp Gly Tyr Pro Lys Asn Tyr Gly Tyr Arg
   20 25 30
   Asn Tyr His Glu Asn Gly Ile Asn Tyr Asp Glu Phe Cys Gly Gly Tyr
   35 40 45
   His Arg Ala Phe Asp Val Tyr Ser Asn Glu Thr Asn Asp Val Pro Ala
   50 55 60
   Val Thr Ser Gly Thr Val Ile Glu Ala Asn Asp Tyr Gly Asn Phe Gly
   65 70 75 80
   Gly Thr Phe Val Ile Arg Asp Ala Asn Asp Asn Asp Trp Ile Tyr Gly
   85 90 95
   His Leu Gln Arg Gly Ser Met Arg Phe Val Val Gly Asp Lys Val Asn
   100 105 110
   Gln Gly Asp Ile Ile Gly Leu Gln Gly Asn Ser Asn Tyr Tyr Asp Asn
   115 120 125
   Pro Met Ser Val His Leu His Leu Gln Leu Arg Pro Lys Asp Ala Lys
   130 135 140
   Lys Asp Glu Lys Ser Gln Val Cys Ser Gly Leu Ala Met Glu Lys Tyr
   145 150 155 160
   Asp Ile Thr Asn Leu Asn Ala Lys Gln Asp Lys Ser Lys Asn Gly Ser
   165 170 175
   Val Lys Glu Leu Lys His Ile Tyr Ser Asn His Ile Lys Gly Asn Lys
   180 185 190
   Ile Thr Ala Pro Lys Pro Ser Ile Gln Gly Val Val Ile His Asn Asp
       195                 200                 205
   Tyr Gly Ser Met Thr Pro Ser Gln Tyr Leu Pro Trp Leu Tyr Ala Arg
       210                 215                 220
   Glu Asn Asn Gly Thr His Val Asn Gly Trp Ala Ser Val Tyr Ala Asn
   225                 230                 235                 240
   Arg Asn Glu Val Leu Trp Tyr His Pro Thr Asp Tyr Val Glu Trp His
                   245                 250                 255
   Cys Gly Asn Gln Trp Ala Asn Ala Asn Leu Ile Gly Phe Glu Val Cys
               260                 265                 270
   Glu Ser Tyr Pro Gly Arg Ile Ser Asp Lys Leu Phe Leu Glu Asn Glu
           275                 280                 285
   Glu Ala Thr Leu Lys Val Ala Ala Asp Val Met Lys Ser Tyr Gly Leu
       290                 295                 300
   Pro Val Asn Arg Asn Thr Val Arg Leu His Asn Glu Phe Phe Gly Thr
   305                 310                 315                 320
   Ser Cys Pro His Arg Ser Trp Asp Leu His Val Gly Lys Gly Glu Pro
                   325                 330                 335
   Tyr Thr Thr Thr Asn Ile Asn Lys Met Lys Asp Tyr Phe Ile Lys Arg
               340                 345                 350
   Ile Lys His Tyr Tyr Asp Gly Gly Lys Leu Glu Val Ser Lys Ala Ala
           355                 360                 365
   Thr Ile Lys Gln Ser Asp Val Lys Gln Glu Val Lys Lys Gln Glu Ala
       370                 375                 380
   Lys Gln Ile Val Lys Ala Thr Asp Trp Lys Gln Asn Lys Asp Gly Ile
   385                 390                 395                 400
   Trp Tyr Lys Ala Glu His Ala Ser Phe Thr Val Thr Ala Pro Glu Gly
                   405                 410                 415
   Ile Ile Thr Arg Tyr Lys Gly Pro Trp Thr Gly His Pro Gln Ala Gly
               420                 425                 430
   Val Leu Gln Lys Gly Gln Thr Ile Lys Tyr Asp Glu Val Gln Lys Phe
                   435                 440                 445
   Asp Gly His Val Trp Val Ser Trp Glu Thr Phe Glu Gly Glu Thr Val
       450                 455                 460
   Tyr Met Pro Val Arg Thr Trp Asp Ala Lys Thr Gly Lys Val Gly Lys
   465                 470                 475                 480
   Leu Trp Gly Glu Ile Lys Ser Lys Thr Tyr Tyr Gly Thr Asn Gly Val
                   485                 490                 495
   His Cys Thr Lys Asn Ser Leu Trp Gly Lys Val Arg Leu Lys Asn Met
               500                 505                 510
   Lys Tyr Asp Gln Asn Thr Thr Tyr Met Gly Arg Leu Gln Asp Ile Leu
           515                 520                 525
   Leu Gly Trp Ala Thr Gly Ala Phe Gly Lys Thr Phe His
       530                 535                 540
<210> 95
   <211> 1626
   <212> DNA
   <213> artificial
<220>
   <223> Ply2638A-OR-7
<400> 95
   atgctaactg ctattgacta tcttacgaaa aaaggttgga aaatatcatc tgaccctcgc 60
   acttacgatg gttaccctaa aaactacggc tacagaaatt accatgaaaa cggcattaat 120
   tatgatgagt tttgtggtgg ttatcataga gcttttgatg tttacagtaa cgaaactaac 180
   gacgtgcctg ctgttactag cggaacagtt attgaagcaa acgattacgg taattttggt 240
   ggtacattcg ttattagaga cgctaacgat aacgattgga tatatgggca tctacaacgt 300
   ggctcaatgc gatttgttgt aggcgacaaa gtcaatcaag gtgacattat tggtttacaa 360
   ggtaatagca actattacga caatcctatg agtgtacatt tacatttaca attacgccct 420
   aaagacgcaa agaaagatga aaaatcacaa gtatgtagtg gtttggctat ggaaaaatat 480
   gacattacaa atttaaatgc taaacaagat aaatcaaaga atgggagcgt gaaagagttg 540
   aaacatatct attcaaacca tattaaaggt aacaagatta cagcaccaaa acctagtatt 600
   caaggtgtgg tcatccacaa tgattatggt agtatgacac ctagtcaata cttaccatgg 660
   ttatatgcac gtgagaataa cggtacacac gttaacggtt gggctagtgt ttatgcaaat 720
   agaaacgaag tgctttggta tcatccgaca gactacgtag agtggcattg tggtaatcaa 780
   tgggcaaatg ctaacttaat cggatttgaa gtgtgtgagt cgtatcctgg tagaatctcg 840
   gacaaattat tcttagaaaa tgaagaagcg acattgaaag tagctgcgga tgtgatgaag 900
   tcgtacggat taccagttaa tcgcaacact gtacgtctgc ataacgaatt cttcggaact 960
   tcttgtccac atcgttcgtg ggacttgcat gttggcaaag gtgagcctta cacaactact 1020
   aatattaata aaatgaaaga ctacttcatc aaacgcatca aacattatta tgacggtgga 1080
   aagctagaag taagcaaagc agcaactatc aaacaatctg acgttaagca agaagttaaa 1140
   aagcaagaag caaaacaaat tgtgaaagca acagattgga aacagaataa agatggcatt 1200
   tggtataaag ctgaacatgc ttcgttcaca gtgacagcac cagagggaat tatcacaaga 1260
   tacaaaggtc cttggactgg tcacccacaa gctggtgtat tacaaaaagg tcaaacgatt 1320
   aaatatgatg aggttcaaaa atttgacggt catgtttggg tatcgtggga aacgtttgag 1380
   ggcgaaactg tatacatgcc ggtacgcaca tgggacgcta aaactggtaa agttggtaag 1440
   ttgtggggcg aaattaaatc caaaacctac tatggcacca acggcgtaca ctgcaccaaa 1500
   aacagcctgt ggggtaaagt gcgtctgaaa aacatgaaat acgaccagaa caccacgtat 1560
   atgggccgtc tccaggatat cctgctgggt tgggcaactg gtgccttcgg caaaaccttt 1620
   cactaa 1626
<210> 96
   <211> 500
   <212> PRT
   <213> artificial
<220>
   <223> Ply2638A-Indolicidin
<400> 96
   Met Leu Thr Ala Ile Asp Tyr Leu Thr Lys Lys Gly Trp Lys Ile Ser
   1               5                   10                  15
   Ser Asp Pro Arg Thr Tyr Asp Gly Tyr Pro Lys Asn Tyr Gly Tyr Arg
               20                  25                  30
   Asn Tyr His Glu Asn Gly Ile Asn Tyr Asp Glu Phe Cys Gly Gly Tyr
           35                  40                  45
   His Arg Ala Phe Asp Val Tyr Ser Asn Glu Thr Asn Asp Val Pro Ala
       50                  55                  60
   Val Thr Ser Gly Thr Val Ile Glu Ala Asn Asp Tyr Gly Asn Phe Gly
   65                  70                  75                  80
   Gly Thr Phe Val Ile Arg Asp Ala Asn Asp Asn Asp Trp Ile Tyr Gly
                   85                  90                  95
   His Leu Gln Arg Gly Ser Met Arg Phe Val Val Gly Asp Lys Val Asn
   100 105 110
   Gln Gly Asp Ile Ile Gly Leu Gln Gly Asn Ser Asn Tyr Tyr Asp Asn
   115 120 125
   Pro Met Ser Val His Leu His Leu Gln Leu Arg Pro Lys Asp Ala Lys
   130 135 140
   Lys Asp Glu Lys Ser Gln Val Cys Ser Gly Leu Ala Met Glu Lys Tyr
   145 150 155 160
   Asp Ile Thr Asn Leu Asn Ala Lys Gln Asp Lys Ser Lys Asn Gly Ser
   165 170 175
   Val Lys Glu Leu Lys His Ile Tyr Ser Asn His Ile Lys Gly Asn Lys
   180 185 190
   Ile Thr Ala Pro Lys Pro Ser Ile Gln Gly Val Val Ile His Asn Asp
   195 200 205
   Tyr Gly Ser Met Thr Pro Ser Gln Tyr Leu Pro Trp Leu Tyr Ala Arg
   210 215 220
   Glu Asn Asn Gly Thr His Val Asn Gly Trp Ala Ser Val Tyr Ala Asn
   225 230 235 240
   Arg Asn Glu Val Leu Trp Tyr His Pro Thr Asp Tyr Val Glu Trp His
   245 250 255
   Cys Gly Asn Gln Trp Ala Asn Ala Asn Leu Ile Gly Phe Glu Val Cys
   260 265 270
   Glu Ser Tyr Pro Gly Arg Ile Ser Asp Lys Leu Phe Leu Glu Asn Glu
   275 280 285
   Glu Ala Thr Leu Lys Val Ala Ala Asp Val Met Lys Ser Tyr Gly Leu
   290 295 300
   Pro Val Asn Arg Asn Thr Val Arg Leu His Asn Glu Phe Phe Gly Thr
   305 310 315 320
   Ser Cys Pro His Arg Ser Trp Asp Leu His Val Gly Lys Gly Glu Pro
   325 330 335
   Tyr Thr Thr Thr Asn Ile Asn Lys Met Lys Asp Tyr Phe Ile Lys Arg
   340 345 350
   Ile Lys His Tyr Tyr Asp Gly Gly Lys Leu Glu Val Ser Lys Ala Ala
   355 360 365
   Thr Ile Lys Gln Ser Asp Val Lys Gln Glu Val Lys Lys Gln Glu Ala
   370 375 380
   Lys Gln Ile Val Lys Ala Thr Asp Trp Lys Gln Asn Lys Asp Gly Ile
   385 390 395 400
   Trp Tyr Lys Ala Glu His Ala Ser Phe Thr Val Thr Ala Pro Glu Gly
   405 410 415
   Ile Ile Thr Arg Tyr Lys Gly Pro Trp Thr Gly His Pro Gln Ala Gly
   420 425 430
   Val Leu Gln Lys Gly Gln Thr Ile Lys Tyr Asp Glu Val Gln Lys Phe
   435 440 445
   Asp Gly His Val Trp Val Ser Trp Glu Thr Phe Glu Gly Glu Thr Val
   450 455 460
   Tyr Met Pro Val Arg Thr Trp Asp Ala Lys Thr Gly Lys Val Gly Lys
   465 470 475 480
   Leu Trp Gly Glu Ile Lys Ser Ile Leu Pro Trp Lys Trp Pro Trp Trp
   485 490 495
   Pro Trp Arg Arg
   500
<210> 97
   <211> 1503
   <212> DNA
   <213> artificial
<220>
   <223> Ply2638A-Indolicidin
<400> 97
   atgctaactg ctattgacta tcttacgaaa aaaggttgga aaatatcatc tgaccctcgc 60
   acttacgatg gttaccctaa aaactacggc tacagaaatt accatgaaaa cggcattaat 120
   tatgatgagt tttgtggtgg ttatcataga gcttttgatg tttacagtaa cgaaactaac 180
   gacgtgcctg ctgttactag cggaacagtt attgaagcaa acgattacgg taattttggt 240
   ggtacattcg ttattagaga cgctaacgat aacgattgga tatatgggca tctacaacgt 300
   ggctcaatgc gatttgttgt aggcgacaaa gtcaatcaag gtgacattat tggtttacaa 360
   ggtaatagca actattacga caatcctatg agtgtacatt tacatttaca attacgccct 420
   aaagacgcaa agaaagatga aaaatcacaa gtatgtagtg gtttggctat ggaaaaatat 480
   gacattacaa atttaaatgc taaacaagat aaatcaaaga atgggagcgt gaaagagttg 540
   aaacatatct attcaaacca tattaaaggt aacaagatta cagcaccaaa acctagtatt 600
   caaggtgtgg tcatccacaa tgattatggt agtatgacac ctagtcaata cttaccatgg 660
   ttatatgcac gtgagaataa cggtacacac gttaacggtt gggctagtgt ttatgcaaat 720
   agaaacgaag tgctttggta tcatccgaca gactacgtag agtggcattg tggtaatcaa 780
   tgggcaaatg ctaacttaat cggatttgaa gtgtgtgagt cgtatcctgg tagaatctcg 840
   gacaaattat tcttagaaaa tgaagaagcg acattgaaag tagctgcgga tgtgatgaag 900
   tcgtacggat taccagttaa tcgcaacact gtacgtctgc ataacgaatt cttcggaact 960
   tcttgtccac atcgttcgtg ggacttgcat gttggcaaag gtgagcctta cacaactact 1020
   aatattaata aaatgaaaga ctacttcatc aaacgcatca aacattatta tgacggtgga 1080
   aagctagaag taagcaaagc agcaactatc aaacaatctg acgttaagca agaagttaaa 1140
   aagcaagaag caaaacaaat tgtgaaagca acagattgga aacagaataa agatggcatt 1200
   tggtataaag ctgaacatgc ttcgttcaca gtgacagcac cagagggaat tatcacaaga 1260
   tacaaaggtc cttggactgg tcacccacaa gctggtgtat tacaaaaagg tcaaacgatt 1320
   aaatatgatg aggttcaaaa atttgacggt catgtttggg tatcgtggga aacgtttgag 1380
   ggcgaaactg tatacatgcc ggtacgcaca tgggacgcta aaactggtaa agttggtaag 1440
   ttgtggggcg aaattaaatc catcctgccg tggaaatggc cgtggtggcc atggcgtcgc 1500
   taa 1503
<210> 98
   <211> 511
   <212> PRT
   <213> artificial
<220>
   <223> Ply2638A-Pseudin1
<400> 98
   Met Leu Thr Ala Ile Asp Tyr Leu Thr Lys Lys Gly Trp Lys Ile Ser
   1 5 10 15
   Ser Asp Pro Arg Thr Tyr Asp Gly Tyr Pro Lys Asn Tyr Gly Tyr Arg
   20 25 30
   Asn Tyr His Glu Asn Gly Ile Asn Tyr Asp Glu Phe Cys Gly Gly Tyr
   35 40 45
   His Arg Ala Phe Asp Val Tyr Ser Asn Glu Thr Asn Asp Val Pro Ala
       50                  55                  60
   Val Thr Ser Gly Thr Val Ile Glu Ala Asn Asp Tyr Gly Asn Phe Gly
   65                  70                  75                  80
   Gly Thr Phe Val Ile Arg Asp Ala Asn Asp Asn Asp Trp Ile Tyr Gly
                   85                  90                  95
   His Leu Gln Arg Gly Ser Met Arg Phe Val Val Gly Asp Lys Val Asn
               100                 105                 110
   Gln Gly Asp Ile Ile Gly Leu Gln Gly Asn Ser Asn Tyr Tyr Asp Asn
           115                 120                 125
   Pro Met Ser Val His Leu His Leu Gln Leu Arg Pro Lys Asp Ala Lys
       130                 135                 140
   Lys Asp Glu Lys Ser Gln Val Cys Ser Gly Leu Ala Met Glu Lys Tyr
   145                 150                 155                 160
   Asp Ile Thr Asn Leu Asn Ala Lys Gln Asp Lys Ser Lys Asn Gly Ser
                   165                 170                 175
   Val Lys Glu Leu Lys His Ile Tyr Ser Asn His Ile Lys Gly Asn Lys
               180                 185                 190
   Ile Thr Ala Pro Lys Pro Ser Ile Gln Gly Val Val Ile His Asn Asp
           195                 200                 205
   Tyr Gly Ser Met Thr Pro Ser Gln Tyr Leu Pro Trp Leu Tyr Ala Arg
       210                 215                 220
   Glu Asn Asn Gly Thr His Val Asn Gly Trp Ala Ser Val Tyr Ala Asn
   225                 230                 235                 240
   Arg Asn Glu Val Leu Trp Tyr His Pro Thr Asp Tyr Val Glu Trp His
                   245                 250                 255
   Cys Gly Asn Gln Trp Ala Asn Ala Asn Leu Ile Gly Phe Glu Val Cys
               260                 265                 270
   Glu Ser Tyr Pro Gly Arg Ile Ser Asp Lys Leu Phe Leu Glu Asn Glu
           275                 280                 285
   Glu Ala Thr Leu Lys Val Ala Ala Asp Val Met Lys Ser Tyr Gly Leu
       290                 295                 300
   Pro Val Asn Arg Asn Thr Val Arg Leu His Asn Glu Phe Phe Gly Thr
   305 310 315 320
   Ser Cys Pro His Arg Ser Trp Asp Leu His Val Gly Lys Gly Glu Pro
   325 330 335
   Tyr Thr Thr Thr Asn Ile Asn Lys Met Lys Asp Tyr Phe Ile Lys Arg
   340 345 350
   Ile Lys His Tyr Tyr Asp Gly Gly Lys Leu Glu Val Ser Lys Ala Ala
   355 360 365
   Thr Ile Lys Gln Ser Asp Val Lys Gln Glu Val Lys Lys Gln Glu Ala
   370 375 380
   Lys Gln Ile Val Lys Ala Thr Asp Trp Lys Gln Asn Lys Asp Gly Ile
   385 390 395 400
   Trp Tyr Lys Ala Glu His Ala Ser Phe Thr Val Thr Ala Pro Glu Gly
   405 410 415
   Ile Ile Thr Arg Tyr Lys Gly Pro Trp Thr Gly His Pro Gln Ala Gly
   420 425 430
   Val Leu Gln Lys Gly Gln Thr Ile Lys Tyr Asp Glu Val Gln Lys Phe
   435 440 445
   Asp Gly His Val Trp Val Ser Trp Glu Thr Phe Glu Gly Glu Thr Val
   450 455 460
   Tyr Met Pro Val Arg Thr Trp Asp Ala Lys Thr Gly Lys Val Gly Lys
   465 470 475 480
   Leu Trp Gly Glu Ile Lys Ser Gly Leu Asn Thr Leu Lys Lys Val Phe
   485 490 495
   Gln Gly Leu His Glu Ala Ile Lys Leu Ile Asn Asn His Val Gln
   500 505 510
<210> 99
   <211> 1536
   <212> DNA
   <213> artificial
<220>
   <223> Ply2638A-Pseudin1
<400> 99
   atgctaactg ctattgacta tcttacgaaa aaaggttgga aaatatcatc tgaccctcgc 60
   acttacgatg gttaccctaa aaactacggc tacagaaatt accatgaaaa cggcattaat 120
   tatgatgagt tttgtggtgg ttatcataga gcttttgatg tttacagtaa cgaaactaac 180
   gacgtgcctg ctgttactag cggaacagtt attgaagcaa acgattacgg taattttggt 240
   ggtacattcg ttattagaga cgctaacgat aacgattgga tatatgggca tctacaacgt 300
   ggctcaatgc gatttgttgt aggcgacaaa gtcaatcaag gtgacattat tggtttacaa 360
   ggtaatagca actattacga caatcctatg agtgtacatt tacatttaca attacgccct 420
   aaagacgcaa agaaagatga aaaatcacaa gtatgtagtg gtttggctat ggaaaaatat 480
   gacattacaa atttaaatgc taaacaagat aaatcaaaga atgggagcgt gaaagagttg 540
   aaacatatct attcaaacca tattaaaggt aacaagatta cagcaccaaa acctagtatt 600
   caaggtgtgg tcatccacaa tgattatggt agtatgacac ctagtcaata cttaccatgg 660
   ttatatgcac gtgagaataa cggtacacac gttaacggtt gggctagtgt ttatgcaaat 720
   agaaacgaag tgctttggta tcatccgaca gactacgtag agtggcattg tggtaatcaa 780
   tgggcaaatg ctaacttaat cggatttgaa gtgtgtgagt cgtatcctgg tagaatctcg 840
   gacaaattat tcttagaaaa tgaagaagcg acattgaaag tagctgcgga tgtgatgaag 900
   tcgtacggat taccagttaa tcgcaacact gtacgtctgc ataacgaatt cttcggaact 960
   tcttgtccac atcgttcgtg ggacttgcat gttggcaaag gtgagcctta cacaactact 1020
   aatattaata aaatgaaaga ctacttcatc aaacgcatca aacattatta tgacggtgga 1080
   aagctagaag taagcaaagc agcaactatc aaacaatctg acgttaagca agaagttaaa 1140
   aagcaagaag caaaacaaat tgtgaaagca acagattgga aacagaataa agatggcatt 1200
   tggtataaag ctgaacatgc ttcgttcaca gtgacagcac cagagggaat tatcacaaga 1260
   tacaaaggtc cttggactgg tcacccacaa gctggtgtat tacaaaaagg tcaaacgatt 1320
   aaatatgatg aggttcaaaa atttgacggt catgtttggg tatcgtggga aacgtttgag 1380
   ggcgaaactg tatacatgcc ggtacgcaca tgggacgcta aaactggtaa agttggtaag 1440
   ttgtggggcg aaattaaatc cggtctgaac accctgaaaa aagtattcca gggtctgcat 1500
   gaagcgatta aactgatcaa caaccacgtt cagtaa 1536
<210> 100
   <211> 510
   <212> PRT
   <213> artificial
<220>
   <223> Ply2638A-Magainin
<400> 100
   Met Leu Thr Ala Ile Asp Tyr Leu Thr Lys Lys Gly Trp Lys Ile Ser
   1 5 10 15
   Ser Asp Pro Arg Thr Tyr Asp Gly Tyr Pro Lys Asn Tyr Gly Tyr Arg
   20 25 30
   Asn Tyr His Glu Asn Gly Ile Asn Tyr Asp Glu Phe Cys Gly Gly Tyr
   35 40 45
   His Arg Ala Phe Asp Val Tyr Ser Asn Glu Thr Asn Asp Val Pro Ala
   50 55 60
   Val Thr Ser Gly Thr Val Ile Glu Ala Asn Asp Tyr Gly Asn Phe Gly
   65 70 75 80
   Gly Thr Phe Val Ile Arg Asp Ala Asn Asp Asn Asp Trp Ile Tyr Gly
   85 90 95
   His Leu Gln Arg Gly Ser Met Arg Phe Val Val Gly Asp Lys Val Asn
   100 105 110
   Gln Gly Asp Ile Ile Gly Leu Gln Gly Asn Ser Asn Tyr Tyr Asp Asn
   115 120 125
   Pro Met Ser Val His Leu His Leu Gln Leu Arg Pro Lys Asp Ala Lys
   130 135 140
   Lys Asp Glu Lys Ser Gln Val Cys Ser Gly Leu Ala Met Glu Lys Tyr
   145 150 155 160
   Asp Ile Thr Asn Leu Asn Ala Lys Gln Asp Lys Ser Lys Asn Gly Ser
   165 170 175
   Val Lys Glu Leu Lys His Ile Tyr Ser Asn His Ile Lys Gly Asn Lys
   180 185 190
   Ile Thr Ala Pro Lys Pro Ser Ile Gln Gly Val Val Ile His Asn Asp
   195 200 205
   Tyr Gly Ser Met Thr Pro Ser Gln Tyr Leu Pro Trp Leu Tyr Ala Arg
   210 215 220
   Glu Asn Asn Gly Thr His Val Asn Gly Trp Ala Ser Val Tyr Ala Asn
   225 230 235 240
   Arg Asn Glu Val Leu Trp Tyr His Pro Thr Asp Tyr Val Glu Trp His
   245 250 255
   Cys Gly Asn Gln Trp Ala Asn Ala Asn Leu Ile Gly Phe Glu Val Cys
   260 265 270
   Glu Ser Tyr Pro Gly Arg Ile Ser Asp Lys Leu Phe Leu Glu Asn Glu
   275 280 285
   Glu Ala Thr Leu Lys Val Ala Ala Asp Val Met Lys Ser Tyr Gly Leu
   290 295 300
   Pro Val Asn Arg Asn Thr Val Arg Leu His Asn Glu Phe Phe Gly Thr
   305 310 315 320
   Ser Cys Pro His Arg Ser Trp Asp Leu His Val Gly Lys Gly Glu Pro
   325 330 335
   Tyr Thr Thr Thr Asn Ile Asn Lys Met Lys Asp Tyr Phe Ile Lys Arg
   340 345 350
   Ile Lys His Tyr Tyr Asp Gly Gly Lys Leu Glu Val Ser Lys Ala Ala
   355 360 365
   Thr Ile Lys Gln Ser Asp Val Lys Gln Glu Val Lys Lys Gln Glu Ala
   370 375 380
   Lys Gln Ile Val Lys Ala Thr Asp Trp Lys Gln Asn Lys Asp Gly Ile
   385 390 395 400
   Trp Tyr Lys Ala Glu His Ala Ser Phe Thr Val Thr Ala Pro Glu Gly
   405 410 415
   Ile Ile Thr Arg Tyr Lys Gly Pro Trp Thr Gly His Pro Gln Ala Gly
   420 425 430
   Val Leu Gln Lys Gly Gln Thr Ile Lys Tyr Asp Glu Val Gln Lys Phe
   435 440 445
   Asp Gly His Val Trp Val Ser Trp Glu Thr Phe Glu Gly Glu Thr Val
   450 455 460
   Tyr Met Pro Val Arg Thr Trp Asp Ala Lys Thr Gly Lys Val Gly Lys
   465 470 475 480
   Leu Trp Gly Glu Ile Lys Ser Gly Ile Gly Lys Phe Leu His Ser Ala
   485 490 495
   Lys Lys Phe Gly Lys Ala Phe Val Gly Glu Ile Met Asn Ser
   500 505 510
<210> 101
   <211> 1533
   <212> DNA
   <213> artificial
<220>
   <223> Ply2638A-Magainin
<400> 101
   atgctaactg ctattgacta tcttacgaaa aaaggttgga aaatatcatc tgaccctcgc     60
   acttacgatg gttaccctaa aaactacggc tacagaaatt accatgaaaa cggcattaat    120
   tatgatgagt tttgtggtgg ttatcataga gcttttgatg tttacagtaa cgaaactaac    180
   gacgtgcctg ctgttactag cggaacagtt attgaagcaa acgattacgg taattttggt    240
   ggtacattcg ttattagaga cgctaacgat aacgattgga tatatgggca tctacaacgt    300
   ggctcaatgc gatttgttgt aggcgacaaa gtcaatcaag gtgacattat tggtttacaa    360
   ggtaatagca actattacga caatcctatg agtgtacatt tacatttaca attacgccct    420
   aaagacgcaa agaaagatga aaaatcacaa gtatgtagtg gtttggctat ggaaaaatat    480
   gacattacaa atttaaatgc taaacaagat aaatcaaaga atgggagcgt gaaagagttg    540
   aaacatatct attcaaacca tattaaaggt aacaagatta cagcaccaaa acctagtatt    600
   caaggtgtgg tcatccacaa tgattatggt agtatgacac ctagtcaata cttaccatgg    660
   ttatatgcac gtgagaataa cggtacacac gttaacggtt gggctagtgt ttatgcaaat    720
   agaaacgaag tgctttggta tcatccgaca gactacgtag agtggcattg tggtaatcaa    780
   tgggcaaatg ctaacttaat cggatttgaa gtgtgtgagt cgtatcctgg tagaatctcg    840
   gacaaattat tcttagaaaa tgaagaagcg acattgaaag tagctgcgga tgtgatgaag    900
   tcgtacggat taccagttaa tcgcaacact gtacgtctgc ataacgaatt cttcggaact    960
   tcttgtccac atcgttcgtg ggacttgcat gttggcaaag gtgagcctta cacaactact   1020
   aatattaata aaatgaaaga ctacttcatc aaacgcatca aacattatta tgacggtgga   1080
   aagctagaag taagcaaagc agcaactatc aaacaatctg acgttaagca agaagttaaa   1140
   aagcaagaag caaaacaaat tgtgaaagca acagattgga aacagaataa agatggcatt   1200
   tggtataaag ctgaacatgc ttcgttcaca gtgacagcac cagagggaat tatcacaaga   1260
   tacaaaggtc cttggactgg tcacccacaa gctggtgtat tacaaaaagg tcaaacgatt   1320
   aaatatgatg aggttcaaaa atttgacggt catgtttggg tatcgtggga aacgtttgag   1380
   ggcgaaactg tatacatgcc ggtacgcaca tgggacgcta aaactggtaa agttggtaag   1440
   ttgtggggcg aaattaaatc cggtatcggc aaattcctgc actccgcaaa aaaattcggc   1500
   aaagctttcg tgggcgaaat tatgaactct taa                                1533
<210> 102
   <211> 512
   <212> PRT
   <213> artificial
<220>
   <223> Ply2638A-Pleurocidin
<400> 102
   Met Leu Thr Ala Ile Asp Tyr Leu Thr Lys Lys Gly Trp Lys Ile Ser
   1               5                   10                  15
   Ser Asp Pro Arg Thr Tyr Asp Gly Tyr Pro Lys Asn Tyr Gly Tyr Arg
               20                  25                  30
   Asn Tyr His Glu Asn Gly Ile Asn Tyr Asp Glu Phe Cys Gly Gly Tyr
           35                  40                  45
   His Arg Ala Phe Asp Val Tyr Ser Asn Glu Thr Asn Asp Val Pro Ala
       50                  55                  60
   Val Thr Ser Gly Thr Val Ile Glu Ala Asn Asp Tyr Gly Asn Phe Gly
   65                  70                  75                  80
   Gly Thr Phe Val Ile Arg Asp Ala Asn Asp Asn Asp Trp Ile Tyr Gly
                   85                  90                  95
   His Leu Gln Arg Gly Ser Met Arg Phe Val Val Gly Asp Lys Val Asn
               100                 105                 110
   Gln Gly Asp Ile Ile Gly Leu Gln Gly Asn Ser Asn Tyr Tyr Asp Asn
           115                 120                 125
   Pro Met Ser Val His Leu His Leu Gln Leu Arg Pro Lys Asp Ala Lys
       130                 135                 140
   Lys Asp Glu Lys Ser Gln Val Cys Ser Gly Leu Ala Met Glu Lys Tyr
   145                 150                 155                 160
   Asp Ile Thr Asn Leu Asn Ala Lys Gln Asp Lys Ser Lys Asn Gly Ser
                   165                 170                 175
   Val Lys Glu Leu Lys His Ile Tyr Ser Asn His Ile Lys Gly Asn Lys
               180                 185                 190
   Ile Thr Ala Pro Lys Pro Ser Ile Gln Gly Val Val Ile His Asn Asp
           195                 200                 205
   Tyr Gly Ser Met Thr Pro Ser Gln Tyr Leu Pro Trp Leu Tyr Ala Arg
   210 215 220
   Glu Asn Asn Gly Thr His Val Asn Gly Trp Ala Ser Val Tyr Ala Asn
   225 230 235 240
   Arg Asn Glu Val Leu Trp Tyr His Pro Thr Asp Tyr Val Glu Trp His
   245 250 255
   Cys Gly Asn Gln Trp Ala Asn Ala Asn Leu Ile Gly Phe Glu Val Cys
   260 265 270
   Glu Ser Tyr Pro Gly Arg Ile Ser Asp Lys Leu Phe Leu Glu Asn Glu
   275 280 285
   Glu Ala Thr Leu Lys Val Ala Ala Asp Val Met Lys Ser Tyr Gly Leu
   290 295 300
   Pro Val Asn Arg Asn Thr Val Arg Leu His Asn Glu Phe Phe Gly Thr
   305 310 315 320
   Ser Cys Pro His Arg Ser Trp Asp Leu His Val Gly Lys Gly Glu Pro
   325 330 335
   Tyr Thr Thr Thr Asn Ile Asn Lys Met Lys Asp Tyr Phe Ile Lys Arg
   340 345 350
   Ile Lys His Tyr Tyr Asp Gly Gly Lys Leu Glu Val Ser Lys Ala Ala
   355 360 365
   Thr Ile Lys Gln Ser Asp Val Lys Gln Glu Val Lys Lys Gln Glu Ala
   370 375 380
   Lys Gln Ile Val Lys Ala Thr Asp Trp Lys Gln Asn Lys Asp Gly Ile
   385 390 395 400
   Trp Tyr Lys Ala Glu His Ala Ser Phe Thr Val Thr Ala Pro Glu Gly
   405 410 415
   Ile Ile Thr Arg Tyr Lys Gly Pro Trp Thr Gly His Pro Gln Ala Gly
   420 425 430
   Val Leu Gln Lys Gly Gln Thr Ile Lys Tyr Asp Glu Val Gln Lys Phe
   435 440 445
   Asp Gly His Val Trp Val Ser Trp Glu Thr Phe Glu Gly Glu Thr Val
   450 455 460
   Tyr Met Pro Val Arg Thr Trp Asp Ala Lys Thr Gly Lys Val Gly Lys
   465 470 475 480
   Leu Trp Gly Glu Ile Lys Ser Gly Trp Gly Ser Phe Phe Lys Lys Ala
   485 490 495
   Ala His Val Gly Lys His Val Gly Lys Ala Ala Leu Thr His Tyr Leu
   500 505 510
<210> 103
   <211> 1539
   <212> DNA
   <213> artificial
<220>
   <223> Ply2638A-Pleurocidin
<400> 103
   atgctaactg ctattgacta tcttacgaaa aaaggttgga aaatatcatc tgaccctcgc    60
   acttacgatg gttaccctaa aaactacggc tacagaaatt accatgaaaa cggcattaat   120
   tatgatgagt tttgtggtgg ttatcataga gcttttgatg tttacagtaa cgaaactaac   180
   gacgtgcctg ctgttactag cggaacagtt attgaagcaa acgattacgg taattttggt   240
   ggtacattcg ttattagaga cgctaacgat aacgattgga tatatgggca tctacaacgt   300
   ggctcaatgc gatttgttgt aggcgacaaa gtcaatcaag gtgacattat tggtttacaa   360
   ggtaatagca actattacga caatcctatg agtgtacatt tacatttaca attacgccct   420
   aaagacgcaa agaaagatga aaaatcacaa gtatgtagtg gtttggctat ggaaaaatat   480
   gacattacaa atttaaatgc taaacaagat aaatcaaaga atgggagcgt gaaagagttg   540
   aaacatatct attcaaacca tattaaaggt aacaagatta cagcaccaaa acctagtatt   600
   caaggtgtgg tcatccacaa tgattatggt agtatgacac ctagtcaata cttaccatgg   660
   ttatatgcac gtgagaataa cggtacacac gttaacggtt gggctagtgt ttatgcaaat   720
   agaaacgaag tgctttggta tcatccgaca gactacgtag agtggcattg tggtaatcaa   780
   tgggcaaatg ctaacttaat cggatttgaa gtgtgtgagt cgtatcctgg tagaatctcg   840
   gacaaattat tcttagaaaa tgaagaagcg acattgaaag tagctgcgga tgtgatgaag   900
   tcgtacggat taccagttaa tcgcaacact gtacgtctgc ataacgaatt cttcggaact   960
   tcttgtccac atcgttcgtg ggacttgcat gttggcaaag gtgagcctta cacaactact  1020
   aatattaata aaatgaaaga ctacttcatc aaacgcatca aacattatta tgacggtgga  1080
   aagctagaag taagcaaagc agcaactatc aaacaatctg acgttaagca agaagttaaa  1140
   aagcaagaag caaaacaaat tgtgaaagca acagattgga aacagaataa agatggcatt  1200
   tggtataaag ctgaacatgc ttcgttcaca gtgacagcac cagagggaat tatcacaaga  1260
   tacaaaggtc cttggactgg tcacccacaa gctggtgtat tacaaaaagg tcaaacgatt  1320
   aaatatgatg aggttcaaaa atttgacggt catgtttggg tatcgtggga aacgtttgag  1380
   ggcgaaactg tatacatgcc ggtacgcaca tgggacgcta aaactggtaa agttggtaag  1440
   ttgtggggcg aaattaaatc cggctggggt tctttcttta aaaaagcggc tcacgttggc  1500
   aaacatgtag gtaaagcagc tctgacccac tatctgtaa                         1539
<210> 104
   <211> 508
   <212> PRT
   <213> artificial
<220>
   <223> Ply2638A-BuforinII
<400> 104
   Met Leu Thr Ala Ile Asp Tyr Leu Thr Lys Lys Gly Trp Lys Ile Ser
   1               5                   10                  15
   Ser Asp Pro Arg Thr Tyr Asp Gly Tyr Pro Lys Asn Tyr Gly Tyr Arg
               20                  25                  30
   Asn Tyr His Glu Asn Gly Ile Asn Tyr Asp Glu Phe Cys Gly Gly Tyr
           35                  40                  45
   His Arg Ala Phe Asp Val Tyr Ser Asn Glu Thr Asn Asp Val Pro Ala
       50                  55                  60
   Val Thr Ser Gly Thr Val Ile Glu Ala Asn Asp Tyr Gly Asn Phe Gly
   65                  70                  75                  80
   Gly Thr Phe Val Ile Arg Asp Ala Asn Asp Asn Asp Trp Ile Tyr Gly
                   85                  90                  95
   His Leu Gln Arg Gly Ser Met Arg Phe Val Val Gly Asp Lys Val Asn
               100                 105                 110
   Gln Gly Asp Ile Ile Gly Leu Gln Gly Asn Ser Asn Tyr Tyr Asp Asn
           115                 120                 125
   Pro Met Ser Val His Leu His Leu Gln Leu Arg Pro Lys Asp Ala Lys
       130                 135                 140
   Lys Asp Glu Lys Ser Gln Val Cys Ser Gly Leu Ala Met Glu Lys Tyr
   145                 150                 155                 160
   Asp Ile Thr Asn Leu Asn Ala Lys Gln Asp Lys Ser Lys Asn Gly Ser
                   165                 170                 175
   Val Lys Glu Leu Lys His Ile Tyr Ser Asn His Ile Lys Gly Asn Lys
   180 185 190
   Ile Thr Ala Pro Lys Pro Ser Ile Gln Gly Val Val Ile His Asn Asp
   195 200 205
   Tyr Gly Ser Met Thr Pro Ser Gln Tyr Leu Pro Trp Leu Tyr Ala Arg
   210 215 220
   Glu Asn Asn Gly Thr His Val Asn Gly Trp Ala Ser Val Tyr Ala Asn
   225 230 235 240
   Arg Asn Glu Val Leu Trp Tyr His Pro Thr Asp Tyr Val Glu Trp His
   245 250 255
   Cys Gly Asn Gln Trp Ala Asn Ala Asn Leu Ile Gly Phe Glu Val Cys
   260 265 270
   Glu Ser Tyr Pro Gly Arg Ile Ser Asp Lys Leu Phe Leu Glu Asn Glu
   275 280 285
   Glu Ala Thr Leu Lys Val Ala Ala Asp Val Met Lys Ser Tyr Gly Leu
   290 295 300
   Pro Val Asn Arg Asn Thr Val Arg Leu His Asn Glu Phe Phe Gly Thr
   305 310 315 320
   Ser Cys Pro His Arg Ser Trp Asp Leu His Val Gly Lys Gly Glu Pro
   325 330 335
   Tyr Thr Thr Thr Asn Ile Asn Lys Met Lys Asp Tyr Phe Ile Lys Arg
   340 345 350
   Ile Lys His Tyr Tyr Asp Gly Gly Lys Leu Glu Val Ser Lys Ala Ala
   355 360 365
   Thr Ile Lys Gln Ser Asp Val Lys Gln Glu Val Lys Lys Gln Glu Ala
   370 375 380
   Lys Gln Ile Val Lys Ala Thr Asp Trp Lys Gln Asn Lys Asp Gly Ile
   385 390 395 400
   Trp Tyr Lys Ala Glu His Ala Ser Phe Thr Val Thr Ala Pro Glu Gly
   405 410 415
   Ile Ile Thr Arg Tyr Lys Gly Pro Trp Thr Gly His Pro Gln Ala Gly
   420 425 430
   Val Leu Gln Lys Gly Gln Thr Ile Lys Tyr Asp Glu Val Gln Lys Phe
   435 440 445
   Asp Gly His Val Trp Val Ser Trp Glu Thr Phe Glu Gly Glu Thr Val
   450 455 460
   Tyr Met Pro Val Arg Thr Trp Asp Ala Lys Thr Gly Lys Val Gly Lys
   465 470 475 480
   Leu Trp Gly Glu Ile Lys Ser Thr Arg Ser Ser Arg Ala Gly Leu Gln
   485 490 495
   Phe Pro Val Gly Arg Val His Arg Leu Leu Arg Lys
   500 505
<210> 105
   <211> 1527
   <212> DNA
   <213> artificial
<220>
   <223> Ply2638A-BuforinII
<400> 105
   atgctaactg ctattgacta tcttacgaaa aaaggttgga aaatatcatc tgaccctcgc    60
   acttacgatg gttaccctaa aaactacggc tacagaaatt accatgaaaa cggcattaat   120
   tatgatgagt tttgtggtgg ttatcataga gcttttgatg tttacagtaa cgaaactaac   180
   gacgtgcctg ctgttactag cggaacagtt attgaagcaa acgattacgg taattttggt   240
   ggtacattcg ttattagaga cgctaacgat aacgattgga tatatgggca tctacaacgt   300
   ggctcaatgc gatttgttgt aggcgacaaa gtcaatcaag gtgacattat tggtttacaa   360
   ggtaatagca actattacga caatcctatg agtgtacatt tacatttaca attacgccct   420
   aaagacgcaa agaaagatga aaaatcacaa gtatgtagtg gtttggctat ggaaaaatat   480
   gacattacaa atttaaatgc taaacaagat aaatcaaaga atgggagcgt gaaagagttg   540
   aaacatatct attcaaacca tattaaaggt aacaagatta cagcaccaaa acctagtatt   600
   caaggtgtgg tcatccacaa tgattatggt agtatgacac ctagtcaata cttaccatgg   660
   ttatatgcac gtgagaataa cggtacacac gttaacggtt gggctagtgt ttatgcaaat   720
   agaaacgaag tgctttggta tcatccgaca gactacgtag agtggcattg tggtaatcaa   780
   tgggcaaatg ctaacttaat cggatttgaa gtgtgtgagt cgtatcctgg tagaatctcg   840
   gacaaattat tcttagaaaa tgaagaagcg acattgaaag tagctgcgga tgtgatgaag   900
   tcgtacggat taccagttaa tcgcaacact gtacgtctgc ataacgaatt cttcggaact   960
   tcttgtccac atcgttcgtg ggacttgcat gttggcaaag gtgagcctta cacaactact  1020
   aatattaata aaatgaaaga ctacttcatc aaacgcatca aacattatta tgacggtgga  1080
   aagctagaag taagcaaagc agcaactatc aaacaatctg acgttaagca agaagttaaa  1140
   aagcaagaag caaaacaaat tgtgaaagca acagattgga aacagaataa agatggcatt  1200
   tggtataaag ctgaacatgc ttcgttcaca gtgacagcac cagagggaat tatcacaaga  1260
   tacaaaggtc cttggactgg tcacccacaa gctggtgtat tacaaaaagg tcaaacgatt  1320
   aaatatgatg aggttcaaaa atttgacggt catgtttggg tatcgtggga aacgtttgag  1380
   ggcgaaactg tatacatgcc ggtacgcaca tgggacgcta aaactggtaa agttggtaag  1440
   ttgtggggcg aaattaaatc cacccgtagc tctcgtgctg gcctgcagtt tccggttggt  1500
   cgcgtgcacc gtctgctccg caaataa                                      1527